(19)

Europäisches Patentamt
European Patent Office
Office européen des brevets

(11)  **EP 3 864 152 B1**

(12)  **EUROPEAN PATENT SPECIFICATION**

(45) Date of publication and mention
of the grant of the patent:
**16.08.2023  Bulletin 2023/33**

(51) International Patent Classification (IPC):
**C12N 15/113** (2010.01)      **C12N 15/11** (2006.01)
**C12N 15/10** (2006.01)       **A61K 31/712** (2006.01)

(21) Application number: **19870299.5**

(22) Date of filing: **12.10.2019**

(52) Cooperative Patent Classification (CPC):
**C12N 15/102; A61K 31/7105; A61K 31/712;
A61K 31/7125; C12N 15/111; C12N 15/113;
C12N 2310/20**

(86) International application number:
**PCT/CN2019/110782**

(87) International publication number:
**WO 2020/074001 (16.04.2020 Gazette 2020/16)**

(54)  **METHODS AND COMPOSITIONS FOR EDITING RNAS**

VERFAHREN UND ZUSAMMENSETZUNGEN ZUM EDITIEREN VON RNAS

PROCÉDÉS ET COMPOSITIONS POUR ÉDITER DES ARN

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Validation States:
**TN**

(30) Priority:  **12.10.2018  PCT/CN2018/110105
15.04.2019  PCT/CN2019/082713**

(43) Date of publication of application:
**18.08.2021  Bulletin 2021/33**

(60) Divisional application:
**23184286.5**

(73) Proprietors:
• **Peking University
Beijing 100871 (CN)**
• **EdiGene Therapeutics (Beijing) Inc.
Beijing 102206 (CN)**

(72) Inventors:
• **WEI, Wensheng
Beijing 100871 (CN)**
• **QU, Liang
Beijing 100871 (CN)**
• **YI, Zongyi
Beijing 100871 (CN)**
• **ZHU, Shiyou
Beijing 100871 (CN)**
• **WANG, Chunhui
Beijing 100871 (CN)**
• **CAO, Zhongzheng
Beijing 100871 (CN)**
• **ZHOU, Zhuo
Beijing 100871 (CN)**
• **YUAN, Pengfei
Beijing 102206 (CN)**

(74) Representative: **Lavoix
Bayerstraße 83
80335 München (DE)**

(56) References cited:
WO-A1-2016/097212      WO-A1-2017/220751
WO-A1-2018/041973      WO-A1-2018/161032

• **JOHN R. SINNAMON ET AL: "Site-directed RNA
repair of endogenous Mecp2 RNA in neurons",
PROCEEDINGS OF THE NATIONAL ACADEMY
OF SCIENCES, vol. 114, no. 44, 16 October 2017
(2017-10-16), pages E9395-E9402, XP055588475,
US ISSN: 0027-8424, DOI:
10.1073/pnas.1715320114**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- DAVID B. T. COX ET AL: "RNA editing with CRISPR-Cas13", SCIENCE, vol. 358, no. 6366, 24 November 2017 (2017-11-24), pages 1019-1027, XP055491658, US ISSN: 0036-8075, DOI: 10.1126/science.aaq0180
- QU LIANG ET AL: "Programmable RNA editing by recruiting endogenous ADAR using engineered RNAs", NATURE BIOTECHNOLOGY, GALE GROUP INC, NEW YORK, vol. 37, no. 9, 15 July 2019 (2019-07-15), pages 1059-1069, XP036888288, ISSN: 1087-0156, DOI: 10.1038/S41587-019-0178-Z [retrieved on 2019-07-15]
- LEANNA R. MONTELEONE ET AL: "A Bump-Hole Approach for Directed RNA Editing", CELL CHEMICAL BIOLOGY, vol. 26, no. 2, 1 February 2019 (2019-02-01), pages 269-277.e5, XP055703024, AMSTERDAM, NL ISSN: 2451-9456, DOI: 10.1016/j.chembiol.2018.10.025
- WOOLF, T.M . et al.: "Toward the therapeutic editing of mutated RNA sequences", PROC. NATL. ACAD. SCI. USA, vol. 92, 31 August 1995 (1995-08-31), pages 8298-8302, XP000574995, DOI: 10.1073/pnas.92.18.8298
- QU, L. et al.: "Programmable RNA editing by recruiting endogenous ADAR using engineered RNAs", NATURE BIOTECHNOLOGY, vol. 37, 30 September 2019 (2019-09-30), pages 1059-1069, XP036888288, DOI: 10.1038/s41587-019-0178-z
- COX, D.B.T. et al.: "RNA editing with CRISPR-CasI3", SCIENCE, vol. 358, 24 November 2017 (2017-11-24), pages 1019-1027, XP055491658, DOI: 10.1126/science.aaq0180

Remarks:
The complete document including Reference Table(s) and the Sequence Listing(s) can be downloaded from the EPO website

**Description**

CROSS-REFERENCE TO RELATED APPLICATIONS

This application claims priority to an international application with the International Application No. PCT/CN2018/110105 filed on October 12, 2018 and an international application with the International Application No. CN2019/082713 filed on April 15, 2019.

**SUB**MISSION OF SEQUENCE LISTING ON ASCII TEXT FILE

The content of the following submission on ASCII text file is a computer readable form (CRF) of the Sequence Listing (file name: FC00158PCT New-sequence listing-YZG-zhc.TXT, date recorded: October 11, 2019, size: 104 KB).

**FIELD OF THE INVENTION**

The present invention is related to methods and compositions for editing RNAs using an engineered RNA capable of recruiting an adenosine deaminase to deaminate one or more adenosines in target RNAs.

**BACKGROUND OF THE INVENTION**

Genome editing is a powerful tool for biomedical research and development of therapeutics for diseases. So far, the most popular gene editing technology is the Clustered Regularly Interspaced Short Palindromic Repeats (CRISPR)-Cas system, which was developed from the adaptive immune system of bacteria and archaea. CRISPR-Cas can precisely target and cleave genome DNA, generating Double-Strand DNA Breaks (DSB). DSB can be repaired through non-homologous end joining (NHEJ) pathways, resulting in an insertion or deletion (Indel), which, in most cases, inactivates the gene. Alternatively, the homology-directed repair (HDR) pathway can repair the DSB using homologous templates dsDNA or ssDNA, and thus achieve precise genome editing.

Recently, taking advantage of the deaminase proteins, such as Adenosine Deaminase Acting on RNA (ADAR), novel tools were developed for RNA editing. In mammalian cells, there are three types of ADAR proteins, Adar1 (two isoforms, p110 and p150), Adar2 and Adar3 (catalytically inactive). The catalytic substrate of ADAR protein is double-stranded RNA, and it can remove the $-NH_2$ group from an adenosine (A) nucleobase, changing A to inosine (I), which is recognized as guanosine (G) and paired with cytidine (C) during subsequent cellular transcription and translation processes. Researchers fused λN peptide to human Adar1 or Adar2 deaminase domain to construct the λN-ADARDD system, which could be guided to bind specific RNA targets by a fusion RNA consisting of BoxB stem loop and antisense RNA. This method can edit target A to I by introducing an A-C mismatch at the target A base, resulting in A to G RNA base editing. Other methods for RNA editing include fusing antisense RNA to R/G motif (ADAR-recruiting RNA scaffold) to edit target RNA by overexpressing Adar1 or Adar2 proteins in mammalian cells, and using dCas13-ADAR to precisely target and edit RNA.

**SUMMARY OF THE INVENTION**

Nucleic acid editing carries enormous potential for biological research and the development of therapeutics. Current tools for DNA or RNA editing rely on introducing exogenous proteins into living organisms, which is subject to potential risks or technical barriers due to possible aberrant effector activity, delivery limits and immunogenicity. In some aspects, the present application provides a programmable approach that employs a short RNA to leverage endogenous ADAR (Adenosine Deaminase Acting on RNA) proteins for targeted RNA editing. In some aspects, the present application provides an engineered RNA that is partially complementary to the target transcript to recruit native ADAR1 or ADAR2 to change adenosine to inosine at a specific site in a target RNA. The methods described herein are collectively referred to as "LEAPER" (Leveraging Endogenous ADAR for Programmable Editing on RNA) and the ADAR-recruiting RNAs are referred to interchangeably as "dRNA" or "arRNA."

In one aspect, the present application provides a method for editing on a target RNA in a host cell, comprising introducing a deaminase-recruiting RNA (dRNA) or a construct encoding the deaminase-recruiting RNA into the host cell, wherein the dRNA comprises a complementary RNA sequence that hybridizes to the target RNA and a cytidine mismatch directly opposite the target adenosine in the target RNA, wherein the cytidine mismatch is located at least 20 nucleotides away from the 3' end of the complementary sequence, and at least 5 nucleotides away from the 5' end of the complementary sequence in the dRNA, wherein the dRNA is 50 to 260 nucleotides in length and does not comprise a chemically modified nucleotide, and wherein the dRNA is capable of recruiting an adenosine deaminase acting on RNA 1 or 2 (ADAR1 or ADAR2) to deaminate a target adenosine (A) in the target RNA, wherein the dRNA does not comprise an ADA-recruiting domain. In certain embodiments, the host cell is a eukaryotic cell. In some embodiments, the host cell is a mammalian cell. In some embodiments, the host cell is a human cell. In some embodiments, the host cell is a mouse cell. In some embodiments, the host cell is a prokaryotic cell.

In certain embodiments, the ADAR is naturally or endogenously present in the host cell, for example, naturally or endogenously present in the eukaryotic cell. In some embodiments, the ADAR is endogenously expressed by the host cell. In certain embodiments, the ADAR is exogenous to the host cell. In some embodiments, the ADAR is encoded by a nucleic acid (e.g., DNA or RNA). In some embodiments, the method comprises introducing the ADAR or a construct encoding the ADAR into the host cell. In some embodiments, the method does not comprise introducing any protein into the host cell. The ADAR is ADAR1 and/or ADAR 2. In some embodiments, the ADAR is one or more ADARs selected from the group consisting of hADAR1, hADAR2, murine ADAR1 and murine ADAR2.

In certain embodiments, the dRNA is not recognized by a Cas. In some embodiments, the dRNA does not comprise crRNA, tracrRNA or gRNA used in a CRISPR/Cas system. In some embodiments, the method does not comprise introducing a Cas or Cas fusion protein into the host cell.

In certain embodiments, the deamination of the target A in the target RNA results in a missense mutation, an early stop codon, aberrant splicing, or alternative splicing in the target RNA. In some embodiments, the target RNA encodes a protein, and the deamination of the target A in the target RNA results in a point mutation, truncation, elongation and/or misfolding of the protein. In some embodiments, the deamination of the target A in the target RNA results in reversal of a missense mutation, an early stop codon, aberrant splicing, or alternative splicing in the target RNA. In some embodiments, wherein the target RNA encodes a truncated, elongated, mutated, or misfolded protein, the deamination of the target A in the target RNA results in a functional, full-length, correctly-folded and/or wild-type protein by reversal of a missense mutation, an early stop codon, aberrant splicing, or alternative splicing in the target RNA. In some embodiments, the target RNA is a regulatory RNA, and the deamination of the target A results in change in the expression of a downstream molecule regulated by the target RNA. In certain embodiments, the method is for leveraging an endogenous adenosine deaminase for editing on a target RNA to generate point mutation and/or misfolding of the protein encoded by the target RNA, and/or generating an early stop codon, an aberrant splice site, and/or an alternative splice site in the target RNA.

In certain embodiments, the method comprises introducing a plurality of dRNAs or constructs encoding the a plurality of dRNAs into the host cells, wherein each of the plurality of deaminase-recruiting RNAs comprises a complementary RNA sequence that hybridizes to a corresponding target RNA in the plurality of target RNAs, and wherein each dRNA is capable of recruiting an adenosine deaminase acting on RNA (ADAR) to deaminate a target adenosine (A) in the corresponding target RNA.

In some embodiments, there is provided an edited RNA or a host cell having an edited RNA produced by any one of the methods of RNA editing as described above.

One aspect of the present application provides a dRNA for deamination of a target adenosine in a target RNA by recruiting an Adenosine Deaminase Acting on RNA (ADAR), comprising a complementary RNA sequence that hybridizes to the target RNA, wherein the dRNA comprises a nucleic acid sequence of any one of SEQ ID NOs: 25-44, 142-205, 341-342.

In some embodiments according to any one of the methods or dRNAs described herein, the dRNA comprises a RNA sequence comprising a cytidine (C), preferably further adenosine (A) or uridine (U), directly opposite the target adenosine to be edited in the target RNA. In certain embodiments, the RNA sequence further comprises one or more guanosines each directly opposite a non-target adenosine(s) in the target RNA. In certain embodiments, the 5' nearest neighbor of the target A in the target RNA sequence is a nucleotide selected from U, C, A and G with the preference U>C≈A>G and the 3' nearest neighbor of the target A in the target RNA sequence is a nucleotide selected from G, C, A and U with the preference G>C>A≈U. In certain embodiments, the target A is in a three-base motif selected from the group consisting of UAG, UAC, UAA, UAU, CAG, CAC, CAA, CAU, AAG, AAC, AAA, AAU, GAG, GAC, GAA and GAU in the target RNA. In certain embodiments, wherein the three-base motif is UAG, the dRNA comprises an A directly opposite the U in the three-base motif, a C directly opposite the target A, and a C, G or U directly opposite the G in the three-base motif. In certain embodiments, wherein the three-base motif is UAG in the target RNA, the dRNA comprises ACC, ACG or ACU opposite the UAG of the target RNA.

In some embodiments according to any one of the methods or dRNAs described herein, the deaminase-recruiting RNA comprises more than 40, 45, 50, 55, 60, 65, 70, 75 or 80 nucleotides. In certain embodiments, the deaminase-recruiting RNA is 50-240, 60-230, 65-220, 70-210, 70-200, 70-190, 70-180, 70-170, 70-160, 70-150, 70-140, 70-130, 70-120, 70-110, 70-100, 70-90, 70-80, 75-200, 80-190, 85-180, 90-170, 95-160, 100-150 or 105-140 nucleotides in length. In certain embodiments, the target RNA is a RNA selected from the group consisting of a pre-messenger RNA, a messenger RNA, a ribosomal RNA, a transfer RNA, a long non-coding RNA and a small RNA (e.g., miRNA).

In some embodiments according to any one of the methods or dRNAs described herein, the dRNA is a single-stranded RNA. In some embodiments, the complementary RNA sequence is single-stranded, and wherein the dRNA further comprises one or more double-stranded regions. In some embodiments, the dRNA comprises one or more modifications, such as 2'-O-methyl modification and/or phosphorothioate modification.

In some embodiments, there is provided a construct (e.g., viral vector or plasmid) encoding any one of the dRNA described above. In some embodiments, the construct comprises a promoter operably linked to a sequence encoding the dRNA. In some embodiments, the construct is a DNA construct. In some embodiments, the construct is a viral vector, such as an AAV, such as AAV8.

In some embodiments, there is provided a library comprising a plurality of the dRNAs according to any one of the dRNAs described above or a plurality of the constructs according to any one of the constructs described above.

Also provided are compositions, and host cells comprising any one of the dRNAs described herein, any one of the constructs described herein, or any one of the libraries described herein.

## BRIEF DESCRIPTION OF THE DRAWINGS

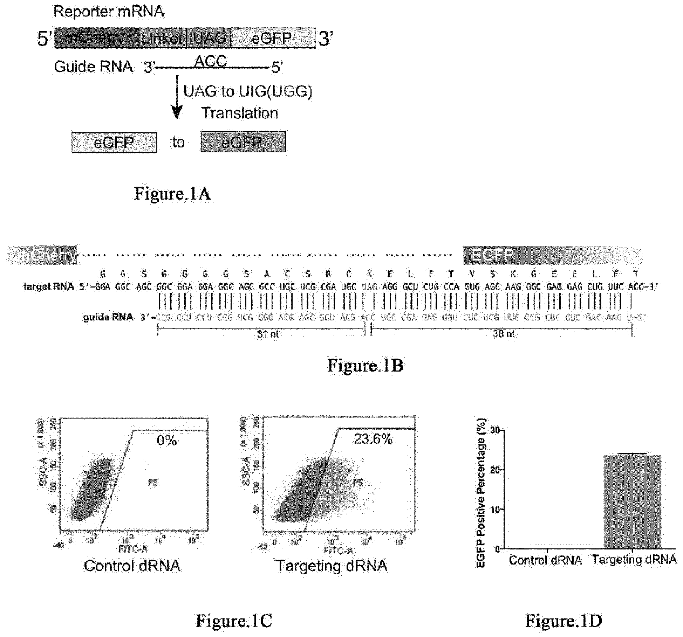

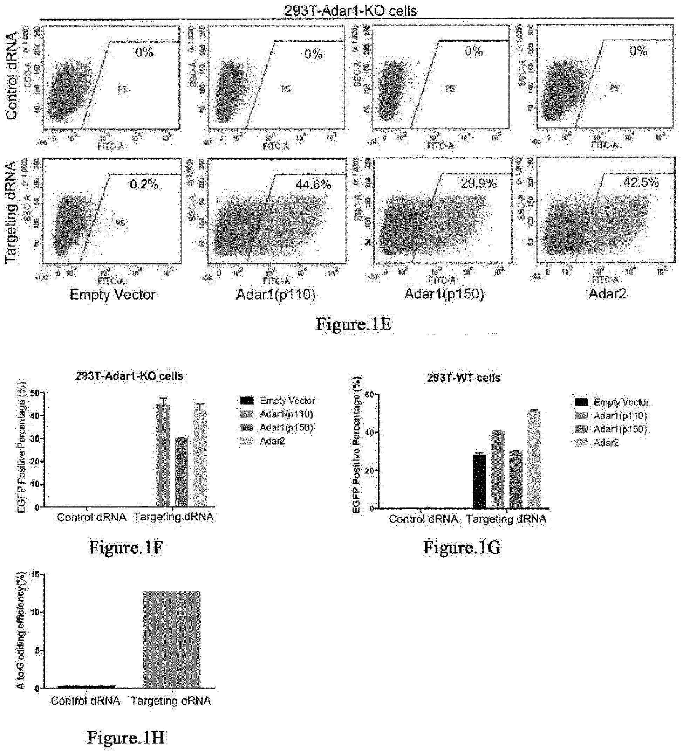

FIGs. 1A-1H show RNA editing with single dRNA utilizing endogenous Adar1 protein. FIG. 1A and 1B show schematic representations of RNA editing with endogenous Adar1 protein. FIG. 1C shows editing reporter mRNA with dRNA using endogenous Adar1 protein. FIG. 1D shows statistical analysis of the results in FIG.1B. FIG. 1E shows Adar1 knockout and Adar1(p110), Adar1(p150) and Adar2 rescue results. FIG. 1F shows statistical analysis of the results in FIG. 1D. FIG. 1G shows the effect of Adar1(p110), Adar1(p150) or Adar2 overexpression on RNA editing mediated by dRNA in 293T-WT cells. FIG. 1H shows that deep sequencing (i.e., Next Generation Sequencing, NGS) results confirmed A to G editing in the targeting site.

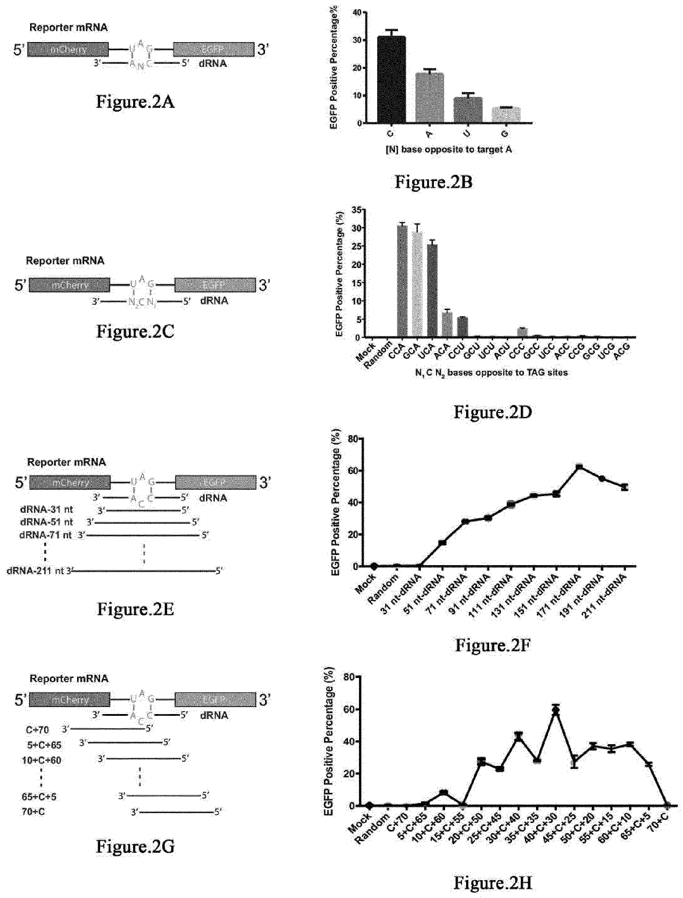

FIGs. 2A-2H show optimization of dRNAs. FIG. 2A shows schematic representation of four kinds of base (A, U, C and G) identify opposite to the targeting adenosine. FIG. 2B shows effects of base identify opposite to the targeting adenosine on RNA editing efficiency by dRNA. FIG. 2C shows schematic representation of dRNA with one, two or three bases mismatched with UAG targeting site. FIG. 2D shows effects of one, two or three bases mismatched with UAG targeting site on Reporter RNA editing by dRNA. dRNA preferred A-C mismatch on the targeting adenosine. FIG. 2E shows schematic representation of dRNA with variant length. FIG. 2F shows the effect of dRNA length on RNA editing efficiency based on dual fluorescence reporter-2. FIG. 2G shows schematic representation of different A-C mismatch position. FIG. 2H shows effect of A-C mismatch position on RNA editing efficiency.

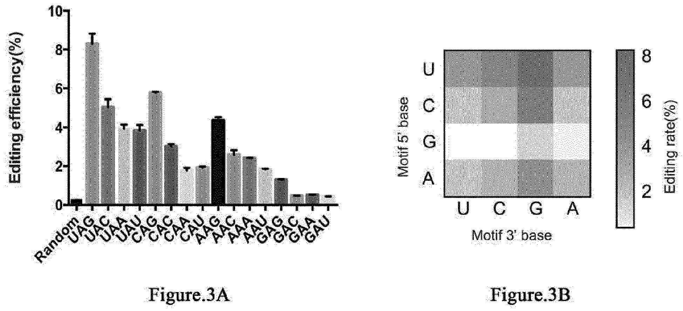

FIGs. 3A-3B show editing flexibility for endogenous RNA editing through exemplary RNA editing method of the present application. FIG. 3A shows percentage quantification of endogenous RNA editing efficiency at all 16 different 3- base motifs. FIG. 3B shows heatmap of 5' and 3' base preferences of endogenous RNA editing for 16 different 3 base motifs.

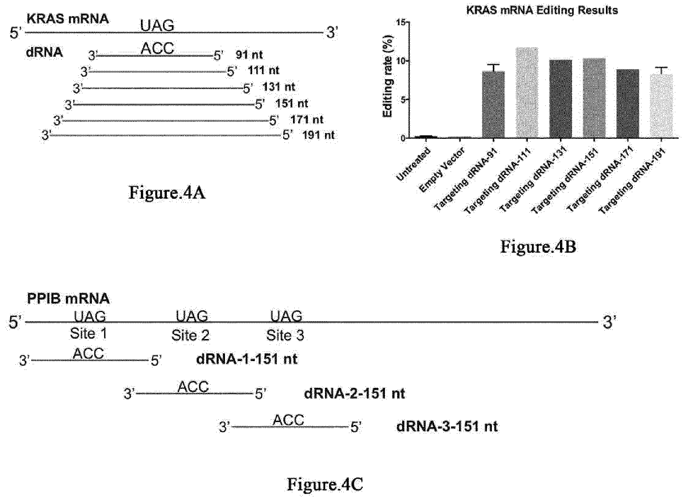

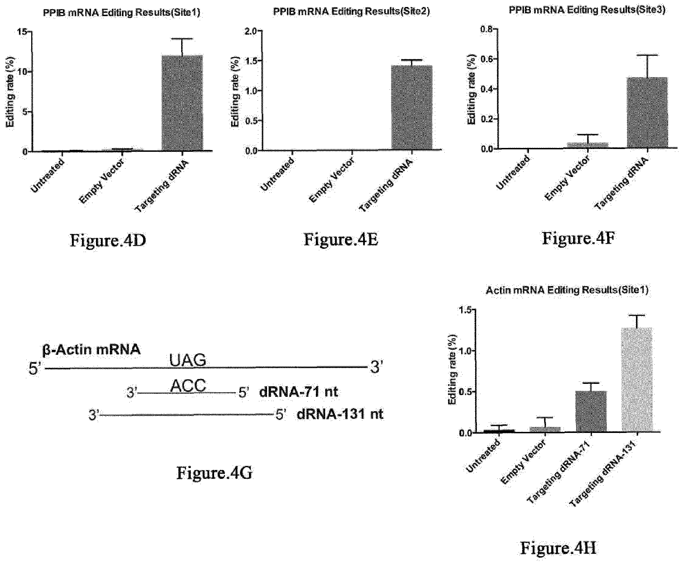

FIGs. 4A-4H show editing the mRNA of endogenous genes with dRNA in 293T cells. FIG. 4A shows schematic representation of KRAS mRNA target and dRNA with variant length. FIG. 4B shows editing the mRNA of endogenous KRAS gene with dRNA in 293T cells. Empty vector, dRNA-91nt plasmids were transfected into 293T-WT cells, respectively. 60 hours later, the RNA was isolated for RT-PCR, and then cDNA was amplified and sequenced on Illumina NextSeq. FIG. 4C shows schematic representation of PPIB mRNA target (site1, site2 and site3) and the corresponding dRNA design. FIGs. 4D, 4E and 4F show editing the mRNA of endogenous PPIB gene with dRNA in 293T cells. FIG. 4G shows schematic representation of β-Actin mRNA target and dRNA (71-nt and 131-nt). FIG. 4H shows editing the mRNA of endogenous β-Actin gene with dRNA in 293T cells.

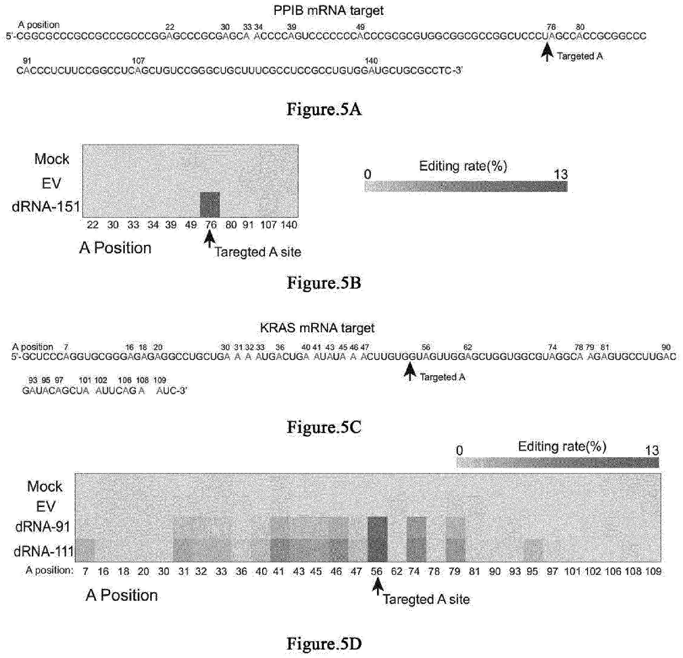

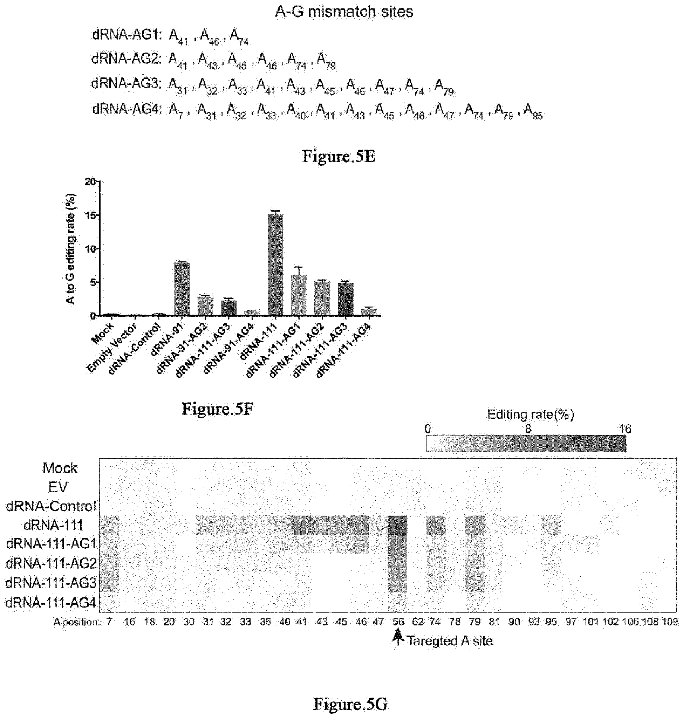

FIGs. 5A-5G show off-target analysis. FIG. 5A shows schematic representation of the sequence window in which A to I edits were analyzed for PPIB mRNA target (PPIB site 1). The black arrow indicates the targeted adenosine. FIG. 5B shows deep sequencing quantification of A to I RNA editing by 151-nt dRNA targeting PPIB mRNA target (PPIB site 1). FIG. 5C shows schematic representation of the sequence window in which A to I edits were analyzed for KRAS mRNA target. The black arrow indicates the targeted adenosine. FIG. 5D shows deep sequencing quantification of A to I RNA editing by 91-nt and 111-nt dRNA targeting KRAS mRNA target. FIG. 5E shows schematic representation of designed four kinds of 91-nt or 111-nt dRNA variants containing different A-G mismatch combinations. The A-G mismatch was designed based on the statistical results in FIG. 5D and existing knowledge on genic codes for different amino acids. FIG. 5F shows the results of targeted A56 editing by dRNA and different kinds of dRNA variants in FIG. 5E. FIG. 5G shows deep sequencing quantification of A to I RNA editing by 111-nt dRNA and four kinds of 111-nt dRNA variants targeting KRAS mRNA target.

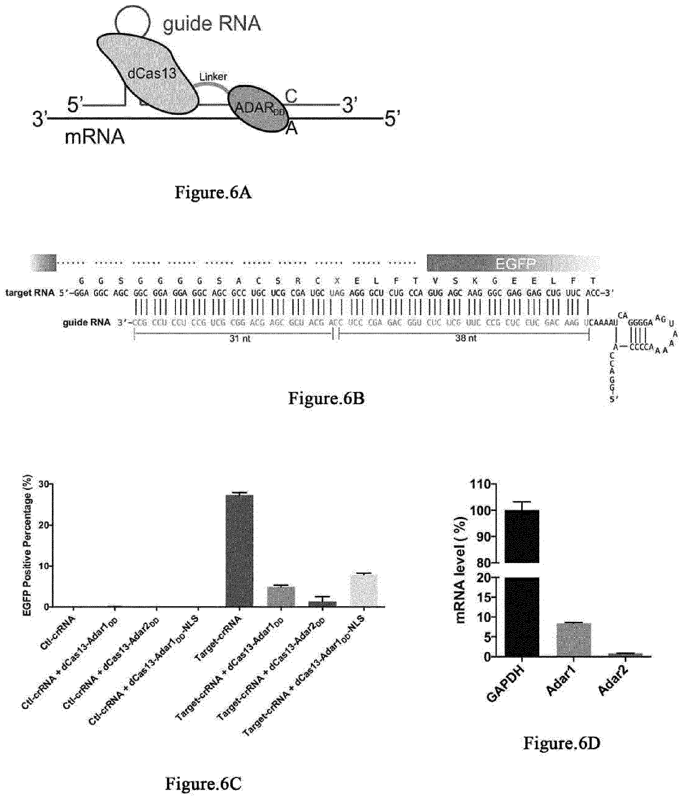

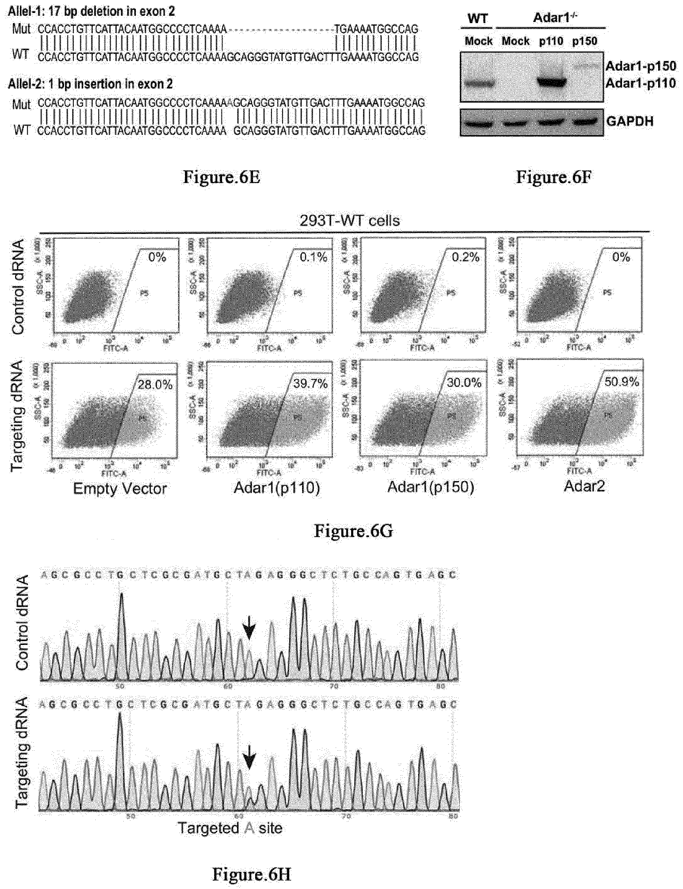

FIGs. 6A-6H show RNA editing with single dRNA utilizing endogenous Adar1 protein. FIG. 6A shows schematic representation of RNA editing by dLbuCas13-ADARDD fusion proteins. The catalytically inactive dLbuCas13 was fused to the RNA deaminase domains of ADAR1 or ADAR2. FIG. 6B shows schematic representation of dual fluorescence reporter mRNA target and guide RNA design. FIG. 6C shows statistical analysis of the results in FIGs. 6A and 6B. FIG. 6D shows the mRNA level of Adar1 and Adar2 in 293T-WT cells. FIG. 6E shows genotyping results of ADAR1 gene in 293T-ADAR1-KO cell lines by genome PCR. FIG. 6F shows the expression level of Adar1(p110) and Adar1(p150) in 293T-WT and 293T-ADAR1-KO cell lines via western blotting. FIG. 6G shows the effects of Adar1(p110), Adar1(p150) or Adar2 overexpression on RNA editing mediated by dRNA in 293T-WT cells via FACS. FIG. 6H shows Sanger sequencing results showed A to G editing in the targeted adenosine site.

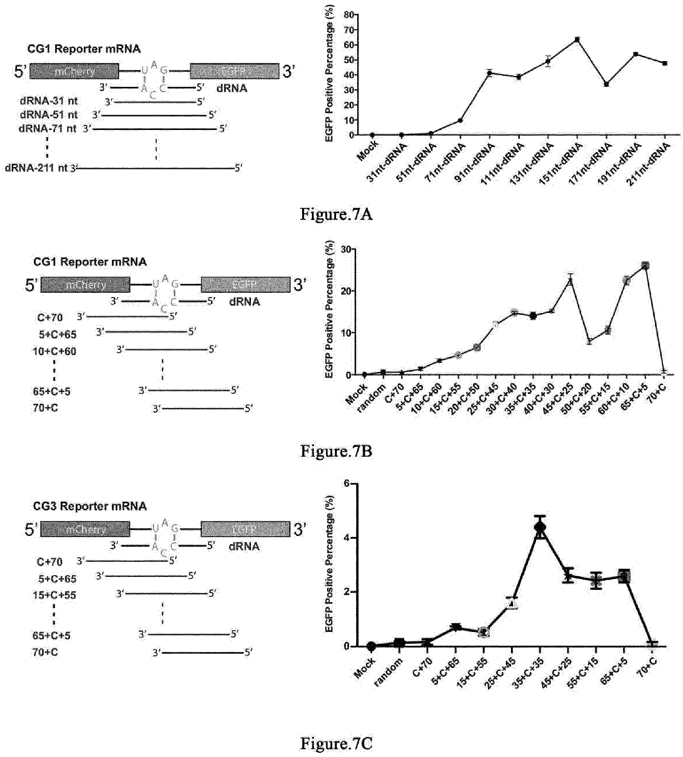

FIGs. 7A-7C shows optimization of dRNAs. FIG. 7A shows schematic representation of dRNA with variant length and the targeted mRNA editing results by dRNA with variant length based on dual fluorescence reporter-1. FIG. 7B shows schematic representation of different A-C mismatch position and the effect of A-C mismatch position on RNA editing efficiency based on dual fluorescence reporter-1. FIG. 7C shows schematic representation of different A-C mismatch position and the effect of A-C mismatch position on RNA editing efficiency based on dual fluorescence reporter-3.

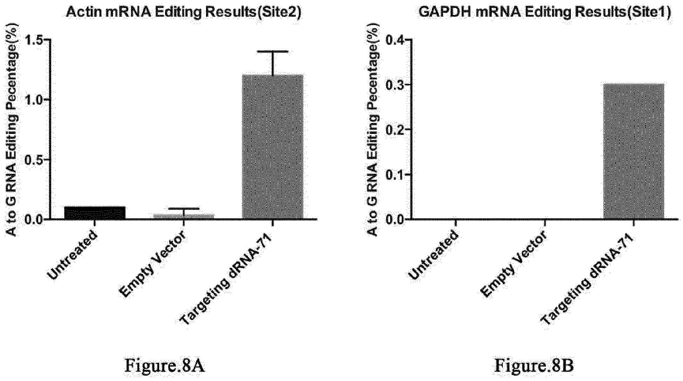

FIGs. 8A-8B shows editing the mRNA of endogenous genes with dRNA in 293T cells. FIG. 8A shows editing the mRNA of endogenous β-Actin gene (site2) with dRNA in 293T cells. FIG. 8B shows editing the mRNA of endogenous GAPDH gene with dRNA in 293T cells.

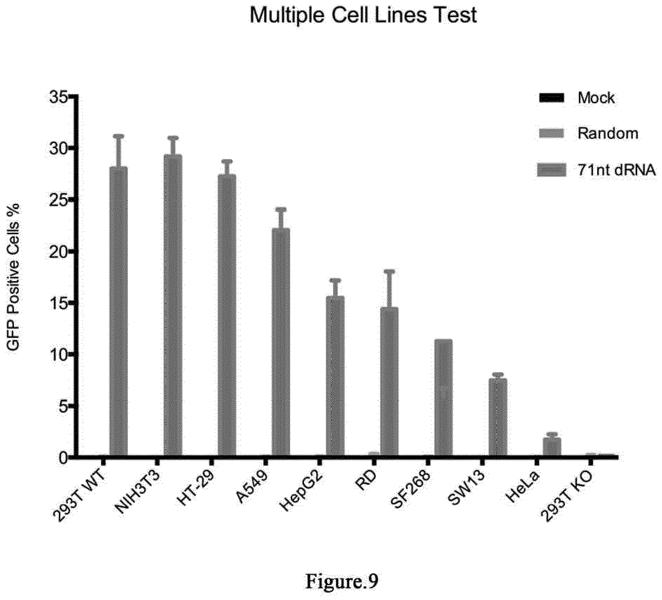

FIG. 9 shows RNA editing by dRNA in different cell lines. FIG. 9A shows that reporter plasmids and dRNA plasmids were co-transfected into different cell lines, and the results showed that dRNA could function well in multiple cell lines, indicating the universality of dRNA application.

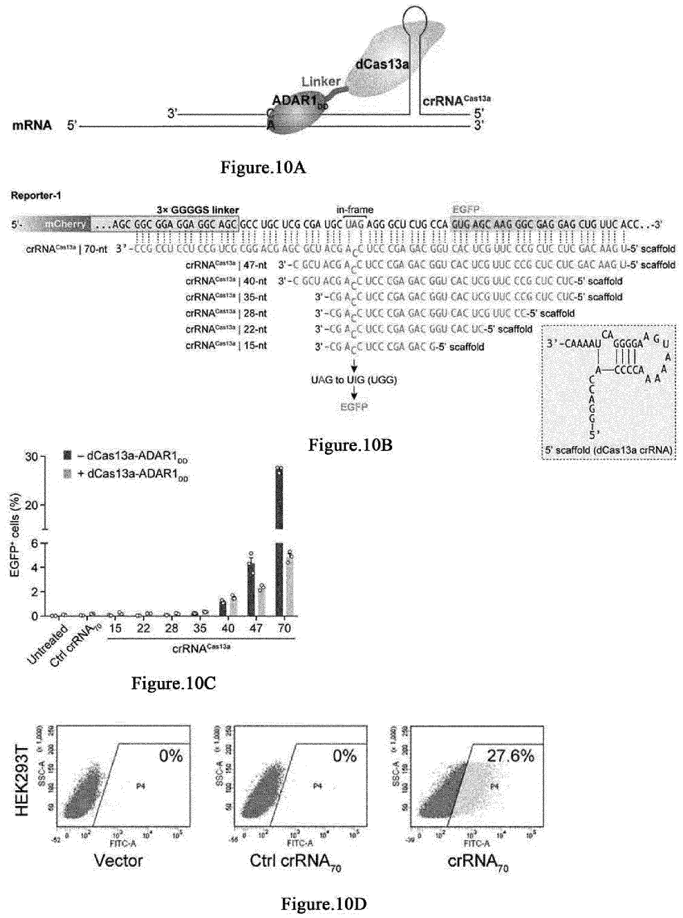

FIGs. 10A-10D show exploration of an efficient exemplary RNA editing platform. FIG. 10A, Schematic of dLbuCas13a-ADAR1$_{DD}$ (E1008Q) fusion protein and the corresponding crRNA. The catalytic inactive LbuCas13a was fused to the deaminase domain of ADAR1 (hyperactive E1008Q variant) using $3\times$ GGGGS linker. The crRNA (crRNA$^{Cas13a}$) consisted of Lbu-crRNA scaffold and a spacer, which was complementary to the targeting RNA with an A-C mismatch as indicated. FIG. 10B, Schematic of dual fluorescent reporter system and the Lbu-crRNA with various lengths of spacers as indicated. FIG. 10C, Quantification of the EGFP positive (EGFP$^+$) cells. HEK293T cells stably expressing the Repoter-1 were transfected with indicated lengths of crRNA$^{Cas13a}$, with or without co-expression of the dLbuCas13a-ADAR1$_{DD}$ (E1008Q), followed by FACS analysis. Data are presented as the mean $\pm$ s.e.m. (n = 3). FIG. 10D, Representative FACS result from the experiment performed with the control (Ctrl crRNA$_{70}$) or the targeting spacer (crRNA$_{70}$).

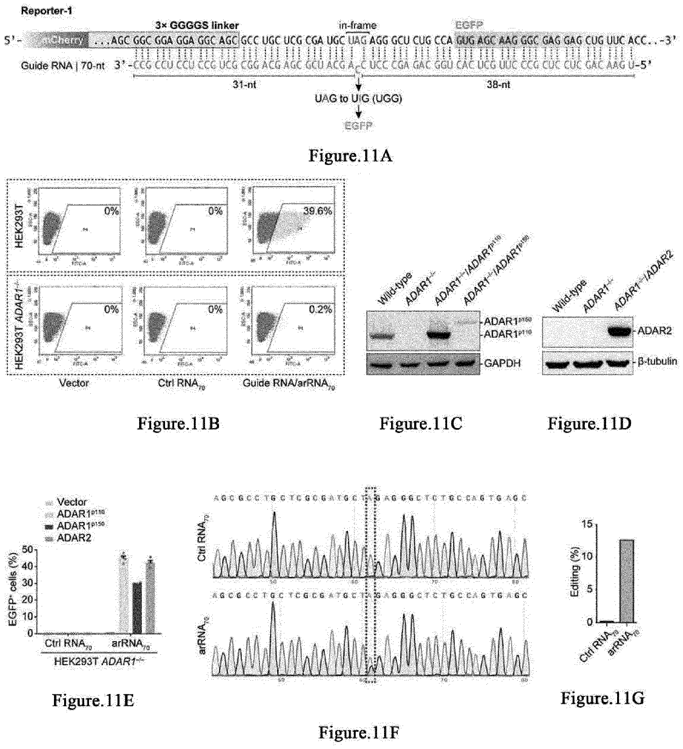

FIGs. 11A-11G show exemplary methods of leveraging endogenous ADAR1 protein for targeted RNA editing. FIG. 11A, Schematic of the Reporter-1 and the 70-nt arRNA. FIG. 11B, Representative FACS analysis of arRNA-induced EGFP expression in wild-type (HEK293T, upper) or ADAR1 knockout (HEK293T *ADAR1$^{-/-}$*, lower) cells stably expressing the Repoter-1. FIG. 11C, Western blot analysis showing expression levels of ADAR1 proteins in wild-type and HEK293T *ADAR1$^{-/-}$* cells, as well as those in HEK293T *ADAR1$^{-/-}$* cells transfected with ADAR1 isoforms (p110 and p150). FIG. 11D, Western blot analysis showing expression levels of ADAR2 proteins in wild-type and HEK293T *ADAR1$^{-/-}$* cells, as well as those in HEK293T *ADAR1$^{-/-}$* cells transfected with ADAR2. FIG. 11E, Quantification of the EGFP positive (EGFP$^+$) cells. Reporter-1 and indicated ADAR-expressing constructs were co-transfected into HEK293T *ADAR1$^{-/-}$* cells, along with the Ctrl RNA$_{70}$ or with the targeting arRNA$_{70}$, followed by FACS analysis. EGFP$^+$ percentages were normalized by transfection efficiency, which was determined by mCherry$^+$. Data are mean values $\pm$ s.e.m. (n = 4). FIG. 11F, The Electropherograms showing Sanger sequencing results in the Ctrl RNA$_{70}$ (upper) or the arRNA$_{70}$ (lower)-targeted region. FIG. 11G, Quantification of the A to I conversion rate at the targeted site by deep sequencing.

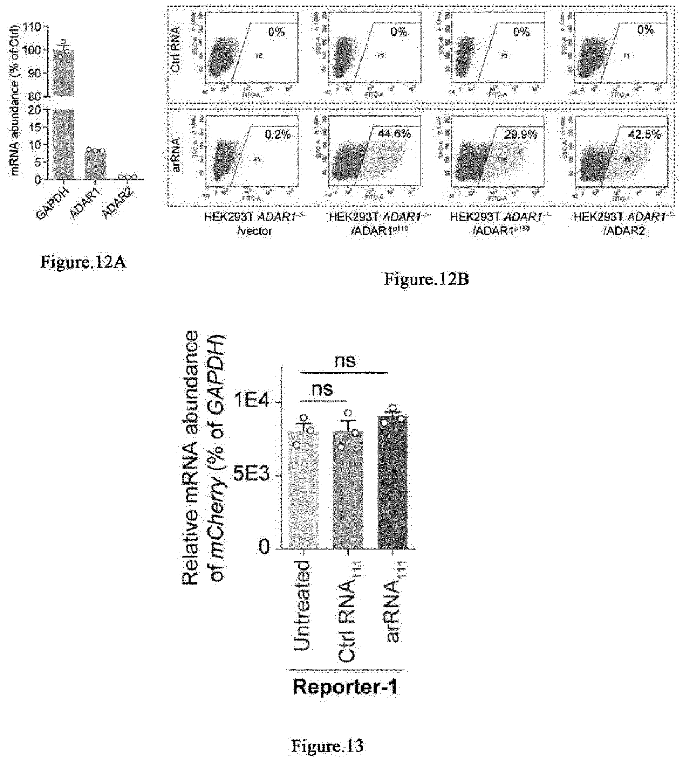

FIGs. 12A-12B show mRNA expression level of ADAR1/ADAR2 and arRNA-mediated RNA editing. FIG. 12A, Quantitative PCR showing the mRNA levels of *ADAR1* and *ADAR2* in HEK293T cells. Data are presented as the mean $\pm$ s.e.m. (n = 3). FIG. 12B, Representative FACS results from Fig. 1E.

FIG. 13 shows quantitative PCR results demonstrating the effects of an exemplary LEAPER method on the expression levels of targeted Reporter-1 transcripts by 111-nt arRNA or control RNA in HEK293T cells. Data are presented as the mean $\pm$ s.e.m. (n = 3); unpaired two-sided Student's *t*-test, ns, not significant.

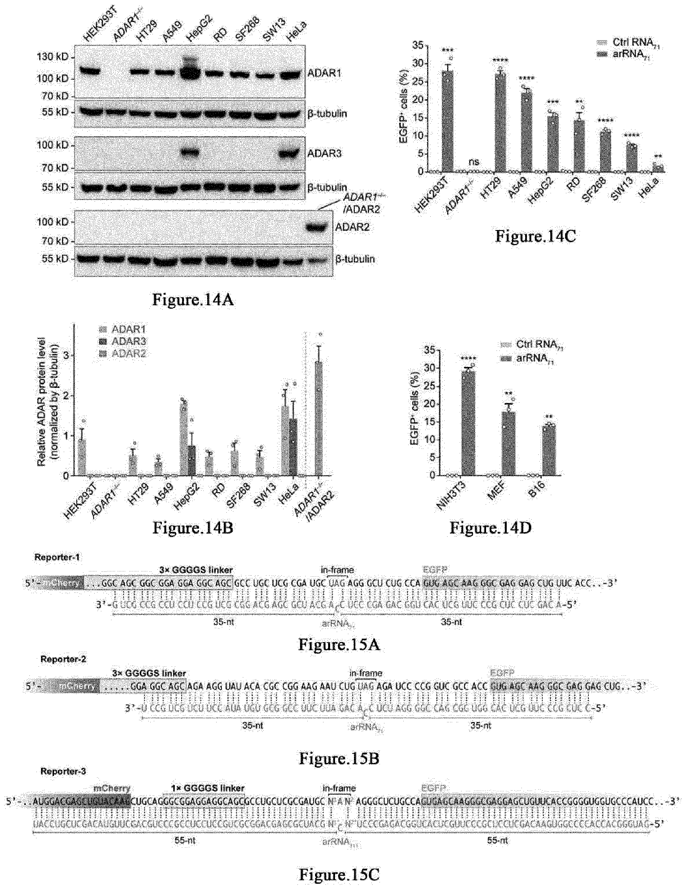

FIGs. 14A-14D show targeted RNA editing with an exemplary LEAPER method in multiple cell lines. FIG. 14A, Western-blot results showing the expression levels of ADAR1, ADAR2 and ADAR3 in indicated human cell lines. β-tubulin was used as a loading control. Data shown is the representative of three independent experiments. *ADAR1$^{-/-}$*/ADAR2 represents ADAR1-knockout HEK293T cells overexpressing ADAR2. FIG. 14B, Relative ADAR protein expression levels normalized by β-tubulin expression. FIG. 14C, Indicated human cells were transfected with Reporter-1, along with the 71-nt control arRNA (Ctrl RNA$_{71}$) or with the 71-nt targeting arRNA (arRNA$_{71}$) followed by FACS analysis. FIG. 14D, Indicated mouse cell lines were analyzed as described in FIG. 14C. EGFP$^+$ percentages were normalized by transfection efficiency, which was determined by mCherry$^+$. Error bars in FIGs. 14 B, 14c, and 14d all indicate the mean $\pm$ s.e.m. (n = 3); unpaired two-sided Student's *t*-test, $*P < 0.05$; $**P < 0.01$; $***P < 0.001$; $****P < 0.0001$; ns, not significant.

FIGs. 15A-15C show schematics of Reporter-1 (a), -2 (b), and -3 (c), as well as their corresponding arRNAs.

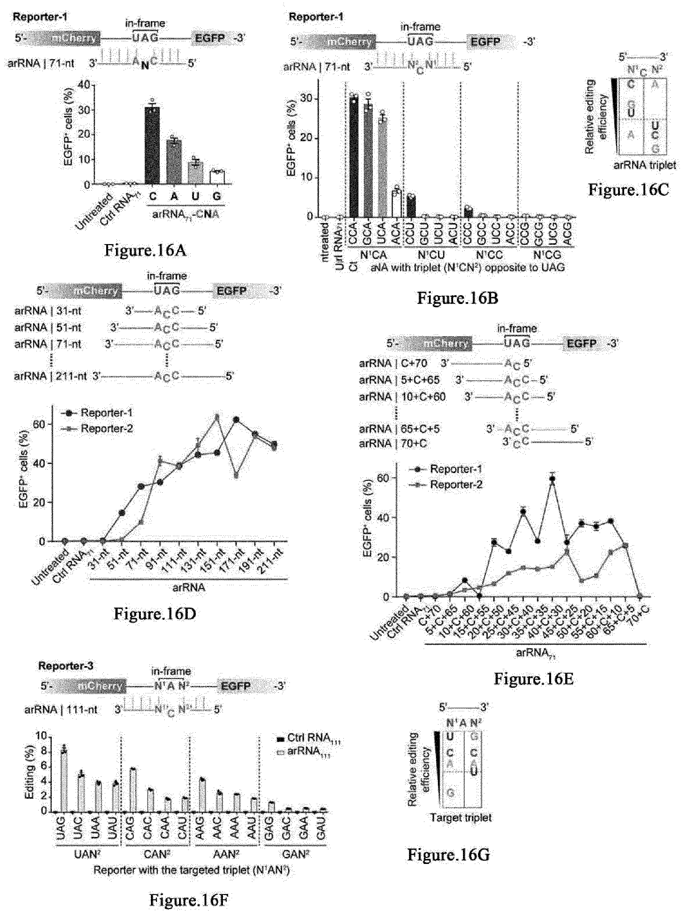

FIGs. 16A-16G show characterization and optimization of exemplary LEAPER methods. FIG. 16A, Top, schematic of the design of arRNAs with changed triplet (5'-CNA, N denotes A, U, C or G) opposite to the target UAG. Bottom, EGFP$^+$ percent showing the effects of variable bases opposite to the targeted adenosine on RNA editing efficiency.

FIG. 16B, Top, the design of arRNAs with changed neighboring bases flanking the cytidine in the A-C mismatch (5'-$N^1CN^2$). Bottom, the effects of 16 different combinations of $N^1CN^2$ on RNA editing efficiency. FIG. 16C, Summary of the preference of 5' and 3' nearest neighboring sites of the cytidine in the A-C mismatch. FIG. 16D, Top, the design of arRNAs with variable length. Bottom, the effect of arRNA length on RNA editing efficiency based on Reporter-1 and Reporter-2. FIG. 16E, Top, the design of arRNAs with variable A-C mismatch position. Bottom, the effect of A-C mismatch position on RNA editing efficiency based on Reporter 1 and Reporter-2. FIG. 16F, Top, the design of the triplet motifs in the reporter-3 with variable nearest neighboring bases surrounding the targeting adenosine (5'-$N^1AN^2$) and the opposite motif (5'-$N^2CN^1$) on the 111-nt arRNA (arRNA$_{111}$). Bottom, deep sequencing results showing the editing rate on targeted adenosine in the 5'-$N^1AN^2$ motif. FIG. 16G, Summary of the 5' and 3' base preferences of LEAPER-mediated editing at the Reporter-3. Error bars in FIGs. 16A, 16B, 16D, 16E and 16F all indicate mean values $\pm$ s.e.m. (n = 3).

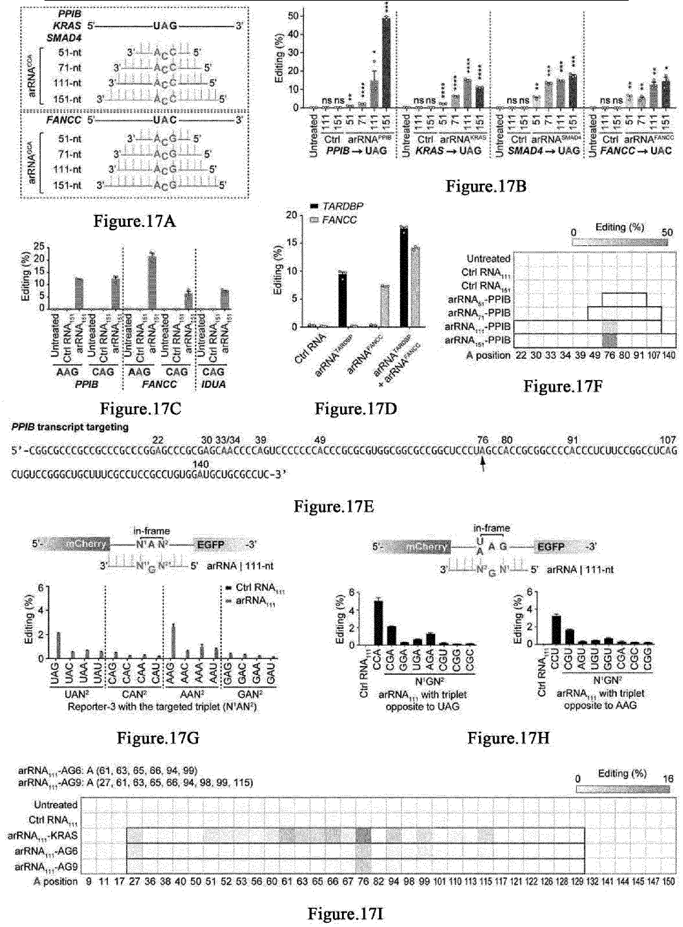

FIGs. 17A-17Ishow editing of endogenous transcripts with exemplary LEAPER methods. FIG. 17A, Schematic of the targeting endogenous transcripts of four disease-related genes (*PPIB, KRAS, SMAD4* and *FANCC*) and the corresponding arRNAs. FIG. 17B, Deep sequencing results showing the editing rate on targeted adenosine of the *PPIB, KRAS, SMAD4* and *FANCC* transcripts by introducing indicated lengths of arRNAs. FIG. 17C, Deep sequencing results showing the editing rate on non-UAN sites of endogenous PPIB, FANCC and IDUA transcripts. FIG. 17D, Multiplex editing rate by two 111-nt arRNAs. Indicated arRNAs were transfected alone or were co-transfected into the HEK293T cells. The targeted editing at the two sites was measured from co-transfected cells. FIG. 17E, Schematic of the *PPIB* transcript sequence covered by the 151-nt arRNA. The black arrow indicates the targeted adenosine. All adenosines were marked in red. FIG. 17F, Heatmap of editing rate on adenosines covered by indicated lengths of arRNAs targeting the *PPIB* gene (marked in bold frame in blue). For the 111-nt arRNA or arRNA$_{151}$-PPIB covered region, the editing rates of A22, A30, A33, and A34 were determined by RNA-seq because of the lack of effective PCR primers for amplifying this region. Otherwise the editing rate was determined by targeted deep-sequencing analysis. FIG. 17G, Top, the design of the triplet motifs in the reporter-3 with variable nearest neighboring bases surrounding the targeting adenosine (5'-$N^1AN^2$) and the opposite motif (5'-$N^{2'}GN^{1'}$) in the 111-nt arRNA (arRNA$_{111}$). Bottom, deep sequencing results showing the editing rate. FIG. 17H, Top, the design of arRNAs with two consecutive mismatches in the 5'-$N^1GN^2$ motif opposite to the 5'-UAG or the 5'-AAG motifs. Deep sequencing results showing the editing rate by an arRNA$_{111}$ with two consecutive mismatches in the 5'-$N^1GN^2$ motif opposite to the 5'-UAG motif (bottom left) or the 5'-AAG motif (bottom right). FIG. 17I, Heatmap of the editing rate on adenosines covered by engineered arRNA$_{111}$ variants targeting the *KRAS* gene. Data in FIGs. 17B, 17C, 17D, 17G and 17H are presented as the mean $\pm$ s.e.m. (n = 3); unpaired two-sided Student's t-test, $*P < 0.05$; $**P < 0.01$; $***P < 0.001$; $****P < 0.0001$; NS, not significant. Data in (**f** and **i**) are presented as the mean (n = 3).

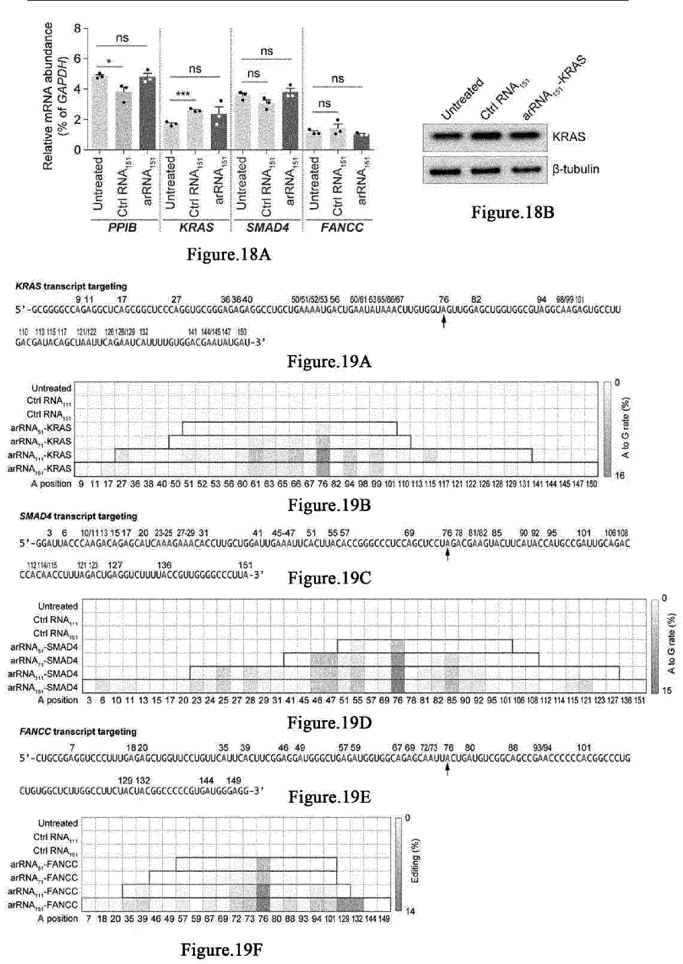

FIGs. 18A-18B show effects of exemplary LEAPER methods on the expression levels of targeted transcripts and protein products. FIG. 18A, Quantitative PCR showing the expression levels of targeted transcripts from PPIB, KRAS, SMAD4 and FANCC by the corresponding 151-nt arRNA or Control RNA in HEK293T cells. Data are presented as the mean $\pm$ s.e.m. (n = 3); unpaired two-sided Student's t-test, $*P < 0.05$; $**P < 0.01$; $***P < 0.001$; $****P < 0.0001$; ns, not significant. FIG. 18B, Western blot results showing the effects on protein products of targeted KRAS gene by 151-nt arRNA in HEK293T cells. $\beta$-tubulin was used as a loading control.

FIGs. 19A-19F show editing of endogenous transcripts with exemplary LEAPER methods. FIG. 19A, Schematic of the *KARS* transcript sequence covered by the 151-nt arRNA. The arrow indicates the targeting adenosine. All adenosines were marked in red. FIG. 19B, Heatmap of editing rate on adenosines covered by indicated arRNAs in the *KARS* transcript (marked in the bold frame in blue). FIG. 19C, Schematic of the *SMAD4* transcript covered by the 151-nt arRNA. FIG. 19D, Heatmap of editing rate on adenosines covered by indicated arRNAs in the *SMAD4* transcript. FIG. 19E, Schematic of the *FANCC* transcript covered by the 151-nt arRNA. FIG. 19F, Heatmap of editing rate on adenosines covered by indicated arRNAs in the *FANCC* transcript. For each arRNA, the region of duplex RNA is highlighted with bold frame in blue. Data (FIGs. 19B, 19D, and 19F) are presented as the mean (n = 3).

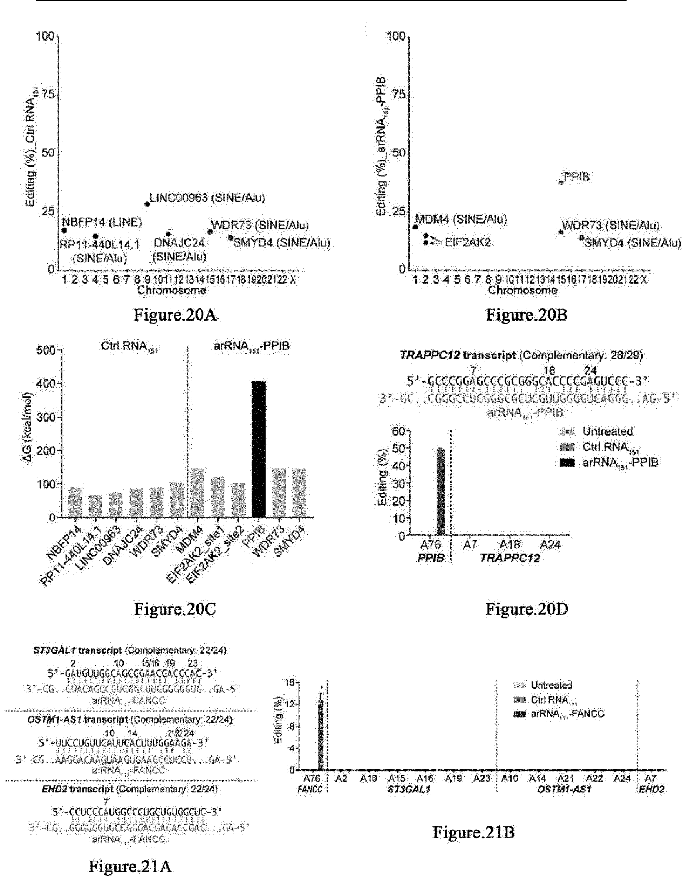

FIGs. 20A-20D show transcriptome-wide specificity of RNA editing by LEAPER. FIGs. 20A and 20B, Transcriptome-wide off-targeting analysis of Ctrl RNA$_{151}$ and arRNA$_{151}$-PPIB. The on-targeting site (PPIB) is highlighted in red. The potential off-target sites identified in both Ctrl RNA and PPIB-targeting RNA groups are labeled in blue. FIG. 20C, The predicted annealing affinity between off-target sites and the corresponding Ctrl RNA$_{151}$ or arRNA$_{151}$-PPIB. The minimum free energy ($\Delta G$) of double-stranded RNA formed by off-target sites (150-nt upstream and downstream of the editing sites) and the corresponding Ctrl RNA$_{151}$ or arRNA$_{151}$-PPIB was predicted with RNAhybrid, an online website tool. FIG. 20D, Top, schematic of the highly complementary region between arRNA$_{151}$-PPIB and the indicated potential off-target sites, which were predicted by searching homologous sequences through NCBI-BLAST. Bottom, Deep sequencing showing the editing rate on the on-target site and all predicted off-target sites of arRNA$_{151}$-PPIB. Data are presented as the mean $\pm$ s.e.m. (n = 3).

FIGs. 21A-21B show evaluation of potential off-targets. FIG. 21A, Schematic of the highly complementary region of arRNA$_{111}$-FANCC and the indicated potential off-target sequence, which were predicted by searching homologous sequences through NCBI-BLAST. FIG. 21B, Deep sequencing showing the editing rate on the on-target site and all predicted off-target sites of arRNA$_{111}$-FANCC. All data are presented as the mean $\pm$ s.e.m. (n = 3).

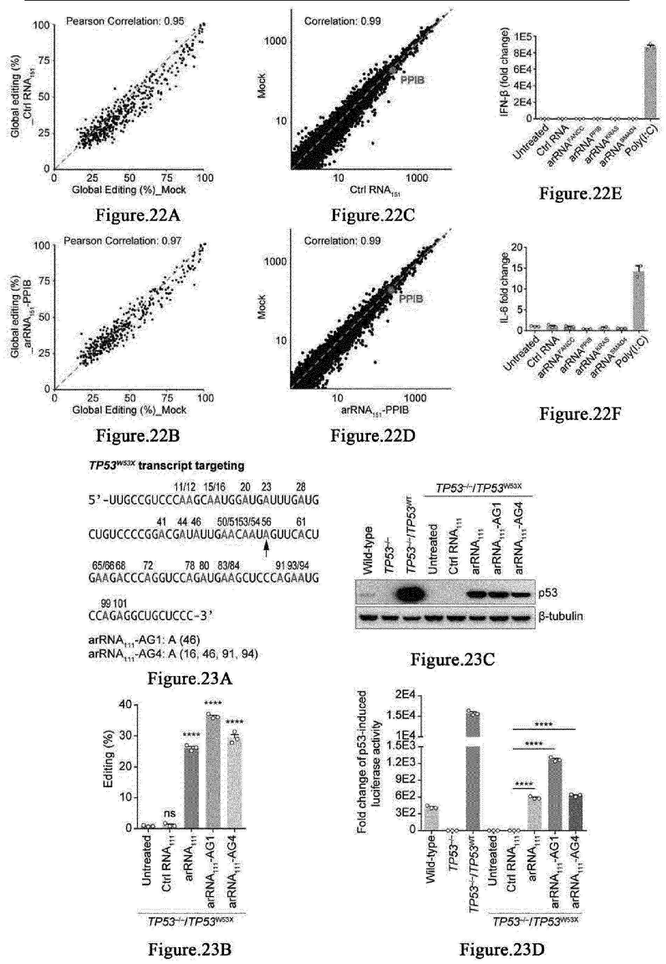

FIGs. 22A-22F show safety evaluation of applying exemplary LEAPER methods in mammalian cells. FIGs. 22A and 22B, Transcriptome-wide analysis of the effects of Ctrl RNA$_{151}$ (a) arRNA$_{151}$-PPIB (b) on native editing sites by transcriptome-wide RNA-sequencing. Pearson's correlation coefficient analysis was used to assess the differential RNA editing rate on native editing sites. FIGs. 22C and 22D, Differential gene expression analysis of the effects of Ctrl RNA$_{151}$ (c) arRNA$_{151}$-PPIB (d) with RNA-seq data at the transcriptome level. Pearson's correlation coefficient analysis was used to assess the differential gene expression. FIGs. 22E and 22F, Effect of arRNA transfection on innate immune response. The indicated arRNAs or the poly(I:C) were transfected into HEK293T cells. Total RNA was then analyzed using quantitative PCR to determine expression levels of IFN-$\beta$ (e) and IL-6 (f). Data (e and f) are presented as the mean $\pm$ s.e.m. (n = 3).

FIGs. 23A-23D show recovery of transcriptional regulatory activity of mutant TP53W53X by LEAPER. FIG. 23A, Top, Schematic of the *TP53* transcript sequence covered by the 111-nt arRNA containing c.158G>A clinical-relevant non-sense mutation (Trp53Ter). The black arrow indicates the targeted adenosine. All adenosines were marked in red. Bottom, the design of two optimized arRNAs targeting *TP53*$^{W53X}$ transcripts with A-G mismatch on A$^{46th}$ for arRNA$_{111}$-AG1, and on A$^{16th}$, A$^{46th}$, A$^{91th}$ and A$^{94th}$ together for arRNA$_{111}$-AG4 to minimize the potential off-targets on "editing-prone" motifs. FIG. 23B, Deep sequencing results showing the targeted editing on *TP53*$^{W53X}$ transcripts by arRNA$_{111}$, arRNA$_{111}$-AG1 and arRNA$_{111}$-AG4. FIG. 23C, Western blot showing the recovered production of full-length p53 protein from the *TP53*$^{W53X}$ transcripts in the HEK293T *TP53*$^{-/-}$ cells. FIG. 23D, Detection of the transcriptional regulatory activity of restored p53 protein using a p53-Firefly-luciferase reporter system, normalized by co-transfected Renilla-luciferase vector. Data (b, c and d) are presented as the mean $\pm$ s.e.m. (n = 3); unpaired two-sided Student's t-test, *$P < 0.05$; **$P < 0.01$; ***$P < 0.001$; ****$P < 0.0001$; ns, not significant.

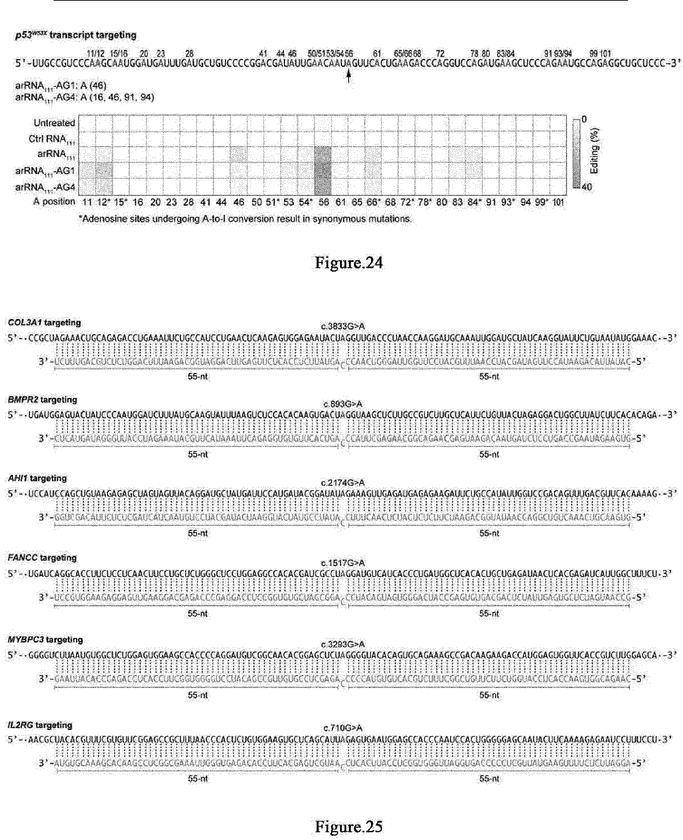

FIG. 24 show editing of mutant TP53W53X transcripts by an exemplary LEAPER method. Top, schematic of the *TP53* transcript sequence covered by the 111-nt arRNAs. The arrow indicates the targeted adenosine. All adenosines were marked in red. Bottom, a heatmap of editing rate on adenosines covered by indicated arRNAs in the *TP53* transcript.

FIG. 25 shows a schematic representation of the selected disease-relevant cDNA containing G to A mutation from ClinVar data and the corresponding 111-nt arRNA.

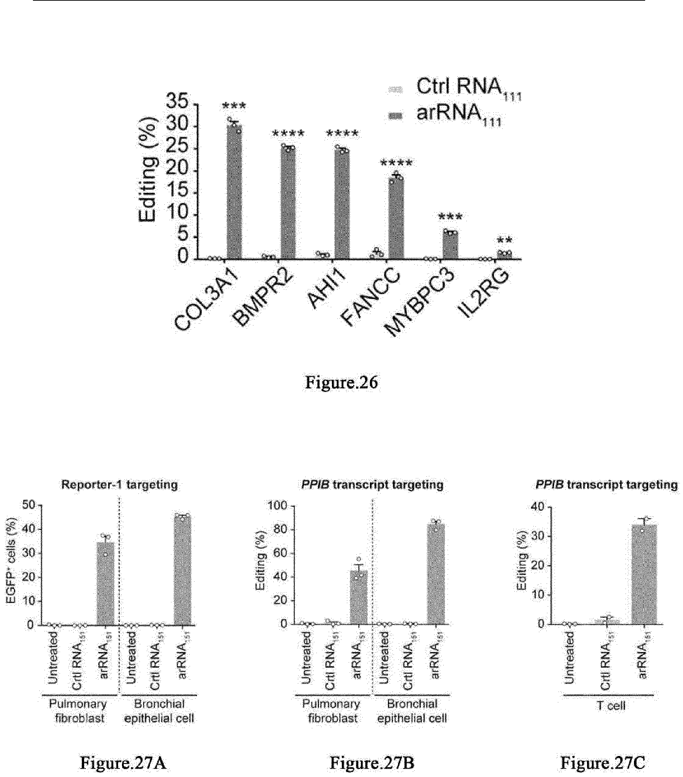

FIG. 26 shows correction of pathogenic mutations by an exemplary LEAPER method. A to I correction of disease-relevant G>A mutation from ClinVar data by the corresponding 111-nt arRNA, targeting clinical-related mutations from six pathogenic genes as indicated (Fig. 25 and the tables of the sequences of arRNAs and control RNAs and disease-related cDNAs below). Data are presented as the mean $\pm$ s.e.m. (n = 3); unpaired two-sided Student's t-test, *$P < 0.05$; **$P < 0.01$; ***$P < 0.001$; ****$P < 0.0001$; ns, not significant.

FIGs. 27A-27C show RNA editing in multiple human primary cells by exemplary LEAPER methods. FIG. 27A, Quantification of the EGFP positive (EGFP$^+$) cells induced by LEAPER-mediated RNA editing. Human primary pulmonary fibroblasts and human primary bronchial epithelial cells were transfected with Reporter-1, along with the 151-nt control RNA (Ctrl RNA$_{151}$) or the 151-nt targeting arRNA (arRNA$_{151}$) followed by FACS analysis. FIGs. 27B and 27C, Deep sequencing results showing the editing rate on *PPIB* transcripts in human primary pulmonary fibroblasts, human primary bronchial epithelial cells (b), and human primary T cells (c). Data in a, b and Untreated group (c) are presented as the mean $\pm$ s.e.m. (n = 3); data of Ctrl RNA$_{151}$ and arRNA$_{151}$ (c) are presented as the mean $\pm$ s.e.m. (n = 2).

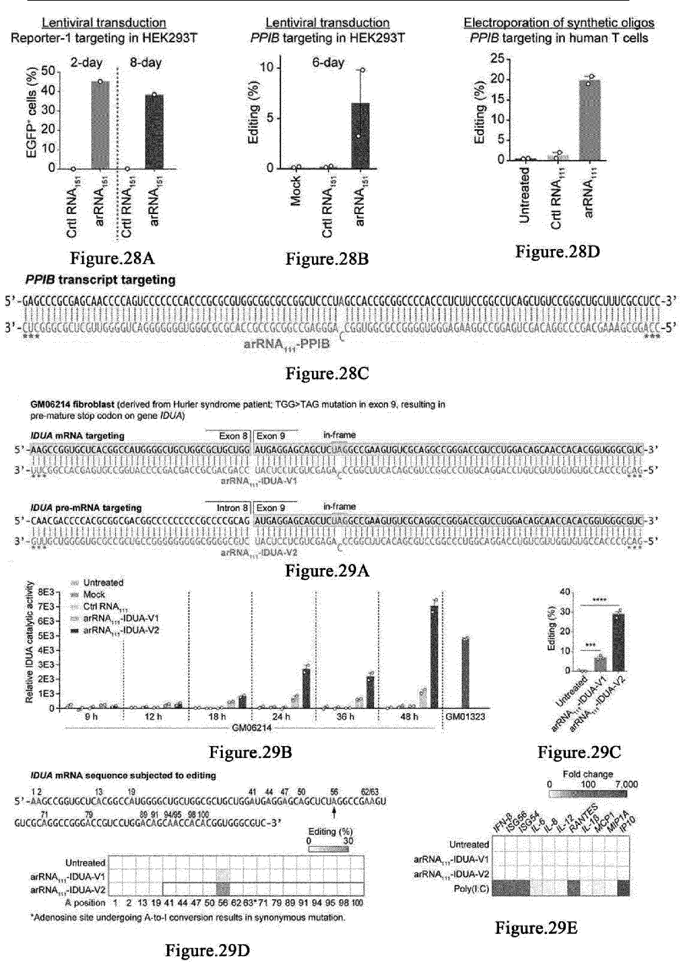

FIGs. 28A-28D show targeted editing by lentiviral transduction of arRNA and electroporation of synthesized arRNA oligonucleotides. FIG. 28A, Quantification of the EGFP$^+$ cells. HEK293T cells stably expressing the Repoter-1 were infected with lentivirus expressing 151-nt of Ctrl RNA or the targeting arRNA. FACS analyses were performed 2 days and 8 days post infection. The ratios of EGFP$^+$ cells were normalized by lentiviral transduction efficiency (BFP$^+$ ratios). FIG. 28B, Deep sequencing results showing the editing rate on the *PPIB* transcripts upon lentiviral transduction of 151-nt arRNAs into HEK293T cells. FIG. 28C, Schematic of the *PPIB* sequence and the corresponding 111-nt targeting arRNA. *(in red) represents nucleotide with 2'-O-methyl and phosphorothioate linkage modifications. FIG. 28D, Deep sequencing results showing the editing rate on the *PPIB* transcripts upon electroporation of 111-nt synthetic arRNA oligonucleotides into human primary T cells.

FIGs. 29A-29E show restoration of $\alpha$-L-iduronidase activity in Hurler syndrome patient-derived primary fibroblast by an exemplary LEAPER method. FIG. 29A, Top, genetic information of pathogenic mutation in patient-derived fibroblast GM06214; Medium, schematic of the *IDUA* mature mRNA sequence of GM06214 cells (Black) containing a homozygous TGG>TAG mutation in exon 9 of the *IDUA* gene (Trp402Ter), and the corresponding 111-nt targeting arRNA$_{111}$-IDUA-V1 (Blue); Bottom, schematic of the *IDUA* pre-mRNA sequence of GM06214 cells (Black) and the corresponding 111-nt targeting arRNA$_{111}$-IDUA-V2 (Blue). *(in red) represents nucleotides with 2'-O-methyl and phosphorothioate linkage modifications. FIG. 29B, Measuring the catalytic activity of $\alpha$-L-iduronidase with 4-methylumbelliferyl $\alpha$-L-iduronidase substrate at different time points. Data are presented as the mean $\pm$ s.e.m. (n = 2).

FIG. 29C, Deep sequencing results showing the targeted editing rate on *IDUA* transcripts in GM06214 cells, 48 hours post electroporation. FIG. 29D, Top, schematic of the *IDUA* transcript sequence covered by the 111-nt arRNAs. The arrow indicates the targeted adenosine. All adenosines were marked in red. Bottom, a heatmap of editing rate on adenosines covered by indicated arRNAs in the *IDUA* transcript (marked in the bold frame in blue). **e**, Quantitative PCR showing the expressions of type I interferon, interferon-stimulated genes, and pro-inflammatory genes upon arRNA or poly(I:C) electroporation. Data are presented as the mean (n = 3).

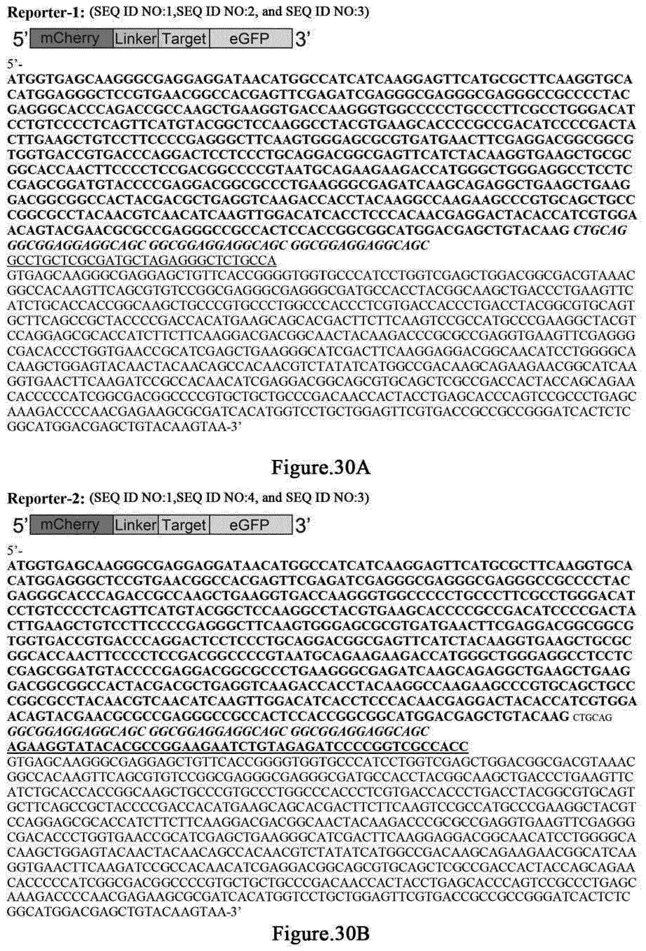

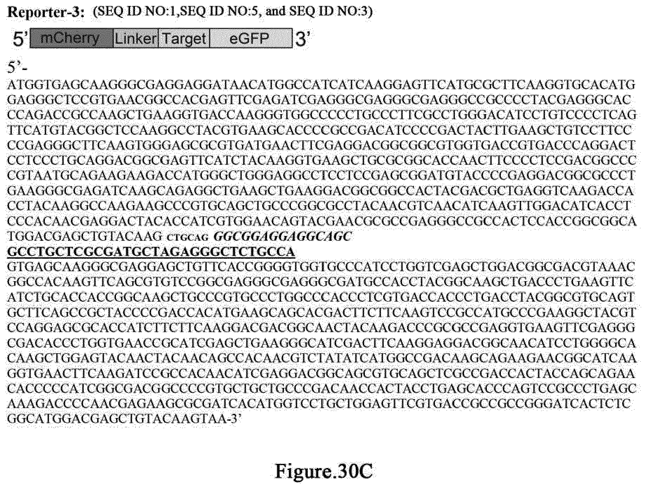

FIGs. 30A-30C shows three versions of dual fluorescence reporters (Reporter-1, -2 and -3), mCherry and EGFP. FIG. 30A, structure of Reporter-1, FIG. 30B, structure of Reporter-2, and FIG. 30C, structure of Reporter-3.

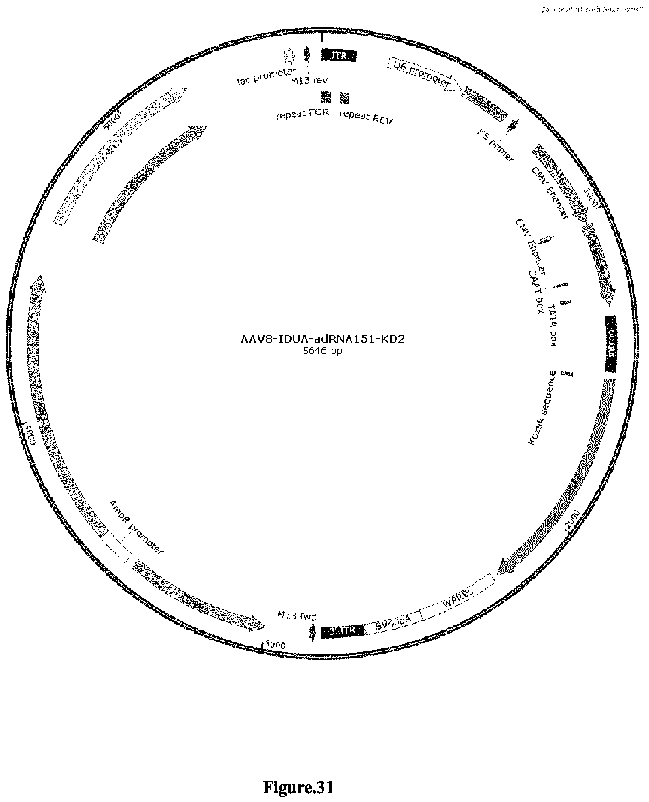

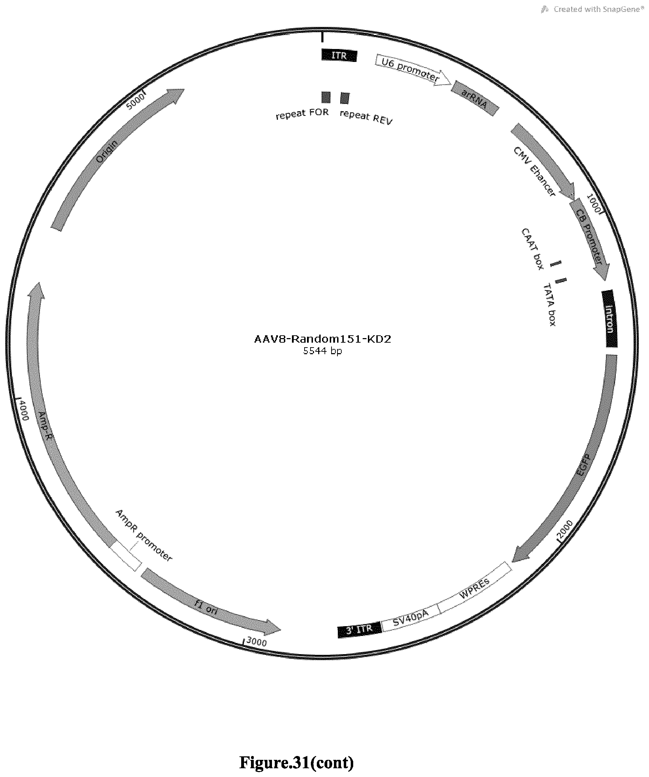

FIG. 31 shows the plasmid information of AAV8-IDUA-adRNA151-KD2 and AAV8-Random151-KD2.

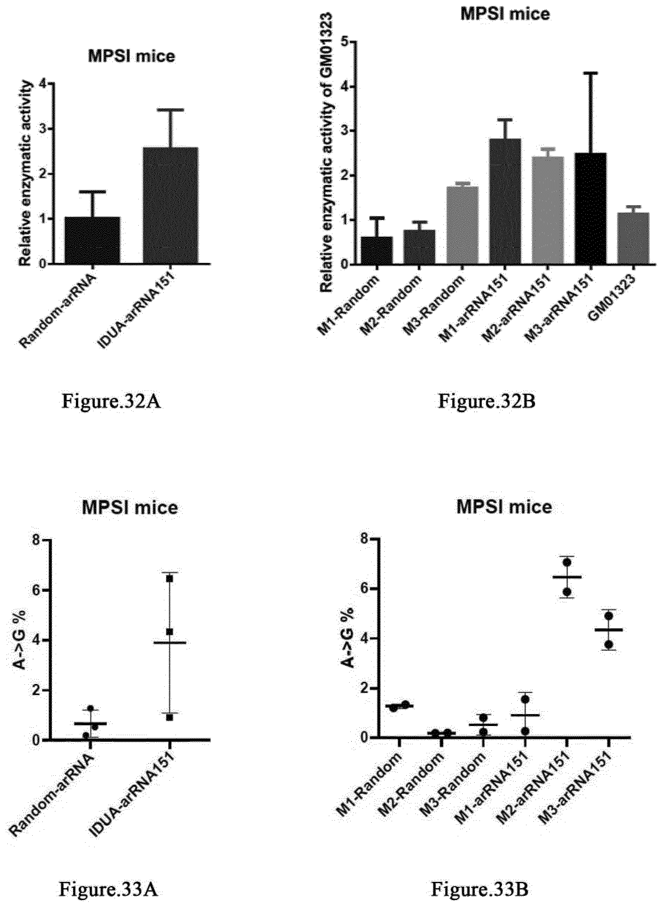

FIG. 32A-B show α-L-iduronidase relative enzymatic activity of MPSI mice injected with AAV8-IDUA-adRNA151-KD2 and AAV8-Random151-KD2. GM01323 cells as a control.

FIG. 33A-B show the RNA editing efficiency of MPSI mice injected with AAV8-IDUA-adRNA151-KD2 and AAV8-Random151-KD2.

## DETAILED DESCRIPTON OF THE INVENTION

The present application provides RNA editing methods (referred herein as "LEAPER" methods) and specially designed RNAs, referred herein as deaminase-recruiting RNAs ("dRNAs") or ADAR-recruiting RNAs ("arRNAs"), to edit target RNAs in a host cell. Without being bound by any theory or hypothesis, the dRNA acts through hybridizing to its target RNA in a sequence-specific fashion to form a double-stranded RNA, which recruits an Adenosine Deaminase Acting on RNA (ADAR) to deaminate a target adenosine in the target RNA. As such, efficient RNA editing can be achieved in some embodiments without ectopic or overexpression of the ADAR proteins in the host cell.

The RNA editing methods described herein do not use fusion proteins comprising an ADAR and a protein that specifically binds to a guide nucleic acid, such as Cas. The deaminase-recruiting RNAs ("dRNA") described herein do not comprise crRNA, tracrRNA or gRNA used in the CRISPR/Cas system. The dRNA does not comprise an ADAR-recruiting domainIn some embodiments the dRNA does not comprise chemical modification(s). In some embodiments, the dRNA can be expressed from a plasmid or a viral vector, or synthesized as an oligonucleotide, which could achieve desirable editing efficiency.

The LEAPER methods described herein have manageable off-target rates on the targeted transcripts and rare global off-targets. Inventors have used the LEAPER method to restore p53 function by repairing a specific cancer-relevant point mutation. The LEAPER methods described herein can also be applied to a broad spectrum of cell types including multiple human primary cells, and can be used to restore the α-L-iduronidase catalytic activity in Hurler syndrome patient-derived primary fibroblasts without evoking innate immune responses. In some embodiments, the LEAPER method involves a single molecule (*i.e.*, dRNA) system. The LEAPER methods described herein enable precise and efficient RNA editing, which offers transformative potential for basic research and therapeutics.

### Definitions

The present invention will be described with respect to particular embodiments and with reference to certain drawings. Any reference signs in the claims shall not be construed as limiting the scope. Where the term "comprising" is used in the present description and claims, it does not exclude other elements or steps. Where an indefinite or definite article is used when referring to a singular noun e.g. "a" or "an", "the", this includes a plural of that noun unless something else is specifically stated. For the recitation of numeric ranges of nucleotides herein, each intervening number there between, is explicitly contemplated. For example, for the range of 40-260 nucleotides, any integer of nucleotides between 40 and 260 nucleotides is contemplated in addition to the numbers of 40 nucleotides and 260 nucleotides.

The following terms or definitions are provided solely to aid in the understanding of the invention. Unless specifically defined herein, all terms used herein have the same meaning as they would to one skilled in the art of the present invention. Practitioners are particularly directed to Sambrook et al., Molecular Cloning: A Laboratory Manual, 2nd ed., Cold Spring Harbor Press, Plainsview, New York (1989); and Ausubel et al., Current Protocols in Molecular Biology (Supplement 47), John Wiley & Sons, New York (1999), for definitions and terms of the art. The definitions provided herein should not be construed to have a scope less than understood by a person of ordinary skill in the art.

The terms "deaminase-recruiting RNA," "dRNA," "ADAR-recruiting RNA" and "arRNA" are used herein interchangeably to refer to an engineered RNA capable of recruiting an ADAR to deaminate a target adenosine in an RNA.

The terms "polynucleotide", "nucleotide sequence" and "nucleic acid" are used interchangeably. They refer to a polymeric form of nucleotides of any length, either deoxyribonucleotides or ribonucleotides, or analogs thereof.

The terms "adenine", "guanine", "cytosine", "thymine", "uracil" and "hypoxanthine" as used herein refer to the nucleobases as such. The terms "adenosine", "guanosine", "cytidine", "thymidine", "uridine" and "inosine", refer to the nucleobases linked to the ribose or deoxyribose sugar moiety. The term "nucleoside" refers to the nucleobase linked to the ribose or deoxyribose. The term "nucleotide" refers to the respective nucleobase-ribosyl-phosphate or nucleobase-deoxyribosyl-phosphate. Sometimes the terms adenosine and adenine (with the abbreviation, "A"), guanosine and guanine (with the abbreviation, "G"), cytosine and cytidine (with the abbreviation, "C"), uracil and uridine (with the abbreviation, "U"), thymine and thymidine (with the abbreviation, "T"), inosine and hypo-xanthine (with the abbreviation, "I"), are used interchangeably to refer to the corresponding nucleobase, nucleoside or nucleotide. Sometimes the terms nucleobase, nucleoside and nucleotide are used interchangeably, unless the context clearly requires differently.

In the context of the present application, "target RNA" refers to an RNA sequence to which a deaminase-recruiting RNA sequence is designed to have perfect complementarity or substantial complementarity, and hybridization between the target sequence and the dRNA forms a double stranded RNA (dsRNA) region containing a target adenosine, which recruits an adenosine deaminase acting on RNA (ADAR) that deaminates the target adenosine. In some embodiments, the ADAR is naturally present in a host cell, such as a eukaryotic cell (preferably, a mammalian cell, more preferably, a human cell). In some embodiments, the ADAR is introduced into the host cell.

As used herein, "complementarity" refers to the ability of a nucleic acid to form hydrogen bond(s) with another nucleic acid by traditional Watson-Crick base-pairing. A percent complementarity indicates the percentage of residues in a nucleic acid molecule which can form hydrogen bonds (i.e., Watson-Crick base pairing) with a second nucleic acid (e.g., about 5, 6, 7, 8, 9, 10 out of 10, being about 50%, 60%, 70%, 80%, 90%, and 100% complementary respectively). "Perfectly complementary" means that all the contiguous residues of a nucleic acid sequence form hydrogen bonds with the same number of contiguous residues in a second nucleic acid sequence. "Substantially complementary" as used herein refers to a degree of complementarity that is at least about any one of 70%, 75%, 80%, 85%, 90%, 95%, 97%, 98%, 99%, or 100% over a region of about 40, 50, 60, 70, 80, 100, 150, 200, 250 or more nucleotides, or refers to two nucleic acids that hybridize under stringent conditions.

As used herein, "stringent conditions" for hybridization refer to conditions under which a nucleic acid having complementarity to a target sequence predominantly hybridizes with the target sequence, and substantially does not hybridize to non-target sequences. Stringent conditions are generally sequence-dependent, and vary depending on a number of factors. In general, the longer the sequence, the higher the temperature at which the sequence specifically hybridizes to its target sequence. Non-limiting examples of stringent conditions are described in detail in Tijssen (1993), Laboratory Techniques In Biochemistry And Molecular Biology- Hybridization With Nucleic Acid Probes Part I, Second Chapter "Overview of principles of hybridization and the strategy of nucleic acid probe assay", Elsevier, N,Y.

"Hybridization" refers to a reaction in which one or more polynucleotides react to form a complex that is stabilized via hydrogen bonding between the bases of the nucleotide residues. The hydrogen bonding may occur by Watson Crick base pairing, Hoogstein binding, or in any other sequence specific manner. A sequence capable of hybridizing with a given sequence is referred to as the "complement" of the given sequence.

As used herein, the terms "cell", "cell line", and "cell culture" are used interchangeably and all such designations include progeny. It is understood that all progeny may not be precisely identical in DNA content, due to deliberate or inadvertent mutations. Variant progeny that have the same function or biological activity as the original cells are included.

## Methods of RNA editing

There is provided a method for editing a target RNA in a host cell (e.g., eukaryotic cell), comprising introducing a deaminase-recruiting RNA (dRNA) or a construct encoding the dRNA into the host cell, wherein the dRNA comprises a complementary RNA sequence that hybridizes to the target RNA and a cytidine mismatch directly opposite the target adenosine in the target RNA, wherein the cytidine mismatch is located at least 20 nucleotides away from the 3' end of the complementary sequence, and at least 5 nucleotides away from the 5' end of the complementary sequence in the dRNA, wherein the dRNA is 50 to 260 nucleotides in length and does not comprise a chemically modified nucleotide, and wherein the dRNA is capable of recruiting an adenosine deaminase acting on RNA 1 or 2 (ADAR1 or ADAR2) to deaminate a target adenosine (A) in the target RNA, and the dRNA does not comprise an ADAR-recruiting domain.

In some embodiments, the method is for editing a target RNA in a host cell (*e.g.*, eukaryotic cell), comprising introducing a dRNA or a construct encoding the dRNA into the host cell, wherein the dRNA comprises a complementary RNA sequence that hybridizes to the target RNA, and wherein the dRNA recruits an endogenously expressed ADAR of the host cell to deaminate a target A in the target RNA. In some embodiments, the method does not comprise introducing any protein or construct encoding a protein (*e.g.*, Cas, ADAR or a fusion protein of ADAR and Cas) to the host cell.

In some embodiments, the method for editing a target RNA in a host cell (e.g., eukaryotic cell), comprises introducing: (a) a dRNA or a construct encoding the dRNA, and (b) an ADAR or a construct encoding the ADAR into the host cell, wherein the dRNA comprises a complementary RNA sequence that hybridizes to the target RNA, and wherein the dRNA recruits the ADAR to deaminate a target A in the target RNA. In some embodiments, the ADAR is an endogenously encoded ADAR of the host cell, wherein introduction of the ADAR comprises over-expressing the ADAR in the host cell. In some embodiments, the ADAR is exogenous to the host cell. In some embodiments, the construct encoding the ADAR is a vector, such as a plasmid, or a viral vector (*e.g.*, a lentiviral vector).

In some embodiments, the method is for editing a plurality (*e.g.*, at least about 2, 3, 4, 5, 10, 20, 50, 100 or more) of target RNAs in a host cell (*e.g.,* an eukaryotic cell), comprising introducing a plurality of dRNAs or constructs encoding the plurality of dRNAs into the host cell, wherein each dRNA comprises a complementary RNA sequence that hybridizes to a corresponding target RNA in the plurality of target RNAs, and wherein each dRNA is capable of recruiting an ADAR to deaminate a target A in the corresponding target RNA.

In some embodiments, the method is for editing a plurality (*e.g.*, at least about 2, 3, 4, 5, 10, 20, 50, 100 or more) of target RNAs in a host cell (*e.g.,* an eukaryotic cell), comprising introducing a plurality of dRNAs or constructs encoding the plurality of dRNAs into the host cell, wherein each dRNA comprises a complementary RNA sequence that hybridizes to a corresponding target RNA in the plurality of target RNAs, and wherein each dRNA recruits an endogenously expressed ADAR to deaminate a target A in the corresponding target RNA.

In some embodiments, the method is for editing a plurality (*e.g.*, at least about 2, 3, 4, 5, 10, 20, 50, 100, 1000 or more) of target RNAs in a host cell (*e.g.,* a eukaryotic cell), comprising introducing: (a) a plurality of dRNAs or constructs encoding the plurality of dRNAs, and (b) an ADAR or a construct encoding ADAR into the host cell, wherein each dRNA comprises a complementary RNA sequence that hybridizes to a corresponding target RNA in the plurality of target RNAs, and wherein each dRNA recruits the ADAR to deaminate a target A in the corresponding target RNA.

In one aspect, the present application provides a method for editing a plurality of RNAs in host cells by introducing a plurality of the deaminase-recruiting RNAs, one or more constructs encoding the deaminase-recruiting RNAs, or a library described herein, into the host cells.

In certain embodiments, the method for editing on a target RNA comprises introducing multiple deaminase-recruiting RNAs or one or more constructs comprising the multiple deaminase-recruiting RNAs into host cells to recruit adenosine deaminase acting on RNA (ADAR) to perform deamination reaction on one or more target adenosines in one or more target RNAs, wherein each deaminase-recruiting RNA comprises a RNA sequences complementary to a corresponding target RNA.

In certain embodiments according to any one of the methods described herein, the dRNA comprises more than about any one of 40, 45, 50, 55, 60, 65, 70, 75, 80, 90, 100, 110, 120, 130, 140, 150, 160, 170, 180, 190, 200, 210, 220, 230, 240, or 250 nucleotides. In certain embodiments, the dRNA is about any one of 50-240, 60-230, 65-220, 70-220, 70-210, 70-200, 70-190, 70-180, 70-170, 70-160, 70-150, 70-140, 70-130, 70-120, 70-110, 70-100, 70-90, 70-80, 75-200, 80-190, 85-180, 90-170, 95-160, 100-200, 100-150, 100-175, 110-200, 110-175, 110-150, or 105-140 nucleotides in length. In some embodiments, the dRNA is about 71 nucleotides long. In some embodiments, the dRNA is about 111 nucleotides long.

In the methods described herein, the dRNA does not comprise an ADAR-recruiting domain. "ADAR-recruiting domain" can be a nucleotide sequence or structure that binds at high affinity to ADAR, or a nucleotide sequence that binds to a binding partner fused to ADAR in an engineered ADAR construct. Exemplary ADAR-recruiting domains include, but are not limited to, GluR-2, GluR-B (R/G), GluR-B (Q/R), GluR-6 (R/G), 5HT2C, and FlnA (Q/R) domain; *see,* for example, Wahlstedt, Helene, and Marie, "Site-selective versus promiscuous A-to-I editing." Wiley Interdisciplinary Reviews: RNA 2.6 (2011): 761-771. In some embodiments, the dRNA does not comprise a double-stranded portion. In some embodiments, the dRNA does not comprise a hairpin, such as MS2 stem loop. In some embodiments, the dRNA is single stranded. In some embodiments, the ADAR not comprise a DSB-binding domain. In some embodiments, the dRNA consists of (or consists essentially of) the complementary RNA sequence.

In certain embodiments according to any one of the methods described herein, the dRNA does not comprise chemical modifications. In some embodiments, the dRNA does not comprise a chemically modified nucleotide, such as 2'-O-methyl nucleotide or a nucleotide having a phosphorothioate linkage. In some embodiments, the dRNA comprises 2'-O-methyl and phosphorothioate linkage modifications only at the first three and last three residues. In some embodiments, the dRNA is not an antisense oligonucleotide (ASO).

In certain embodiments according to any one of the methods described herein, the host cell is a prokaryotic cell. In some embodiments, the host cell is a eukaryotic cell. In some embodiments, the host cell is a mammalian cell. In some embodiments, the host cell is a human cell. In some embodiments, the host cell is a murine cell. In some embodiments, the host cell is a plant cell or a fungal cell.

In some embodiments according to any one of the methods described herein, the host cell is a cell line, such as HEK293T, HT29, A549, HepG2, RD, SF268, SW13 and HeLa cell. In some embodiments, the host cell is a primary cell, such as fibroblast, epithelial, or immune cell. In some embodiments, the host cell is a T cell. In some embodiments, the host cell is a post-mitosis cell. In some embodiments, the host cell is a cell of the central nervous system (CNS), such as a brain cell, *e.g.,* a cerebellum cell.

In some embodiments, the method of editing a target RNA in a primary host cell (*e.g.,* T cell or a CNS cell) comprises introducing a dRNA or a construct encoding the dRNA into the host cell, wherein the dRNA comprises a complementary RNA sequence that hybridizes to the target RNA, and wherein the dRNA recruits an endogenously expressed ADAR of the host cell to deaminate a target A in the target RNA.

In certain embodiments according to any one of the methods described herein, the ADAR is endogenous to the host cell. In some embodiments, the adenosine deaminase acting on RNA (ADAR) is naturally or endogenously present in the host cell, for example, naturally or endogenously present in the eukaryotic cell. In some embodiments, the ADAR is endogenously expressed by the host cell. In certain embodiments, the ADAR is exogenously introduced into the host cell. The ADAR is ADAR1 and/or ADAR2. In certain embodiments, the ADAR is one or more ADARs selected from the group consisting of hADAR1, hADAR2, mouse ADAR1 and ADAR2. In some embodiments, the ADAR is ADAR1, such as p110 isoform of ADAR1 ("ADAR1$^{p110}$") and/or p150 isoform of ADAR1 ("ADAR1$^{p150}$"). In some embodiments, the ADAR is ADAR2. In some embodiments, the ADAR is an ADAR2 expressed by the host cell, *e.g.*, ADAR2 expressed by cerebellum cells.

In some embodiments, the ADAR is an ADAR exogenous to the host cell. In some embodiments, the ADAR is a hyperactive mutant of a naturally occurring ADAR. In some embodiments, the ADAR is ADAR1 comprising an E1008Q mutation. In some embodiments, the ADAR is not a fusion protein comprising a binding domain. In some embodiments, the ADAR does not comprise an engineered double-strand nucleic acid-binding domain. In some embodiments, the ADAR does not comprise a MCP domain that binds to MS2 hairpin that is fused to the complementary RNA sequence in the dRNA. In some embodiments, the ADAR does not comprise a DSB-binding domain.

In some embodiments according to any one of the methods described herein, the host cell has high expression level of ADAR1 (such as ADAR1$^{p110}$ and/or ADAR1$^{p150}$), *e.g.*, at least about any one of 10%, 20%, 50%, 100%, 2x, 3x, 5x, or more relative to the protein expression level of $\beta$-tubulin. In some embodiments, the host cell has high expression level of ADAR2, *e.g.*, at least about any one of 10%, 20%, 50%, 100%, 2x, 3x, 5x, or more relative to the protein expression level of $\beta$-tubulin. In some embodiments, the host cell has low expression level of ADAR3, *e.g.,* no more than about any one of 5x, 3x, 2x, 100%, 50%, 20% or less relative to the protein expression level of $\beta$-tubulin.

In certain embodiments according to any one of the methods described herein, the complementary RNA sequence comprises a cytidine, preferably further adenosine or uridine, directly opposite the target A in the target RNA. In some embodiments, complementary RNA sequence comprises a cytidine mismatch directly opposite the target A in the target RNA. In some embodiments, the cytidine mismatch is located at least 10, 15, 20, 25, 30, or more nucleotides, away from the 5' end of the complementary RNA sequence. In some embodiments, the cytidine mismatch is located at least 25, 30, 35, or more nucleotides, away from the 3' end of the complementary RNA sequence. The cytidine mismatch is located at least 20 nucleotides (e.g., at least 25, 30, 35, or more nucleotides) away from the 3' end and at least 5 nucleotides (e.g., at least 10, 15, 20, 25, 30, or more nucleotides) away from the 5' end of the complementary RNA sequence. In some embodiments, the cytidine mismatch is located in the center of the complementary RNA sequence. In some embodiments, the cytidine mismatch is located within 20 nucleotides *(e.g.,* 15, 10, 9, 8, 7, 6, 5, 4, 3, 2, or 1 nucleotide) of the center of the complementary sequence in the dRNA.

In certain embodiments according to any one of the methods described herein, the complementary RNA sequence further comprises one or more guanosine(s), such as 1, 2, 3, 4, 5, 6, or more Gs, that is each directly opposite a non-target adenosine in the target RNA. In some embodiments, the complementary RNA sequence comprises two or more consecutive mismatch nucleotides (*e.g.*, 2, 3, 4, 5, or more mismatch nucleotides) opposite a non-target adenosine in the target RNA. In some embodiments, the target RNA comprises no more than about 20 non-target As, such as no more than about any one of 15, 10, 9, 8, 7, 6, 5, 4, 3, 2, or 1 non-target A. The Gs and consecutive mismatch nucleotides opposite non-target As may reduce off-target editing effects by ADAR.

In certain embodiments according to any one of the methods described herein, the 5' nearest neighbor of the target A is a nucleotide selected from U, C, A and G with the preference U>C≈A>G and the 3' nearest neighbor of the target A is a nucleotide selected from G, C, A and U with the preference G>C>A≈U. In certain embodiments, the target A is in a three-base motif selected from the group consisting of UAG, UAC, UAA, UAU, CAG, CAC, CAA, CAU, AAG, AAC, AAA, AAU, GAG, GAC, GAA and GAU in the target RNA. In certain embodiments, the three-base motif is UAG, and the dRNA comprises an A directly opposite the U in the three-base motif, a C directly opposite the target A, and a C, G or U directly opposite the G in the three-base motif. In certain embodiments, the three-base motif is UAG in the target RNA, and the dRNA comprises ACC, ACG or ACU that is opposite the UAG of the target RNA. In certain embodiments, the three-base motif is UAG in the target RNA, and the dRNA comprises ACC that is opposite the UAG of the target RNA.

In certain embodiments according to any one of the methods described herein, the target RNA is any one selected from the group consisting of a pre-messenger RNA, a messenger RNA, a ribosomal RNA, a transfer RNA, a long non-coding RNA and a small RNA (e.g., miRNA). In some embodiments, the target RNA is a pre-messenger RNA. In some embodiments, the target RNA is a messenger RNA.

In certain embodiments according to any one of the methods described herein, the method further comprises introducing an inhibitor of ADAR3 to the host cell. In some embodiments, the inhibitor of ADAR3 is an RNAi against ADAR3, such as a shRNA against ADAR3 or a siRNA against ADAR3. In some embodiments, the method further comprises introducing a stimulator of interferon to the host cell. In some embodiments, the ADAR is inducible by interferon, for example, the ADAR is ADAR$^{p150}$. In some embodiments, the stimulator of interferon is IFN$\alpha$. In some embodiments, the inhibitor of ADAR3 and/or the stimulator of interferon are encoded by the same construct (*e.g.*, vector) that encodes the dRNA.

In certain embodiments according to any one of the methods described herein, the efficiency of editing of the target RNA is at least about 5%, such as at least about any one of 6%, 7%, 8%, 9%, 10%, 15%, 20%, 25%, 30%, 35%, 40%, 45%, 50%, 55%, 60%, 65%, or higher. In some embodiments, the efficiency of editing of the target RNA is at least about 5% *(e.g.,* at least about 7%) *in* vivo, e.g., in an animal. In some embodiments, the efficiency of editing of the target RNA is at least about 10% *(e.g.,* at least about 15%) in a cell *in vitro* or *ex vivo.* In some embodiments, the efficiency of editing is determined by Sanger sequencing. In some embodiments, the efficiency of editing is determined by next-generation sequencing.

In certain embodiments according to any one of the methods described herein, the method has low off-target editing rate. In some embodiments, the method has lower than about 1% *(e.g.,* no more than about any one of 0.5%, 0.1%, 0.05%, 0.01%, 0.001% or lower) editing efficiency on non-target As in the target RNA. In some embodiments, the method does not edit non-target As in the target RNA. In some embodiments, the method has lower than about 0.1% *(e.g.,* no more than about any one of 0.05%, 0.01%, 0.005%, 0.001%, 0.0001% or lower) editing efficiency on As in non-target RNA.

In certain embodiments according to any one of the methods described herein, the method does not induce immune response, such as innate immune response. In some embodiments, the method does not induce interferon and/or interleukin expression in the host cell. In some embodiments, the method does not induce IFN-β and/or IL-6 expression in the host cell.

Also provided are edited RNA or host cells having an edited RNA produced by any one of the methods described herein. In some embodiments, the edited RNA comprises an inosine. In some embodiments, the host cell comprises an RNA having a missense mutation, an early stop codon, an alternative splice site, or an aberrant splice site. In some embodiments, the host cell comprises a mutant, truncated, or misfolded protein.

"Host cell" as described herein refers to any cell type that can be used as a host cell provided it can be modified as described herein. For example, the host cell may be a host cell with endogenously expressed adenosine deaminase acting on RNA (ADAR), or may be a host cell into which an adenosine deaminase acting on RNA (ADAR) is introduced by a known method in the art. For example, the host cell may be a prokaryotic cell, a eukaryotic cell or a plant cell. In some embodiments, the host cell is derived from a pre-established cell line, such as mammalian cell lines including human cell lines or non-human cell lines. In some embodiments, the host cell is derived from an individual, such as a human individual.

"Introducing" or "introduction" used herein means delivering one or more polynucleotides, such as dRNAs or one or more constructs including vectors as described herein, one or more transcripts thereof, to a host cell. The invention serves as a basic platform for enabling targeted editing of RNA, for example, pre-messenger RNA, a messenger RNA, a ribosomal RNA, a transfer RNA, a long non-coding RNA and a small RNA (such as miRNA). The methods of the present application can employ many delivery systems, including but not limited to, viral, liposome, electroporation, microinjection and conjugation, to achieve the introduction of the dRNA or construct as described herein into a host cell. Conventional viral and non-viral based gene transfer methods can be used to introduce nucleic acids into mammalian cells or target tissues. Such methods can be used to administer nucleic acids encoding dRNA of the present application to cells in culture, or in a host organism. Non-viral vector delivery systems include DNA plasmids, RNA (e.g. a transcript of a construct described herein), naked nucleic acid, and nucleic acid complexed with a delivery vehicle, such as a liposome. Viral vector delivery systems include DNA and RNA viruses, which have either episomal or integrated genomes for delivery to the host cell.

Methods of non-viral delivery of nucleic acids include lipofection, nucleofection, microinjection, biolistics, virosomes, liposomes, immunoliposomes, polycation or lipid: nucleic acid conjugates, electroporation, nanoparticles, exosomes, microvesicles, or gene-gun, naked DNA and artificial virions.

The use of RNA or DNA viral based systems for the delivery of nucleic acids has high efficiency in targeting a virus to specific cells and trafficking the viral payload to the cellular nuclei.

In certain embodiments according to any one of the methods described herein, the method comprises introducing a viral vector (such as lentiviral vector) encoding the dRNA to the host cell. In some embodiments, the viral vector is an AAV, e.g., AAV8. In some embodiments, the method comprises introducing a plasmid encoding the dRNA to the host cell. In some embodiments, the method comprises introducing *(e.g.,* by electroporation) the dRNA *(e.g.,* synthetic dRNA) into the host cell. In some embodiments, the method comprises transfection of the dRNA into the host cell.

After deamination, modification of the target RNA and/or the protein encoded by the target RNA, can be determined using different methods depending on the positions of the targeted adenosines in the target RNA. For example, in order to determine whether "A" has been edited to "I" in the target RNA, RNA sequencing methods known in the art can be used to detect the modification of the RNA sequence. When the target adenosine is located in the coding region of an mRNA, the RNA editing may cause changes to the amino acid sequence encoded by the mRNA. For example, point mutations may be introduced to the mRNA of an innate or acquired point mutation in the mRNA may be reversed to yield wild-type gene product(s) because of the conversion of "A" to "I". Amino acid sequencing by methods known in the art can be used to find any changes of amino acid residues in the encoded protein. Modifications of a stop codon may be determined by assessing the presence of a functional, elongated, truncated, full-length and/or wild-type protein. For example, when the target adenosine is located in a UGA, UAG, or UAA stop codon, modification of the target A (UGA or UAG) or As (UAA) may create a read-through mutation and/or an elongated protein, or a truncated protein encoded by the target RNA may be reversed to create a functional, full-length and/or wild-type protein. Editing of a target RNA may also generate an aberrant splice site, and/or alternative splice site in the target RNA, thus leading to an elongated, truncated, or misfolded protein, or an aberrant splicing or alternative splicing site encoded in the target RNA may be reversed to create a functional, correctly-folding, full-length and/or wild-type protein. In some embodiments, the present application contemplates editing of both innate and acquired genetic changes, for example, missense mutation, early stop codon, aberrant splicing or alternative splicing site encoded by a target RNA. Using known methods to assess the function of the protein encoded by the target RNA can find out whether the RNA editing achieves the desired effects. Because deamination of the adenosine (A) to an inosine (I) may correct a mutated A at the target position in a mutant RNA encoding a protein, identification of the deamination into inosine may provide assessment on whether a functional protein is present, or whether a disease or drug resistance-associated RNA caused by the presence of a mutated adenosine is reversed or partly reversed. Similarly, because deamination of the adenosine (A) to an inosine (I) may introduce a point mutation in the resulting protein, identification of the deamination into inosine may provide a functional indication for identifying a cause of disease or a relevant factor of a disease.

When the presence of a target adenosine causes aberrant splicing, the read-out may be the assessment of occurrence and frequency of aberrant splicing. On the other hand, when the deamination of a target adenosine is desirable to introduce a splice site, then similar approaches can be used to check whether the required type of splicing occurs. An exemplary suitable method to identify the presence of an inosine after deamination of the target adenosine is RT-PCR and sequencing, using methods that are well-known to the person skilled in the art.

The effects of deamination of target adenosine(s) include, for example, point mutation, early stop codon, aberrant splice site, alternative splice site and misfolding of the resulting protein. These effects may induce structural and functional changes of RNAs and/or proteins associated with diseases, whether they are genetically inherited or caused by acquired genetic mutations, or may induce structural and functional changes of RNAs and/or proteins associated with occurrence of drug resistance. Hence, the dRNAs, the constructs encoding the dRNAs, and the RNA editing methods of present application can be used in prevention or treatment of hereditary genetic diseases or conditions, or diseases or conditions associated with acquired genetic mutations by changing the structure and/or function of the disease-associated RNAs and/or proteins.

In some embodiments, the target RNA is a regulatory RNA. In some embodiments, the target RNA to be edited is a ribosomal RNA, a transfer RNA, a long non-coding RNA or a small RNA (*e.g.*, miRNA, pri-miRNA, pre-miRNA, piRNA, siRNA, snoRNA, snRNA, exRNA or scaRNA). The effects of deamination of the target adenosines include, for example, structural and functional changes of the ribosomal RNA, transfer RNA, long non-coding RNA or small RNA (*e.g.*, miRNA), including changes of three-dimensional structure and/or loss of function or gain of function of the target RNA. In some embodiments, deamination of the target As in the target RNA changes the expression level of one or more downstream molecules (e.g., protein, RNA and/or metabolites) of the target RNA. Changes of the expression level of the downstream molecules can be increase or decrease in the expression level.

Some embodiments of the present application involve multiplex editing of target RNAs in host cells, which are useful for screening different variants of a target gene or different genes in the host cells. In some embodiments, wherein the method comprises introducing a plurality of dRNAs to the host cells, at least two of the dRNAs of the plurality of dRNAs have different sequences and/or have different target RNAs. In some embodiments, each dRNA has a different sequence and/or different target RNA. In some embodiments, the method generates a plurality (*e.g.*, at least 2, 3, 5, 10, 50, 100, 1000 or more) of modifications in a single target RNA in the host cells. In some embodiments, the method generates a modification in a plurality (*e.g.,* at least 2, 3, 5, 10, 50, 100, 1000 or more) of target RNAs in the host cells. In some embodiments, the method comprises editing a plurality of target RNAs in a plurality of populations of host cells. In some embodiments, each population of host cells receive a different dRNA or a dRNAs having a different target RNA from the other populations of host cells.

## Deaminase-recruiting RNA, construct, and library

In one aspect, the present application provides a deaminase-recruiting RNA useful for any one of the methods described herein. Any one of the dRNAs described in this section may be used in the methods of RNA editing and treatment described herein. It is intended that any of the features and parameters described herein for dRNAs can be combined with each other, as if each and every combination is individually described. The dRNAs described herein do not comprise a tracrRNA, crRNA or gRNA used in a CRISPR/Cas system.

In some embodiments, there is provided a deaminase-recruiting RNA (dRNA) for deamination of a target adenosine in a target RNA by recruiting an ADAR, comprising a complementary RNA sequence that hybridizes to the target RNA.

In one aspect, the present provides a construct comprising any one of the deaminase-recruiting RNAs described herein. In certain embodiments, the construct is a viral vector (such as a lentivirus vector) or a plasmid. In some embodiments, the viral vector is an AAV, such as AAV8. In some embodiments, the construct encodes a single dRNA. In some embodiments, the construct encodes a plurality (*e.g.*, about any one of 1, 2, 3, 4, 5, 10, 20 or more) dRNAs.

In one aspect, the present application provides a library comprising a plurality of the deaminase-recruiting RNAs or a plurality of the constructs described herein.

In one aspect, the present application provides a composition or a host cell comprising the deaminase-recruiting RNA or the construct described herein. In certain embodiments, the host cell is a prokaryotic cell or a eukaryotic cell. Preferably, the host cell is a mammalian cell. Most preferably, the host cell is a human cell.

In the dRNAs, constructs, libraries or compositions described herein, the complementary RNA sequence comprises a cytidine, preferably further adenosine or uridine, directly opposite the target adenosine to be edited in the target RNA. In certain embodiments, the complementary RNA sequence further comprises one or more guanosine(s) that is each directly opposite a non-target adenosine in the target RNA. In certain embodiments, the 5' nearest neighbor of the target A is a nucleotide selected from U, C, A and G with the preference U>C≈A>G and the 3' nearest neighbor of the target A is a nucleotide selected from G, C, A and U with the preference G>C>A≈U. In some embodiments, the 5' nearest neighbor of the target A is U. In some embodiments, the 5' nearest neighbor of the target A is C or A. In some embodiments, the 3' nearest neighbor of the target A is G. In some embodiments, the 3' nearest neighbor of the target A is C.

In certain embodiments according to any one of the dRNAs, constructs, libraries or compositions described herein, the target A is in a three-base motif selected from the group consisting of UAG, UAC, UAA, UAU, CAG, CAC, CAA, CAU, AAG, AAC, AAA, AAU, GAG, GAC, GAA and GAU in the target RNA. In certain embodiments, the three-base motif is UAG, and the dRNA comprises an A directly opposite the U in the three-base motif, a C directly opposite the target A, and a C, G or U directly opposite the G in the three-base motif. In certain embodiments, the three-base motif is UAG in the target RNA, and the dRNA comprises ACC, ACG or ACU that is opposite the UAG of the target RNA.

The dRNA comprises a cytidine mismatch directly opposite the target A in the target RNA. In some embodiments, the cytidine mismatch is close to the center of the complementary RNA sequence, such as within 20, 15, 10, 5, 4, 3, 2, or 1 nucleotide away from the center of the complementary RNA sequence. In some embodiments, the cytidine mismatch is at least 5 nucleotides away from the 5' end of the complementary RNA sequence. In some embodiments, the cytidine mismatch is at least 20 nucleotides away from the 3' end of the complementary RNA sequence.

In certain embodiments according to any one of the dRNAs, constructs, libraries or compositions described herein, the dRNA comprises more than about any one of 40, 45, 50, 55, 60, 65, 70, 75, 80, 90, 100, 110, 120, 130, 140, 150, 160, 170, 180, 190, 200, 210, 220, 230, 240, or 250 nucleotides. In certain embodiments, the dRNA is about any one of 50-240, 60-230, 65-220, 70-210, 70-200, 70-190, 70-180, 70-170, 70-160, 70-150, 70-140, 70-130, 70-120, 70-110, 70-100, 70-90, 70-80, 75-200, 80-190, 85-180, 90-170, 95-160, 100-150 or 105-140 nucleotides in length.

The dRNA comprises the nucleic acid sequence of any one of SEQ ID NOs: 25-44, 142-205, 341-342.

The dRNA of the present application comprises a complementary RNA sequence that hybridizes to the target RNA. The complementary RNA sequence is perfectly complementary or substantially complementarity to the target RNA to allow hybridization of the complementary RNA sequence to the target RNA. In some embodiments, the complementary RNA sequence has 100% sequence complementarity as the target RNA. In some embodiments, the complementary RNA sequence is at least about any one of 70%, 80%, 85%, 90%, 95%, 96%, 97%, 98%, or 99% or more complementary to over a continuous stretch of at least about any one of 20, 40, 60, 80, 100, 150, 200, or more nucleotides in the target RNA. In some embodiments, the dsRNA formed by hybridization between the complementary RNA sequence and the target RNA has one or more (*e.g.*, 1, 2, 3, 4, 5, 6, 7, 8, 9, 10 or more) non-Watson-Crick base pairs (*i.e.*, mismatches).

ADAR, for example, human ADAR enzymes edit double stranded RNA (dsRNA) structures with varying specificity, depending on a number of factors. One important factor is the degree of complementarity of the two strands making up the dsRNA sequence. Perfect complementarity of between the dRNA and the target RNA usually causes the catalytic domain of ADAR to deaminate adenosines in a non-discriminative manner. The specificity and efficiency of ADAR can be modified by introducing mismatches in the dsRNA region. For example, A-C mismatch is preferably recommended to increase the specificity and efficiency of deamination of the adenosine to be edited. Conversely, at the other A (adenosine) positions than the target A (*i.e.,* "non-target A"), the G-A mismatch can reduce off-target editing. Perfect complementarity is not necessarily required for a dsRNA formation between the dRNA and its target RNA, provided there is substantial complementarity for hybridization and formation of the dsRNA between the dRNA and the target RNA. In some embodiments, the dRNA sequence or single-stranded RNA region thereof has at least about any one of 70%, 80%, 85%, 90%, 95%, 96%, 97%, 98%, or 99% of sequence complementarity to the target RNA, when optimally aligned. Optimal alignment may be determined with the use of any suitable algorithm for aligning sequences, non-limiting examples of which include the Smith-Waterman algorithm, the Needleman-Wimsch algorithm, algorithms based on the Burrows-Wheeler Transform (e.g. the Burrows Wheeler Aligner).

The nucleotides neighboring the target adenosine also affect the specificity and efficiency of deamination. For example, the 5' nearest neighbor of the target adenosine to be edited in the target RNA sequence has the preference U>C≈A>G and the 3' nearest neighbor of the target adenosine to be edited in the target RNA sequence has the preference G>C>A≈U in terms of specificity and efficiency of deamination of adenosine. In some embodiments, when the target adenosine may be in a three-base motif selected from the group consisting of UAG, UAC, UAA, UAU, CAG, CAC, CAA, CAU, AAG, AAC, AAA, AAU, GAG, GAC, GAA and GAU in the target RNA, the specificity and efficiency of deamination of adenosine are higher than adenosines in other three-base motifs. In some embodiments, where the target adenosine to be edited is in the three-base motif UAG, UAC, UAA, UAU, CAG, CAC, AAG, AAC or AAA, the efficiency of deamination of adenosine is much higher than adenosines in other motifs. With respect to the same three-base motif, different designs of dRNA may also lead to different deamination efficiency. Taking the three-base motif UAG as an example, in some embodiments, when the dRNA comprises cytidine (C) directly opposite the target adenosine to be edited, adenosine (A) directly opposite the uridine, and cytidine (C), guanosine (G) or uridine (U) directly opposite the guanosine, the efficiency of deamination of the target adenosine is higher than that using other dRNA sequences. In some embodiments, when the dRNA comprises ACC, ACG or ACU opposite UAG of the target RNA, the editing efficiency of the A in the UAG of the target RNA may reach about 25%-30%.

Besides the target adenosines, there may be one or more adenosines in the target RNA, which are not desirable to be edited. With respect to these adenosines, it is preferable to reduce their editing efficiency as much as possible. It is found by this invention that where guanosine is directly opposite an adenosine in the target RNA, the deamination efficiency is significantly decreased. Therefore, in order to decrease off-target deamination, dRNAs can be designed to comprise one or more guanosines directly opposite one or more adenosine(s) other than the target adenosine to be edited in the target RNA.

The desired level of specificity and efficiency of editing the target RNA sequence may depend on different applications. Following the instructions in the present patent application, those of skill in the art will be capable of designing a dRNA having complementary or substantially complementary sequence to the target RNA sequence according to their needs, and, with some trial and error, obtain their desired results. As used herein, the term "mismatch" refers to opposing nucleotides in a double stranded RNA (dsRNA) which do not form perfect base pairs according to the Watson-Crick base pairing rules. Mismatch base pairs include, for example, G-A, C-A, U-C, A-A, G-G, C-C, U-U base pairs. Taking A-C match as an example, where a target A is to be edited in the target RNA, a dRNA is designed to comprise a C opposite the A to be edited, generating a A-C mismatch in the dsRNA formed by hybridization between the target RNA and dRNA.

In some embodiments, the dsRNA formed by hybridization between the dRNA and the target RNA does not comprise a mismatch. In some embodiments, the dsRNA formed by hybridization between the dRNA and the target RNA comprises one or more, such as any one of 1, 2, 3, 4, 5, 6, 7 or more mismatches (*e.g.*, the same type of different types of mismatches). In some embodiments, the dsRNA formed by hybridization between the dRNA and the target RNA comprises one or more kinds of mismatches, for example, 1, 2, 3, 4, 5, 6, 7 kinds of mismatches selected from the group consisting of G-A, C-A, U-C, A-A, G-G, C-C and U-U.

The mismatch nucleotides in the dsRNA formed by hybridization between the dRNA and the target RNA can form bulges, which can promote the efficiency of editing of the target RNA. There may be one (which is only formed at the target adenosine) or more bulges formed by the mismatches. The additional bulge-inducing mismatches may be upstream and/or downstream of the target adenosine. The bulges may be single-mismatch bulges (caused by one mismatching base pair) or multi-mismatch bulges (caused by more than one consecutive mismatching base pairs, preferably two or three consecutive mismatching base pairs).

The complementary RNA sequence in the dRNA is single-stranded. The dRNA may be entirely single-stranded or have one or more (*e.g.*, 1, 2, 3, or more) double-stranded regions and/or one or more stem loop regions. In some embodiments, the complementary RNA sequence is at least about any one of 40, 45, 50, 55, 60, 65, 70, 75, 80, 90, 100, 110, 120, 130, 140, 150, 160, 170, 180, 190, 200 or more nucleotides. In certain embodiments, the complementary RNA sequence is about any one of 50-240, 60-230, 65-220, 70-220, 70-210, 70-200, 70-190, 70-180, 70-170, 70-160, 70-150, 70-140, 70-130, 70-120, 70-110, 70-100, 70-90, 70-80, 75-200, 80-190, 85-180, 90-170, 95-160, 100-200, 100-150, 100-175, 110-200, 110-175, 110-150, or 105-140 nucleotides in length. In some embodiments, the complementary RNA sequence is about 71 nucleotides long. In some embodiments, the complementary RNA sequence is about 111 nucleotides long.

In some embodiments, the dRNA, apart from the complementary RNA sequence, further comprises regions for stabilizing the dRNA, for example, one or more double-stranded regions and/or stem loop regions. In some embodiments, the double-stranded region or stem loop region of the dRNA comprises no more than about any one of 200, 150, 100, 50, 40, 30, 20, 10 or fewer base-pairs. In some embodiments, the dRNA does not comprise a stem loop or double-stranded region. The dRNA does not comprise an ADAR-recruiting domain.

The dRNA may comprise one or more modifications. In some embodiments, the dRNA has one or more modified nucleotides, including nucleobase modification and/or backbone modification. Exemplary modifications to the RNA include, but are not limited to, phosphorothioate backbone modification, 2'-substitutions in the ribose (such as 2'-O-methyl and 2'-fluoro substitutions), LNA, and L-RNA. In some embodiments, the dRNA does not have modifications to the nucleobase or backbone.

The present application also contemplates a construct comprising the dRNA described herein. The term "construct" as used herein refers to DNA or RNA molecules that comprise a coding nucleotide sequence that can be transcribed into RNAs or expressed into proteins. In some embodiments, the construct contains one or more regulatory elements operably linked to the nucleotide sequence encoding the RNA or protein. When the construct is introduced into a host cell, under suitable conditions, the coding nucleotide sequence in the construct can be transcribed or expressed.

In some embodiments, the construct comprises a promoter that is operably linked, or spatially connected to the coding nucleotide sequence, such that the promoter controls the transcription or expression of the coding nucleotide sequence. A promoter may be positioned 5' (upstream) of a coding nucleotide sequence under its control. The distance between the promoter and the coding sequence may be approximately the same as the distance between that promoter and the gene it controls in the gene from which the promoter is derived. As is known in the art, variation in this distance may be accommodated without loss of promoter function. In some embodiments, the construct comprises a 5' UTR and/or a 3'UTR that regulates the transcription or expression of the coding nucleotide sequence.

In some embodiments, the construct is a vector encoding any one of the dRNAs disclosed in the present application. The term "vector" refers to a nucleic acid molecule capable of transporting another nucleic acid to which it has been linked. Vectors include, but are not limited to, nucleic acid molecules that are single-stranded, double-stranded, or partially double-stranded; nucleic acid molecules that comprise one or more free ends, no free ends (e.g. circular); nucleic acid molecules that comprise DNA, RNA, or both; and other varieties of polynucleotides known in the art. One type of vector is a "plasmid," which refers to a circular double stranded DNA loop into which additional DNA segments can be inserted, such as by standard molecular cloning techniques. Certain vectors are capable of autonomous replication in a host cell into which they are introduced (e.g., bacterial vectors having a bacterial origin of replication and episomal mammalian vectors). Other vectors (e.g., non-episomal mammalian vectors) are integrated into the genome of a host cell upon introduction into the host cell, and thereby are replicated along with the host genome. Moreover, certain vectors are capable of directing the transcription or expression of coding nucleotide sequences to which they are operatively linked. Such vectors are referred to herein as "expression vectors".

Recombinant expression vectors can comprise a nucleic acid of the invention in a form suitable for transcription or expression of the nucleic acid in a host cell. In some embodiments, the recombinant expression vector includes one or more regulatory elements, which may be selected on the basis of the host cells to be used for transcription or expression, which is operatively linked to the nucleic acid sequence to be transcribed or expressed. Within a recombinant expression vector, "operably linked" is intended to mean that the nucleotide sequence of interest is linked to the regulatory element(s) in a manner that allows for expression of the nucleotide sequence (e.g. in an in vitro transcription/translation system or in a host cell when the vector is introduced into the host cell).

In some embodiments, there is provided a construct (*e.g.*, vector, such as viral vector) comprising a nucleotide sequence encoding the dRNA. In some embodiments, there is provided a construct (*e.g.*, vector, such as viral vector) comprising a nucleotide sequence encoding the ADAR. In some embodiments, there is provided a construct comprising a first nucleotide sequence encoding the dRNA and a second nucleotide sequence encoding the ADAR. In some embodiments, the first nucleotide sequence and the second nucleotide sequence are operably linked to the same promoter. In some embodiments, the first nucleotide sequence and the second nucleotide sequence are operably linked to different promoters. In some embodiments, the promoter is inducible. In some embodiments, the construct does not encode for the ADAR. In some embodiments, the vector further comprises nucleic acid sequence(s) encoding an inhibitor of ADAR3 (*e.g.*, ADAR3 shRNA or siRNA) and/or a stimulator of interferon (*e.g.*, IFN-α). In some embodiments, the construct is an AAV, such as AAV8.

## Compositions

Also provided herein are compositions (such as pharmaceutical compositions) comprising any one of the dRNAs, constructs, libraries, or host cells having edited RNA as described herein.

In some embodiments, there is provided a pharmaceutical composition comprising any one of the dRNAs or constructs encoding the dRNA described herein, and a pharmaceutically acceptable carrier, excipient or stabilizer. Exemplary pharmaceutically acceptable carriers, excipients and stabilizers have been described, for example, in Remington's Pharmaceutical Sciences 16th edition, Osol, A. Ed. (1980). Acceptable carriers, excipients, or stabilizers are nontoxic to recipients at the dosages and concentrations employed, and include buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propylparaben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as olyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counterions such as sodium; metal complexes (*e.g.* Zn-protein complexes); and/or non-ionic surfactants such as TWEEN™, PLURONICS™ or polyethylene glycol (PEG). In some embodiments, lyophilized formulations are provided. Pharmaceutical compositions to be used for *in vivo* administration must be sterile. This is readily accomplished by, *e.g.*, filtration through sterile filtration membranes.

## Exemplary embodiments

### EXAMPLE

The examples below are intended to be exemplary of the present application. The following examples and detailed description are offered by way of illustration.

### Materials and methods

#### *Plasmids construction*

The dual fluorescence reporter was cloned by PCR amplifying mCherry and EGFP (the EGFP first codon ATG was deleted) coding DNA, the $3\times$GS linker and targeting DNA sequence were added via primers during PCR. Then the PCR products were cleaved and linked by Type IIs restriction enzyme BsmB1 (Thermo) and T4 DNA ligase (NEB), which then were inserted into pLenti backbone (pLenti-CMV-MCS-SV-Bsd, Stanley Cohen Lab, Stanford University).

The dLbuCas13 DNA was PCR amplified from the Lbu plasmids (Addgene #83485). The ADAR1DD and ADAR2DD were amplified from Adar1(p150) cDNA and Adar2 cDNA, both of which were gifts from Han's lab at Xiamen University. The ADAR1DD or ADAR2DD were fused to dLbuCas13 DNA by overlap-PCR, and the fused PCR products were inserted into pLenti backbone.

For expression of dRNA in mammalian cells, the dRNA sequences were directly synthesized (for short dRNAs) and annealed or PCR amplified by synthesizing overlapping ssDNA, and the products were cloned into the corresponding vectors under U6 expression by Golden-gate cloning.

The full length Adar1(p110) and Adar1(p150) were PCR amplified from Adar1(p150) cDNA, and the full length Adar2 were PCR amplified from Adar2 cDNA, which were then cloned into pLenti backbone, respectively.

For the three versions of dual fluorescence reporters (Reporter-1, -2 and -3), *mCherry* and *EGFP* (the start codon ATG of EGFP was deleted) coding sequences were PCR amplified, digested using BsmBI (Thermo Fisher Scientific, ER0452), followed by T4 DNA ligase (NEB, M0202L)-mediated ligation with GGGGS linkers. The ligation product was subsequently inserted into the pLenti-CMV-MCS-PURO backbone.

For the dLbuCas13-ADAR$_{DD}$ (E1008Q) expressing construct, the ADAR1$_{DD}$ gene was amplified from the ADAR1$^{p150}$ construct (a gift from Jiahuai Han's lab, Xiamen University). The dLbuCas13 gene was amplified by PCR from the Lbu C2c2 R472A H477A R1048A_ H1053A plasmid (Addgene #83485). The ADAR1$_{DD}$ (hyperactive E1008Q variant) was generated by overlap-PCR and then fused to dLbuCas13. The ligation products were inserted into the pLenti-CMV-MCS-BSD backbone.

For arRNA-expressing construct, the sequences of arRNAs were synthesized and golden-gate cloned into the pLenti-sgRNA-lib 2.0 (Addgene #89638) backbone, and the transcription of arRNA was driven by hU6 promoter. For the ADAR expressing constructs, the full length ADAR1$^{p110}$ and ADAR1$^{p150}$ were PCR amplified from the ADAR1$^{p150}$ construct, and the full length ADAR2 were PCR amplified from the ADAR2 construct (a gift from Jiahuai Han's lab, Xiamen University). The amplified products were then cloned into the pLenti-CMV-MCS-BSD backbone.

For the constructs expressing genes with pathogenic mutations, full length coding sequences of *TP53* (ordered from Vigenebio) and other 6 disease-relevant genes (*COL3A1, BMPR2, AHI1, FANCC, MYBPC3* and *IL2RG,* gifts from Jianwei Wang's lab, Institute of pathogen biology, Chinese Academy of Medical Sciences) were amplified from the constructs encoding the corresponding genes with introduction of G>A mutations through mutagenesis PCR. The amplified products were cloned into the pLenti-CMV-MCS-mCherry backbone through Gibson cloning method[59].

#### *Mammalian cell lines and Cell culture*

Mammalian cell lines were cultured Dulbecco's Modified Eagle Medium (10-013-CV, Corning, Tewksbury, MA, USA), adding 10% fetal bovine serum (Lanzhou Bailing Biotechnology Co., Ltd., Lanzhou, China), supplemented with 1% penicillin-streptomycin under 5% $CO_2$ at 37°C. The Adar1-KO cell line was purchased from EdiGene China, and the genotyping results were also provided by EdiGene China.

The HeLa and B16 cell lines were from Z. Jiang's laboratory (Peking University). And the HEK293T cell line was from C. Zhang's laboratory (Peking University). RD cell line was from J Wang's laboratory (Institute of Pathogen Biology, Peking Union Medical College & Chinese Academy of Medical Sciences). SF268 cell lines were from Cell Center, Institute of Basic Medical Sciences, Chinese Academy of Medical Sciences. A549 and SW13 cell lines were from EdiGene Inc. HepG2, HT29, NIH3T3, and MEF cell lines were maintained in our laboratory at Peking University. These mammalian cell lines were cultured in Dulbecco's Modified Eagle Medium (Corning, 10-013-CV) with 10% fetal bovine serum (CellMax, SA201.02), additionally supplemented with 1% penicillin-streptomycin under 5% $CO_2$ at 37°C. Unless otherwise described, cells were transfected with the X-tremeGENE HP DNA transfection reagent (Roche, 06366546001) according to the manufacturer's instruction.

The human primary pulmonary fibroblasts (#3300) and human primary bronchial epithelial cells (#3210) were purchased from ScienCell Research Laboratories, Inc. and were cultured in Fibroblast Medium (ScienCell, #2301) and Bronchial Epithelial Cell Medium (ScienCell, #3211), respectively. Both media were supplemented with 15% fetal bovine serum (BI) and 1% penicillin-streptomycin. The primary GM06214 and GM01323 cells were ordered from Coriell Institute for Medical Research and cultured in Dulbecco's Modified Eagle Medium (Corning, 10-013-CV) with 15% fetal bovine serum (BI) and 1% penicillin-streptomycin. All cells were cultured under 5% $CO_2$ at 37°C.

### Reporter system transfection, FACS analysis and Sanger Sequencing

For dual fluorescence reporter editing experiments, 293T-WT cells or 293T-Adar1-KO cells were seeded in 6 wells plates ($6\times10^5$ cells/well), 24 hours later, 1.5 μg reporter plasmids and 1.5 μg dRNA plasmids were co-transfected using the X-tremeGENE HP DNA transfection reagent (06366546001; Roche, Mannheim, German), according to the supplier's protocols. 48 to 72 hours later, collected cells and performed FACS analysis. For further confirming the reporter mRNA editing, we sorted the EGFP-positive cells from 293T-WT cells transfected with reporter and dRNA plasmids using a FACS Aria flow cytometer (BD Biosciences), followed by total RNA isolation (TIANGEN, DP430). Then the RNA was reverse-transcribed into cDNA via RT-PCR (TIANGEN, KR103-04), and the targeted locus were PCR amplified with the corresponding primer pairs (23 PCR cycles) and the PCR products were purified for Sanger sequencing.

For Adar1(p110), Adar1(p150) or Adar2 rescue and overexpression experiments, 293T-WT cells or 293T-Adar1-KO cells were seeded in 12 wells plates ($2.5\times10^5$ cells/well), 24 hours later, 0.5 μg reporter plasmids, 0.5 μg dRNA plasmids and 0.5 μg Adar1/2 plasmids (pLenti backbone as control) were co-transfected using the X-tremeGENE HP DNA transfection reagent (06366546001, Roche, Mannheim, German). 48 to 72 hours later, collected cells and performed FACS analysis.

For endogenous mRNA experiments, 293T-WT cells were seeded in 6 wells plates ($6\times10^5$ cells/well), When approximately 70% confluent, 3 μg dRNA plasmids were transfected using the X-tremeGENE HP DNA transfection reagent (06366546001, Roche, Mannheim, German). 72 hours later, collected cells and sorted GFP-positive or BFP-positive cells (according to the corresponding fluorescence maker) via FACS for the following RNA isolation.

### Isolation and culture of human primary T cells

Primary human T cells were isolated from leukapheresis products from healthy human donor. Briefly, Peripheral blood mononuclear cells (PBMCs) were isolated by Ficoll centrifugation (Dakewei, AS1114546), and T cells were isolated by magnetic negative selection using an EasySep Human T Cell Isolation Kit (STEMCELL, 17951) from PBMCs. After isolation, T cells were cultured in X-vivo15 medium, 10% FBS and IL2 (1000 U/ml) and stimulated with CD3/CD28 DynaBeads (ThermoFisher, 11131D) for 2 days. Leukapheresis products from healthy donors were acquired from AllCells LLC China. All healthy donors provided informed consent.

### Lenti-virus package and reporter cells line construction

The expression plasmid was co-transfected into HEK293T-WT cells, together with two viral packaging plasmids, pR8.74 and pVSVG (Addgene) via the X-tremeGENE HP DNA transfection reagent. 72 hours later, the supernatant virus was collected and stored at -80°C. The HEK293T-WT cells were infected with lenti-virus, 72 hours later, mCherry-positive cells were sorted via FACS and cultured to select a single clone cell lines stably expressing dual fluorescence reporter system with much low EGFP background by limiting dilution method.

For the stable reporter cell lines, the reporter constructs (pLenti-CMV-MCS-PURO backbone) were co-transfected into HEK293T cells, together with two viral packaging plasmids, pR8.74 and pVSVG. 72 hours later, the supernatant virus was collected and stored at -80°C. The HEK293T cells were infected with lentivirus, then mCherry-positive cells were sorted via FACS and cultured to select a single clone cell lines stably expressing dual fluorescence reporter system without detectable EGFP background. The HEK293T $ADAR1^{-/-}$ and $TP53^{-/-}$ cell lines were generated according to a previously reported method[60]. ADAR1-targeting sgRNA and PCR amplified donor DNA containing CMV-driven puromycin resistant gene were co-transfected into HEK293T cells. Then cells were treated with puromycin 7 days after transfection. Single clones were isolated from puromycin resistant cells followed by verification through sequencing and Western blot.

*RNA editing of endogenous or exogenous-expressed transcripts*

For assessing RNA editing on the dual fluorescence reporter, HEK293T cells or HEK293T *ADAR1*[-/-] cells were seeded in 6-well plates ($6 \times 10^5$ cells/well). 24 hours later, cells were co-transfected with 1.5 $\mu$g reporter plasmids and 1.5 $\mu$g arRNA plasmids. To examine the effect of ADAR1p110, ADAR1p150 or ADAR2 protein expression, the editing efficiency was assayed by EGFP positive ratio and deep sequencing.

HEK293T *ADAR1*[-/-] cells were seeded in 12-well plates ($2.5 \times 10^5$ cells/well). 24 hours later, cells were co-transfected with 0.5 $\mu$g of reporter plasmids, 0.5 $\mu$g arRNA plasmids and 0.5 $\mu$g ADAR1/2 plasmids (pLenti backbone as control). The editing efficiency was assayed by EGFP positive ratio and deep sequencing.

To assess RNA editing on endogenous mRNA transcripts, HEK293T cells were seeded in 6-well plates ($6 \times 10^5$ cells/well). Twenty-four hours later, cells were transfected with 3 $\mu$g of arRNA plasmids. The editing efficiency was assayed by deep sequencing.

To assess RNA editing efficiency in multiple cell lines, $8-9 \times 10^4$ (RD, SF268, HeLa) or $1.5 \times 10^5$ (HEK293T) cells were seeded in 12-well plates. For cells difficult to transfect, such as HT29, A549, HepG2, SW13, NIH3T3, MEF and B16, $2-2.5 \times 10^5$ cells were seeded in 6-well plate. Twenty-four hours later, reporters and arRNAs plasmid were co-transfected into these cells. The editing efficiency was assayed by EGFP positive ratio.

To evaluate EGFP positive ratio, at 48 to 72 hrs post transfection, cells were sorted and collected by Fluorescence-activated cell sorting (FACS) analysis. The mCherry signal was served as a fluorescent selection marker for the reporter/arRNA-expressing cells, and the percentages of EGFP[+]/mCherry[+] cells were calculated as the readout for editing efficiency.

For NGS quantification of the A to I editing rate, at 48 to 72 hr post transfection, cells were sorted and collected by FACS assay and were then subjected to RNA isolation (TIANGEN, DP420). Then, the total RNAs were reverse-transcribed into cDNA via RT-PCR (TIANGEN, KR103-04), and the targeted locus was PCR amplified with the corresponding primers listed in the following table.

| Name of Primer | Sequence (5'--->3') |
|---|---|
| mCherry-SpeI-F | TATAACTAGTATGGTGAGCAAGGGCGAGGAG (SEQ ID NO: 206) |
| mCherry-BsmBI-RI | TATACGTCTCATCTACAGATTCTTCCGGCGTGTATACCTTC (SEQ ID NO: 207) |
| EGFP-BsmBI-FI (Reporter-1) | TATACGTCTCATAGAGATCCCCGGTCGCCACCGTGAGCAAGGGCGAGGAG CTG (SEQ ID NO: 208) |
| EGFP-AscI-R | TATAGGCGCGCCTTACTTGTACAGCTCGTCCATGCC (SEQ ID NO: 209) |
| mCherry-BsmBI-R2 | TATACGTCTCAAGGCGCTGCCTCCTCCGCCGCTGCCTCCTCCGCCGCTGCC TCCTCCGCCCTGCAGCTTGTACAGCTCGTCCATGCCGCCGGTG (SEQ ID NO: 210) |
| EGFP-BsmBI-F2 (Reporter-2) | TATACGTCTCAGCCTGCTCGCGATGCTAGAGGGCTCTGCCAGTGAGCAAG GGCGAGGAGCTG (SEQ ID NO: 211) |
| LbuCas 13-SpeI-F | TATAACTAGTATGGTGGATTACAAGGATGACGACGATAAGATGAAAGTG ACGAAGGTAGGAGGCATTTCG (SEQ ID NO: 212) |
| LbuCas 13-AscI-R | ATATGGCGCGCCGTTTTCAGACTTTTTCTCTTCCATTTTGTATTCAAACATA ATCTTCAC (SEQ ID NO: 213) |
| hADAR1$_{DD}$-AscI-F | TATAGGCGCGCCAGGCGGAGGAGGCAGCGGCGGAGGAGGCAGCCTCCTC CTCTCAAGGTCCCCAGAAGC (SEQ ID NO: 214) |

(continued)

| Name of Primer | Sequence (5'--->3') |
|---|---|
| hADAR1<sub>DD</sub>-Sbfl-R | TATACCTGCAGGCTACACCTTGCGTTTTTTCTTGGGTACTGGGCAGAGATA AAAGTTCTTTTCC<br>(SEQ ID NO: 215) |
| Deep-seq-F (Reporter-1) | CACTCCACCGGCGGCATGGACGAG (SEQ ID NO: 216) |
| Deep-seq-R (Reporter-1) | CACGCTGAACTTGTGGCCGTTTACGTCG (SEQ ID NO: 217) |
| ADARl-pl50-Spel-F | TATAACTAGTATGAATCCGCGGCAGGGGTATTCCCTCAGC (SEQ ID NO: 218) |
| ADARl-pl50-Ascl-R | TATAGGCGCGCCCTACTTATCGTCGTCATCCTTGTAATCTACTGGGCAGAG ATAAAAGTTCTTTTCCTCCTGG<br>(SEQ ID NO: 219) |
| ADAR2-Spel-F | TATAACTAGTATGGATATAGAAGATGAAGAAAACATGAGTTC (SEQ ID NO: 220) |
| ADAR2-Ascl-R | TATAGGCGCGCCCTACTTATCGTCGTCATCCTTGTAATCGGGCGTGAGTGA GAACTGGTCCTGCTCG<br>(SEQ ID NO: 221) |
| ADAR1-p110-Spel-F | TATAACTAGTATGGCCGAGATCAAGGAGAAAATCTGC<br>(SEQ ID NO: 222) |
| ADAR1-p110-Ascl-R | TATAGGCGCGCCCTACTTATCGTCGTCATCCTTGTAATCTACTGGGCAGAG ATAAAAGTTCTTTTCCTCCTGG<br>(SEQ ID NO: 223) |
| KRAS-deep-seq-F | CGCCATTTCGGACTGGGAG (SEQ ID NO: 224) |
| KRAS-deep-seq-R | AGAGACAGGTTTCTCCATCAATTAC (SEQ ID NO: 225) |
| PPIB-deep-seq-F | GAGCCCGCGAGCAACC (SEQ ID NO: 226) |
| PPIB-deep-seq-R | GCAGCAGGAAGAAGACGGAC (SEQ ID NO: 227) |
| FANCC-deep-seq-F1 (TAC site) | AGAAGCAGTTGAAGACCAGACTC (SEQ ID NO: 228) |
| FANCC-deep-seq-R (TAC site) | GGCCTTCACCTGGACCATAG (SEQ ID NO: 229) |
| FANCC-deep-seq-F2 (TAC site) | AGAGAAGCAGTTGAAGACCAGA (SEQ ID NO: 230) |
| FANCC-deep-seq-R2 (TAC site) | CGGCCTTCACCTGGACCATA (SEQ ID NO: 231) |
| FANCC-deep-seq-F3 (TAC site) | CAGAGAAGCAGTTGAAGACCAGA (SEQ ID NO: 232) |
| FANCC-deep-seq-R3 (TAC site) | CGGCCTTCACCTGGACCATA (SEQ ID NO: 233) |
| SMAD4-deep-seq-F1 | TTTGTGAAAGGCTGGGGACC (SEQ ID NO: 234) |
| SMAD4-deep-seq-Rl | ACAGGATTGTATTTTGTAGTCCACC (SEQ ID NO: 235) |
| SMAD4-deep-seq-F2 | AGGATGAGTTTTGTGAAAGGCTG (SEQ ID NO: 236) |

(continued)

| Name of Primer | Sequence (5'--->3') |
|---|---|
| SMAD4-deep-seq-R2 | ATTTTGTAGTCCACCATCCTGATA (SEQ ID NO: 237) |
| SMAD4-deep-seq-F3 | GATGAGTTTTGTGAAAGGCTGG (SEQ ID NO: 238) |
| SMAD4-deep-seq-R3 | ATTTTGTAGTCCACCATCCTGATAA (SEQ ID NO: 239) |
| TRAPPC12-deep-seq-F | CGAAGAGAACGAGACCGCAT (SEQ ID NO: 240) |
| TRAPPC12-deep-seq-R | GAAGATGGTGCACACCGGG (SEQ ID NO: 241) |
| TARDBP-deep-seq-F | GACAGATGCTTCATCAGCAGTG (SEQ ID NO: 242) |
| TARDBP-deep-seq-R | CGAACAAAGCCAAACCCCTTT (SEQ ID NO: 243) |
| COL3AI-deep-seq-F | TCTGTTAATGGACAAATAGAAAGCC (SEQ ID NO: 244) |
| COL3AI-deep-seq-R | GGAACATTCAAAGGATTGGCACT (SEQ ID NO: 245) |
| BMPR2-deep-seq-F | AGTCACTGCAGATGGACGCA (SEQ ID NO: 246) |
| BMPR2-deep-seq-R | ATCTCGATGGGAAATTGCAGGT (SEQ ID NO: 247) |
| AHII-deep-seq-F | TCAGAGTTTTACCTCATCCTTCTTT (SEQ ID NO: 248) |
| AHII-deep-seq-R | CCTGAATACATATGATGACCTTCAG (SEQ ID NO: 249) |
| FANCC-deep-seq-F (Site2) | AGGGCACAGACACAGACCTC (SEQ ID NO: 250) |
| FANCC-deep-seq-R (Site2) | AGGGCTTTCAATGCCAAGACG (SEQ ID NO: 251) |
| MYBPC3-deep-seq-F | TGACAAGCCAAGTCCTCCC (SEQ ID NO: 252) |
| MYBPC3-deep-seq-R | ATTGCCAATGATGAGCTCTGG (SEQ ID NO: 253) |
| IL2RG-deep-seq-F | TTATAGACATAAGTTCTCCTTGCCT (SEQ ID NO: 254) |
| IL2RG-deep-seq-R | TCAATCCCATGGAGCCAACA (SEQ ID NO: 255) |
| 1-deep-seq-F (Reporter-3) | TACACGACGCTCTTCCGATCTTAAGTAGAGGCCGCCACTCCACCGGCGGC (SEQ ID NO: 256) |
| 2-deep-seq-F (Reporter-3) | TACACGACGCTCTTCCGATCTATCATGCTTAGCCGCCACTCCACCGGCGGC (SEQ ID NO: 257) |
| 3-deep-seq-F (Reporter-3) | TACACGACGCTCTTCCGATCTGATGCACATCTGCCGCCACTCCACCGGCGGC (SEQ ID NO: 258) |

(continued)

| Name of Primer | Sequence (5'--->3') |
|---|---|
| 4-deep-seq-F (Reporter-3) | TACACGACGCTCTTCCGATCTCGATTGCTCGACGCCGCCACTCCACCGGCG GC<br>(SEQ ID NO: 259) |
| 5-deep-seq-F (Reporter-3) | TACACGACGCTCTTCCGATCTTCGATAGCAATTCGCCGCCACTCCACCGGC GGC<br>(SEQ ID NO: 260) |
| 6-deep-seq-F (Reporter-3) | TACACGACGCTCTTCCGATCTATCGATAGTTGCTTGCCGCCACTCCACCGG CGGC<br>(SEQ ID NO: 261) |
| 7-deep-seq-F (Reporter-3) | TACACGACGCTCTTCCGATCTGATCGATCCAGTTAGGCCGCCACTCCACCG GCGGC<br>(SEQ ID NO: 262) |
| 8-deep-seq-F (Reporter-3) | TACACGACGCTCTTCCGATCTCGATCGATTTGAGCCTGCCGCCACTCCACC GGCGGC<br>(SEQ ID NO: 263) |
| 9-deep-seq-F (Reporter-3) | TACACGACGCTCTTCCGATCTACGATCGATACACGATCGCCGCCACTCCA CCGGCGGC<br>(SEQ ID NO: 264) |
| 10-deep-seq-F (Reporter-3) | TACACGACGCTCTTCCGATCTTACGATCGATGGTCCAGAGCCGCCACTCC ACCGGCGGC<br>(SEQ ID NO: 265) |
| 1-deep-seq-R (Reporter-3) | AGACGTGTGCTCTTCCGATCTTAAGTAGAGTCGCCGTCCAGCTCGACCAG (SEQ ID NO: 266) |
| 2-deep-seq-R (Reporter-3) | AGACGTGTGCTCTTCCGATCTATCATGCTTATCGCCGTCCAGCTCGACCAG (SEQ ID NO: 267) |
| 3-deep-seq-R (Reporter-3) | AGACGTGTGCTCTTCCGATCTGATGCACATCTTCGCCGTCCAGCTCGACCA G<br>(SEQ ID NO: 268) |
| 4-deep-seq-R (Reporter-3) | AGACGTGTGCTCTTCCGATCTCGATTGCTCGACTCGCCGTCCAGCTCGACC AG<br>(SEQ ID NO: 269) |
| 5-deep-seq-R (Reporter-3) | AGACGTGTGCTCTTCCGATCTTCGATAGCAATTCTCGCCGTCCAGCTCGAC CAG<br>(SEQ ID NO: 270) |
| 6-deep-seq-R (Reporter-3) | AGACGTGTGCTCTTCCGATCTATCGATAGTTGCTTTCGCCGTCCAGCTCGA CCAG<br>(SEQ ID NO: 271) |
| 7-deep-seq-R (Reporter-3) | AGACGTGTGCTCTTCCGATCTGATCGATCCAGTTAGTCGCCGTCCAGCTCG ACCAG<br>(SEQ ID NO: 272) |
| 8-deep-seq-R (Reporter-3) | AGACGTGTGCTCTTCCGATCTCGATCGATTTGAGCCTTCGCCGTCCAGCTC GACCAG<br>(SEQ ID NO: 273) |
| 9-deep-seq-R (Reporter-3) | AGACGTGTGCTCTTCCGATCTACGATCGATACACGATCTCGCCGTCCAGCT CGACCAG<br>(SEQ ID NO: 274) |

(continued)

| Name of Primer | Sequence (5'--->3') |
|---|---|
| 10-deep-seq-R (Reporter-3) | AGACGTGTGCTCTTCCGATCTTACGATCGATGGTCCAGATCGCCGTCCAGC TCGACCAG (SEQ ID NO: 275) |
| ST3GAL1-deep-seq-F | GGGGAACTCGGGCAACCT (SEQ ID NO: 276) |
| ST3GAL1-deep-seq-R | GAATCGGATCTGCCCCGTG (SEQ ID NO: 277) |
| EHD2-deep-seq-F | CATCGAGGCCAAGCTGGAA (SEQ ID NO: 278) |
| EHD2-deep-seq-R | GTAGTGAGGAGGGAGACCCC (SEQ ID NO: 279) |
| OSTM1-AS1-deep-seq-F | AAGCCTCCTTCCTTCCCCAA (SEQ ID NO: 280) |
| OSTM1-AS1-deep-seq-R | ATCGATACACTCCCTAGCCCA (SEQ ID NO: 281) |
| IL6-qPCR-F1 | ACAAATTCGGTACATCCTCGAC (SEQ ID NO: 282) |
| IL6-qPCR-R1 | TTCAGCCATCTTTGGAAGGTT (SEQ ID NO: 283) |
| INF-β-qPCR-F1 | ACGCCGCATTGACCATCTAT (SEQ ID NO: 284) |
| INF-β-qPCR-R1 | TAGCCAGGAGGTTCTCAACA (SEQ ID NO: 285) |
| GAPDH-F1 | GGCATGGACTGTGGTCATGAG (SEQ ID NO: 286) |
| GAPDH-R1 | TGCACCACCAACTGCTTAGC (SEQ ID NO: 287) |
| Reporter-1-qPCR-F | CCCCGTAATGCAGAAGAAGACC (SEQ ID NO: 288) |
| Reporter-1-qPCR-R | GTCCTTCAGCTTCAGCCTCTG (SEQ ID NO: 289) |
| PPIB-qPCR-F | AACGCAACATGAAGGTGCTC (SEQ ID NO: 290) |
| PPIB-qPCR-R | ACCTTGACGGTGACTTTGGG (SEQ ID NO: 291) |
| KRAS-qPCR-F | CAGTGCAATGAGGGACCAGT (SEQ ID NO: 292) |
| KRAS-qPCR-R | AGGACCATAGGTACATCTTCAGAG (SEQ ID NO: 293) |
| SMAD4-qPCR-F | CGAACGAGTTGTATCACCTGGA (SEQ ID NO: 294) |
| SMAD4-qPCR-R | CGATGGCTGTCCCTCAAAGT (SEQ ID NO: 295) |
| FANCC-qPCR-F | AGTTGCTCTTTTCACTCAAGGTC (SEQ ID NO: 296) |
| FANCC-qPCR-R | TTCTCTCTGAGTTCAGACGCT (SEQ ID NO: 297) |
| PPIB-deep-seq-F (AAG site) | TACACGACGCTCTTCCGATCTTAAGTAGAGTGGCACAGGAGGAAAGAGCA TC (SEQ ID NO: 298) |
| PPIB-deep-seq-R (AAG site) | AGACGTGTGCTCTTCCGATCTTAAGTAGAGGCACCACCTCCATGCCCTC (SEQ ID NO: 299) |
| PPIB-deep-seq-F (CAG site) | TACACGACGCTCTTCCGATCTTAAGTAGAGCATCGCAGACTGCGGCAAG (SEQ ID NO: 300) |
| PPIB-deep-seq-R (CAG site) | AGACGTGTGCTCTTCCGATCTTAAGTAGAGAGTCCATGGGCCTGTGGAAT GT (SEQ ID NO: 301) |

(continued)

| Name of Primer | Sequence (5'--->3') |
|---|---|
| FANCC-deep-seq-F2 (AAG/CAG site) | GAAAAACTGGCCCGAGAGC (SEQ ID NO: 302) |
| FANCC-deep-seq-R2 (AAG/CAG site) | CTGAGTCTGGGCTGAGGGAC (SEQ ID NO: 303) |
| IDUA-deep-seq-F | CGCTTCCAGGTCAACAACAC (SEQ ID NO: 304) |
| IDUA-deep-seq-R | CTCGCGTAGATCAGCACCG (SEQ ID NO: 305) |
| p53-deep-seq-F | CCCCTCTGAGTCAGGAAACAT (SEQ ID NO: 306) |
| p53-deep-seq-R | GAAGATGACAGGGGCCAGG (SEQ ID NO: 307) |
| IFN-β-qPCR-F | TAGCACTGGCTGGAATGAG (SEQ ID NO: 308) |
| IFN-β-qPCR-R | GTTTCGGAGGTAACCTGTAAG (SEQ ID NO: 309) |
| ISG56-qPCR-F | TACAGCAACCATGAGTACAA (SEQ ID NO: 310) |
| ISG56-qPCR-R | TCAGGTGTTTCACATAGGC (SEQ ID NO: 311) |
| ISG54-qPCR-F | CTGCAACCATGAGTGAGAA (SEQ ID NO: 312) |
| ISG54-qPCR-R | CCTTTGAGGTGCTTTAGATAG (SEQ ID NO: 313) |
| IL-6-qPCR-F | GCCCTGAGAAAGGAGACAT (SEQ ID NO: 314) |
| IL-6-qPCR-R | CTGTTCTGGAGGTACTCTAGGTAT (SEQ ID NO: 315) |
| IL-8-qPCR-F | TTTGAAGAGGGCTGAGAA (SEQ ID NO: 316) |
| IL-8-qPCR-R | TGTTCTGGATATTTCATGG (SEQ ID NO: 317) |
| RANTES-qPCR-F | CATCTGCCTCCCCATATTCC (SEQ ID NO: 318) |
| RANTES-qPCR-R | TCCATCCTAGCTCATCTCCAAA (SEQ ID NO: 319) |
| IL-12-qPCR-F | TGCTCCAGAAGGCCAGAC (SEQ ID NO: 320) |
| IL-12-qPCR-R | TTCATAAATACTACTAAGGCACAGG (SEQ ID NO: 321) |
| IL-1β-qPCR-F | ACAGATGAAGTGCTCCTTCCA (SEQ ID NO: 322) |
| IL-1β-qPCR-R | GTCGGAGATTCGTAGCTGGAT (SEQ ID NO: 323) |
| MCP1-qPCR-F | CATTGTGGCCAAGGAGATCTG (SEQ ID NO: 324) |
| MCP1-qPCR-R | CTTCGGAGTTTGGGTTTGCTT (SEQ ID NO: 325) |
| MIP1A-qPCR-F | CATCACTTGCTGCTGACACG (SEQ ID NO: 326) |
| MIP1A-qPCR-R | TGTGGAATCTGCCGGGAG (SEQ ID NO: 327) |
| IP10-qPCR-F | CTGACTCTAAGTGGCATT (SEQ ID NO: 328) |
| IP10-qPCR-R | TGATGGCCTTCGATTCTG (SEQ ID NO: 329) |
| GAPDH-qPCR-F2 | CGGAGTCAACGGATTTGGTCGTA (SEQ ID NO: 330) |
| GAPDH-qPCR-R2 | AGCCTTCTCCATGGTGGTGAAGAC (SEQ ID NO: 331) |

The PCR products were purified for Sanger sequencing or NGS (Illumina HiSeq X Ten).

*Deep sequencing*

For endogenous mRNA editing experiments, 293T-WT cells were seeded on 6 wells plates ($6\times10^5$ cells/well), When approximately 70% confluent, HEK293 cells were transfected with 3 $\mu$g dRNA using the X-tremeGENE HP DNA transfection reagent (Roche). 72 hours later, sorted GFP-positive or BFP-positive cells (according to the corresponding fluorescence marker) via FACS, followed by RNA isolation. Then the isolated RNA was reverse-transcribed into cDNA via RT-PCR, and specific targeted gene locus were amplified with the corresponding primer pairs (23 PCR cycles) and sequenced on an Illumina NextSeq.

*Testing in multiple cell lines*

Besides HEK293T (positive control) and HEK293T ADAR1$^{-/-}$ (negative control) cells, one mouse cell line (NIH3T3) as well as seven human cell lines (RD, HeLa, SF268, A549, HepG2, HT-29, SW13) originating from different tissues and organs were selected to perform the experiment. For the cell lines with higher transfection efficiency, about 8-9 $\times$ $10^4$ cells (RD, HeLa, SF268) or $1.5 \times 10^5$ (HEK293T) were plated onto each well of 12-well plate, as for the ones (A549, HepG2, HT-29, SW13, NIH3T3) which are difficult to transfect, 2-2.5$\times10^5$ cells were plated in 6-well plate. And all these cells were maintained in Dulbecco's modified Eagle's medium (DMEM, Corning) supplemented with 10% fetal bovine serum (FBS, CellMax) with 5% $CO_2$ in 37°C. 24 hrs later, CG2 reporter and 71nt dRNA (35-C-35) plasmid were co-transfected into different type of cells with X-tremeGENE HP DNA transfection reagent (Roche). 48 hrs after transfection, cells were trypsinized and analyzed through FACS (BD). Because the cells with low transfection efficiency had quite fewer mCherry and BFP positive cells, we increased the total cell number for FACS analysis to $1 \times 10^5$ for those cells plated onto 6-well plate.

*RNA editing analysis for targeted sites*

For deep sequencing analysis, an index was generated using the targeted site sequence (upstream and downstream 20-nt) of arRNA covering sequences. Reads were aligned and quantified using BWA version 0.7.10-r789. Alignment BAMs were then sorted by Samtools, and RNA editing sites were analyzed using REDitools version 1.0.4. The parameters are as follows: -U [AG or TC] -t 8 -n 0.0 -T 6-6 -e -d -u. All the significant A>G conversion within arRNA targeting region calculated by Fisher's exact test (p-value < 0.05) were considered as edits by arRNA. The conversions except for targeted adenosine were off-target edits. The mutations that appeared in control and experimental groups simultaneously were considered as SNP.

*Transcriptome-wide RNA-sequencing analysis*

The Ctrl RNA$_{151}$ or arRNAisi-PPIB-expressing plasmids with BFP expression cassette were transfected into HEK293T cells. The BFP$^+$ cells were enriched by FACS 48 hours after transfection, and RNAs were purified with RNAprep Pure Micro kit (TIANGEN, DP420). The mRNA was then purified using NEBNext Poly(A) mRNA Magnetic Isolation Module (New England Biolabs, E7490), processed with the NEBNext Ultra II RNA Library Prep Kit for Illumina (New England Biolabs, E7770), followed by deep sequencing analysis using Illumina HiSeq X Ten platform ($2\times$ 150-bp paired end; 30G for each sample). To exclude nonspecific effect caused by transfection, we included the mock group in which we only treated cells with transfection reagent. Each group contained four replications.

The bioinformatics analysis pipeline was referred to the work by Vogel *et al*[22]. The quality control of analysis was conducted by using FastQC, and quality trim was based on Cutadapt (the first 6-bp for each reads were trimmed and up to 20-bp were quality trimmed). AWK scripts were used to filtered out the introduced arRNAs. After trimming, reads with lengths less than 90-nt were filtered out. Subsequently, the filtered reads were mapped to the reference genome (GRCh38-hg38) by STAR software[61]. We used the GATK Haplotypcaller[62] to call the variants. The raw VCF files generated by GATK were filtered and annotated by GATK VariantFiltration, bcftools and ANNOVAR[63]. The variants in dbSNP, 1000 Genome[64], EVS were filtered out. The shared variants in four replicates of each group were then selected as the RNA editing sites. The RNA editing level of Mock group was viewed as the background, and the global targets of Ctrl RNA$_{151}$ and arRNA$_{151}$-PPIB were obtained by subtracting the variants in the Mock group.

To assess if LEAPER perturbs natural editing homeostasis, we analyzed the global editing sites shared by Mock group and arRNA$_{151}$-PPIB group (or Ctrl RNA$_{151}$ group). The differential RNA editing rates at native A-to-I editing sites were assessed with Pearson's correlation coefficient analysis. Pearson correlations of editing rate between Mock group and arRNAisi-PPIB group (or Ctrl RNA$_{151}$ group) were calculated and annotated in Fig. 6.

$$\rho(X,Y) = \frac{E[(X - \mu_X)(Y - \mu_Y)]}{\sigma_X \sigma_Y}$$

X means the editing rate of each site in the Mock group; Y means the editing rate of each site in the Ctrl RNA$_{151}$ group (Fig. 6A) or arRNA$_{151}$-PPIB group (Fig. 6B); $\sigma_X$ is the standard deviation of X; $\sigma_Y$ is the standard deviation of Y; $\mu_X$ is the mean of X; $\mu_Y$ is the mean of Y; E is the expectation.

The RNA-Seq data were analysed for the interrogation of possible transcriptional changes induced by RNA editing events. The analysis of transcriptome-wide gene expression was performed using HISAT2 and STRINGTIE software[65]. We used Cutadapt and FastQC for the quality control of the sequencing data. The sequencing reads were then mapped to reference genome (GRCh38-hg38) using HISAT2, followed by Pearson's correlation coefficient analysis as mentioned above.

### Western blot

We used the mouse monoclonal primary antibodies respectively against ADAR1 (Santa Cruz, sc-271854), ADAR2 (Santa Cruz, sc-390995), ADAR3 (Santa Cruz, sc-73410), p53 (Santa Cruz, sc-99), KRAS (Sigma, SAB1404011); GAPDH (Santa Cruz, sc-47724) and β-tubulin (CWBiotech, CW0098). The HRP-conjugated goat anti-mouse IgG (H+L, 115-035-003) secondary antibody was purchased from Jackson ImmunoResearch. $2 \times 10^6$ cells were sorted to be lysed and an equal amount of each lysate was loaded for SDS-PAGE. Then, sample proteins were transferred onto PVDF membrane (Bio-Rad Laboratories) and immunoblotted with primary antibodies against one of the ADAR enzymes (anti-ADAR1, 1:500; anti-ADAR2, 1:100; anti-ADAR3, 1:800), followed by secondary antibody incubation (1:10,000) and exposure. The β-Tubulin was re-probed on the same PVDF membrane after stripping of the ADAR proteins with the stripping buffer (CWBiotech, CW0056). The experiments were repeated three times. The semi-quantitative analysis was done with Image Lab software.

### Cytokine expression assay

HEK293T cells were seeded on 12 wells plates ($2 \times 10^5$ cells/well). When approximately 70% confluent, cells were transfected with 1.5 μg of arRNA. As a positive control, 1 μg of poly(I:C) (Invitrogen, tlrl-picw) was transfected. Forty-eight hours later, cells were collected and subjected to RNA isolation (TIANGEN, DP430). Then, the total RNAs were reverse-transcribed into cDNA via RT-PCR (TIANGEN, KR103-04), and the expression of IFN-β and IL-6 were measured by quantitative PCR (TAKARA, RR820A). The sequences of the primers were listed in the above table.

### Transcriptional regulatory activity assay of p53

The TP53$^{W53X}$ cDNA-expressing plasmids and arRNA-expressing plasmids were co-transfected into HEK293T TP53$^{-/-}$ cells, together with p53-Firefly-luciferase cis-reporting plasmids (YRGene, VXS0446) and Renilla-luciferase plasmids (a gift from Z. Jiang's laboratory, Peking University) for detecting the transcriptional regulatory activity of p53. 48 hrs later, the cells were harvested and assayed with the Promega Dual-Glo Luciferase Assay System (Promega, E4030) according to the manufacturer protocol. Briefly, 150 μL Dual-Glo Luciferase Reagent was added to the harvested cell pellet, and 30 minutes later, the Firefly luminescence was measured by adding 100 μL Dual-Glo Luciferase Reagent (cell lysis) to 96-well white plate by Infinite M200 reader (TECAN). 30 min later, 100 μL Dual-Glo stop and Glo Reagent were sequentially added to each well to measure the Renilla luminescence and calculate the ratio of Firefly luminescence to Renilla luminescence.

### Electroporation in primary cells

For arRNA-expressing plasmids electroporation in the human primary pulmonary fibroblasts or human primary bronchial epithelial cells, 20 μg plasmids were electroporated with Nucleofector™ 2b Device (Lonza) and Basic Nucleofector™ Kit (Lonza, VPI-1002), and the electroporation program was U-023. For arRNA-expressing plasmids electroporation in human primary T cells, 20 μg plasmids were electroporated into human primary T with Nucleofector™ 2b Device (Lonza) and Human T cell Nucleofector™ Kit (Lonza, VPA-1002), and the electroporation program was T-024. Forty-eight hours post-electroporation, cells were sorted and collected by FACS assay and were then subjected to the following deep-sequencing for targeted RNA editing assay. The electroporation efficiency was normalized according to the fluorescence marker.

For the chemosynthetic arRNA or control RNA electroporation in human primary T cells or primary GM06214 cells, RNA oligo was dissolved in 100 μL opti-MEM medium (Gbico, 31985070) with the final concentration 2 μM. Then

1×10E6 GM06214 cells or 3×10E6 T cells were resuspended with the above electroporation mixture and electroporated with Agile Pulse In Vivo device (BTX) at 450 V for 1 ms. Then the cells were transferred to warm culture medium for the following assays.

*α-L-iduronidase (IDUA) catalytic activity assay*

The harvested cell pellet was resuspended and lysed with 28 μL 0.5% Triton X-100 in 1×PBS buffer on ice for 30 minutes. And then 25 μL of the cell lysis was added to 25 μL 190 μM 4-methylumbelliferyl-α-L-iduronidase substrate (Cayman, 2A-19543-500), which was dissolved in 0.4 M sodium formate buffer containing 0.2% Triton X-100, pH 3.5, and incubated for 90 minutes at 37°C in the dark. The catalytic reaction was quenched by adding 200 μL 0.5M NaOH/Gly-cine buffer, pH 10.3, and then centrifuged for 2 minutes at 4°C. The supernatant was transferred to a 96-well plate, and fluorescence was measured at 365 nm excitation wavelength and 450 nm emission wavelength with Infinite M200 reader (TECAN).

**Example 1. Testing the RNA editing method of the invention on a reporter**

It has been reported that Cas13 family proteins (C2c2) can edit RNA in mammalian cells. We further tested this system under various conditions. First, we constructed a dual fluorescence reporter system based on mCherry and EGFP fluorescence by introducing 3×GS linker targeting sequence containing stop codon between mCherry and EGFP gene. In addition, we deleted the start codon ATG of EGFP in order to reduce the leakage of EGFP translation.
Dual fluorescence reporter-1 comprises sequence of mCherry (SEQ ID NO:1), sequence comprising 3×GS linker and the targeted A (SEQ ID NO:2), and sequence of eGFP (SEQ ID NO:3).

ATGGTGAGCAAGGGCGAGGAGGATAACATGGCCATCATCAAGGAGTTCAT
GCGCTTCAAGGTGCACATGGAGGGCTCCGTGAACGGCCACGAGTTCGAGA

TCGAGGGCGAGGGCGAGGGCCGCCCCTACGAGGGCACCCAGACCGCCAA
GCTGAAGGTGACCAAGGGTGGCCCCCTGCCCTTCGCCTGGGACATCCTGTC
CCCTCAGTTCATGTACGGCTCCAAGGCCTACGTGAAGCACCCCGCCGACAT
CCCCGACTACTTGAAGCTGTCCTTCCCCGAGGGCTTCAAGTGGGAGCGCGT
GATGAACTTCGAGGACGGCGGCGTGGTGACCGTGACCCAGGACTCCTCCC
TGCAGGACGGCGAGTTCATCTACAAGGTGAAGCTGCGCGGCACCAACTTC
CCCTCCGACGGCCCCGTAATGCAGAAGAAGACCATGGGCTGGGAGGCCTC
CTCCGAGCGGATGTACCCCGAGGACGGCGCCCTGAAGGGCGAGATCAAGC
AGAGGCTGAAGCTGAAGGACGGCGGCCACTACGACGCTGAGGTCAAGAC
CACCTACAAGGCCAAGAAGCCCGTGCAGCTGCCCGGCGCCTACAACGTCA
ACATCAAGTTGGACATCACCTCCCACAACGAGGACTACACCATCGTGGAAC
AGTACGAACGCGCCGAGGGCCGCCACTCCACCGGCGGCATGGACGAGCTG
TACAAG (sequence of mCherry) (SEQ ID NO:1)

CTGCAG***GGCGGAGGAGGCAGCGGCGGAGGAGGCAGCGGCGGAGGAGGCA***
***GC***AGAAGGTATACACGCCGGAAGAATCTGT**A**GAGATCCCCGGTCGCCACC

(sequence comprising 3×GS linker (shown as italic and bold characters) and the targeted A (shown as larger and bold A)) (SEQ ID NO:2)

GTGAGCAAGGGCGAGGAGCTGTTCACCGGGGTGGTGCCCATCCTGGTCGA

GCTGGACGGCGACGTAAACGGCCACAAGTTCAGCGTGTCCGGCGAGGGCG

AGGGCGATGCCACCTACGGCAAGCTGACCCTGAAGTTCATCTGCACCACC

GGCAAGCTGCCCGTGCCCTGGCCCACCCTCGTGACCACCCTGACCTACGG

CGTGCAGTGCTTCAGCCGCTACCCCGACCACATGAAGCAGCACGACTTCTT

CAAGTCCGCCATGCCCGAAGGCTACGTCCAGGAGCGCACCATCTTCTTCAA

GGACGACGGCAACTACAAGACCCGCGCCGAGGTGAAGTTCGAGGGCGAC

ACCCTGGTGAACCGCATCGAGCTGAAGGGCATCGACTTCAAGGAGGACGG

CAACATCCTGGGGCACAAGCTGGAGTACAACTACAACAGCCACAACGTCT

ATATCATGGCCGACAAGCAGAAGAACGGCATCAAGGTGAACTTCAAGATC

CGCCACAACATCGAGGACGGCAGCGTGCAGCTCGCCGACCACTACCAGCA

GAACACCCCCATCGGCGACGGCCCCGTGCTGCTGCCCGACAACCACTACC

TGAGCACCCAGTCCGCCCTGAGCAAAGACCCCAACGAGAAGCGCGATCAC

ATGGTCCTGCTGGAGTTCGTGACCGCCGCCGGGATCACTCTCGGCATGGAC

GAGCTGTACAAGTAA (sequence of eGFP) (SEQ ID NO:3)

Dual fluorescence reporter-2 comprises sequence of mCherry (SEQ ID NO:1), sequence comprising 3×GS linker (shown as italic and bold characters) and the targeted A (shown as larger and bold A) (SEQ ID NO:4), and sequence of eGFP (SEQ ID NO:3).
CTGCAG***GGCGGAGGAGGCAGCGGCGGAGGAGGCAGCGGCGGAGGAGGCA GC***GCCTGCTCGCGATGCT**A**GAGGGCTCTGCCA (sequence comprising 3×GS linker (shown as italic and bold characters) and the targeted A (shown as larger and bold characters)) (SEQ ID NO:4))

Dual fluorescence reporter-3 comprises sequence of mCherry (SEQ ID NO:1), sequence comprising 1×GS linker (shown as italic and bold characters) and the targeted A (SEQ ID NO:5), and sequence of eGFP (SEQ ID NO:3).
CTGCAG***GGCGGAGGAGGC***AGCGCCTGCTCGCGATGCT**A**GAGGGCTCTGCC A (sequence comprising 1×GS linker (shown as italic and bold characters) and the targeted A (shown as larger and bold A)) (SEQ ID NO:5)

We cloned mCherry-3×GS linker-TAG-EGFP into pLenti-backbone, and the reporter plasmid was packed into lentivirus, which infected 293T cells constructing stable cell line expressing the dual fluorescence reporter. Then, we selected a single clone with low EGFP fluorescence background as the reporter system. We tiled LbucC2c2 crRNA guides with spacers from 28 to 78 nucleotides long across the targeting adenosine to test the optimal crRNA design. We found that longer crRNA guides conferred higher EGFP positive efficiency. Strikingly, when we transfected targeting crRNA plasmids without co-transfection of any dC2c2-ADARDD-expressing plasmids, the EGFP protein is substantially expressed. For example, the crRNA guide with the sequence: GGACCACCCCAAAAAUGAAUAUAACCAAAACU-GAACAGCUCCUCGCCCU UGCUCACUGGCAGAGCCUCCAGCAUCGCGAGCAGGCGCUGCCUCCUCC GCC (SEQ ID NO: 6) conferred over 25% EGFP positive efficiency. This indicates that adenine in the stop codon UAG is largely edited. In contrast, the random crRNA could not render the EGFP negative cells into positive (FIGs. 6A, 6B and 6C). Based on these results, we inferred that overexpression of a RNA transcript alone could leverage endogenous ADAR enzyme to edit RNA.

Further, we deleted the scaffold RNA sequence on the RNA guides, creating a linear guide RNA. We found 70-nucleotides long RNA (AAACCGAGGGAUCAUAGGGGACUGAAUCCACCAUUCUUCUCCCAAUCC CUGCAACUCCUUCUUCCCCUGC(SEQ ID NO: 7)) complementary to the targeting RNA with an A-C mismatch could efficiently convert the EGFP negative cells into EGFP positive cells, while the 70-nt random RNA (UGAACAGCUCCUCGCCCUUGCUCACUGGCAGAGCCCUCCAGCAUCGCGA GCAGGCGCUGCCUCCUCCGCC(SEQ ID NO: 8)) could not (FIGs. 1A, 1B, 1C, and 1D). We thus designate this RNA as dRNA (Deaminase-recruiting RNA). To verify that the cellular endogenous ADAR could be recruited to conduct adenine deamination by dRNA, we performed experiments in the Adar1 p110 and Adar1 p150 double knockout 293T cell lines (FIGs. 6E and 6F). Because Adar1 is ubiquitously expressed while Adar2 is mainly expressed in brain at high level. So we proposed the targeting Adenine deamination by dRNA was mainly mediated by Adar1 but not Adar2. As expected, the targeting dRNA could not trigger EGFP expression in 293T-Adar1-/- cells, but overexpressing either exogenous Adar1 p110, p150 or Adar2 could rescue the EGFP expression in 293T-Adar1-/- cells (FIGs. 1E and 1F), suggesting that in 293Tcells, the dRNA could recruit Adar1 or Adar2 to mediate adenine deamination on a target RNA. Moreover, we found Adar1-p110 and Adar2 have higher editing activity than Adar1-p150 (FIG. 1G and FIG. 6G), possible due to the different cell localization of Adar1-p110 and Adar1-p150.

In order to determine the restoration of EGFP fluorescence was due to the targeting RNA editing events, we directly measured the dRNA-mediated editing of Reporter-2 transcripts via RT-PCR followed by targeted Sanger sequencing and Next-generation sequencing. The sequencing results showed the A to G base conversion in the targeted Adenine (A-C mismatch site) and the editing rate could reach to 13% (FIG. 6H and FIG. 1H). Besides, we also observed slightly A to G editing during the sequence windows near the targeted Adenine, most possibly due to the increased duplex RNA regions, later, we would try to get rid of the unexpected editing with several strategies.

## Example 2. Optimizing the factors for designing dRNAs

Next, we set out to optimize the dRNA to achieve higher editing efficiency. First, we aimed to determine which base in the opposite site of the targeted adenine favors editing. Previous studies showed the opposite base of targeted adenosine would affect the editing efficiently. We thus designed 71nt dRNAs with a mismatch N (A, U, C and G) in the middle position opposite to targeted A. Based on the FACS results, we found that the four different dRNAs editing efficiently as follow: C> A> U> G (FIGs. 2A and 2B). Recently, it has been reported that little bubble in the target UAG site may be of benefit to the editing efficiency. Therefore, we designed dRNAs containing two or three mismatch bases with target UAG site to test our hypothesis. 16 different 71 nt dRNAs were designed and constructed on the dRNA vector with BFP marker using Golden Gate cloning method. We found that the dRNAs with CCA and GCA sequence are of the highest efficiency, which means the little bubble contribute little to A-I editing, at least in the case of UAG target site. Besides, four dRNAs of NCA sequence have higher percentage of GFP positive cells, leading to the conclusion that complementary U-A base pair may be important for ADAR editing (FIGs. 2C and 2D). Subsequently, we test the efficiency of different length of dRNA based on Reporter. dRNAs were designed a mismatch C in the middle position with different length ranging from 31 nt to 221 nt. We found that editing efficiency increases with longer dRNA. The peak of editing of reporter system is located at 171 nt dRNA. 51 nt dRNA could light up reporter system with a good efficiency (18%) (FIGs. 2E and 2F). Finally, we examined whether the position of mismatch C of dRNA affect the editing efficiency. dRNAs were kept the same 71 nt length, a mismatch C in different position from transcription beginning was designed. Based on the FACS results, we found that the location of the opposite mismatch C could affect the editing efficiency, and the mismatch C located in the 5' or 3' of dRNA has a lower efficiency (FIGs. 2G and 2H). 16 different reporter comprising target sequences containing all possible 3 base motifs were constructed through Gibson cloning, and then cloned into pLenti backbone (pLenti-CMV-MCS-SV-Bsd, Stanley Cohen Lab, Stanford University). The target sequences are shown as follows.

Target sequences containing all possible 3 base motifs:

TAT:

ATGGACGAGCTGTACAAGCTGCAGGGCGGAGGAGGCAGCGCCTGCTCGCG

ATGCTATAGGGCTCTGCCAGTGAGCAAGGGCGAGGAGCTGTTCACCGGGG

TGGTGCCCATC (SEQ ID NO: 9)

TAA:

ATGGACGAGCTGTACAAGCTGCAGGGCGGAGGAGGCAGCGCCTGCTCGCG
ATGCTAAAGGGCTCTGCCAGTGAGCAAGGGCGAGGAGCTGTTCACCGGGG
TGGTGCCCATC (SEQ ID NO: 10)

TAC:

ATGGACGAGCTGTACAAGCTGCAGGGCGGAGGAGGCAGCGCCTGCTCGCG
ATGCTACAGGGCTCTGCCAGTGAGCAAGGGCGAGGAGCTGTTCACCGGGG
TGGTGCCCATC (SEQ ID NO: 11)

TAG:

ATGGACGAGCTGTACAAGCTGCAGGGCGGAGGAGGCAGCGCCTGCTCGCG
ATGCTAGAGGGCTCTGCCAGTGAGCAAGGGCGAGGAGCTGTTCACCGGGG
TGGTGCCCATC (SEQ ID NO: 12)

AAT:

ATGGACGAGCTGTACAAGCTGCAGGGCGGAGGAGGCAGCGCCTGCTCGCG
ATGCAATAGGGCTCTGCCAGTGAGCAAGGGCGAGGAGCTGTTCACCGGGG
TGGTGCCCATC (SEQ ID NO: 13)

AAA:

ATGGACGAGCTGTACAAGCTGCAGGGCGGAGGAGGCAGCGCCTGCTCGCG
ATGCAAAAGGGCTCTGCCAGTGAGCAAGGGCGAGGAGCTGTTCACCGGGG
TGGTGCCCATC (SEQ ID NO: 14)

AAC:

ATGGACGAGCTGTACAAGCTGCAGGGCGGAGGAGGCAGCGCCTGCTCGCG
ATGCAACAGGGCTCTGCCAGTGAGCAAGGGCGAGGAGCTGTTCACCGGGG
TGGTGCCCATC (SEQ ID NO: 15)

AAG:

ATGGACGAGCTGTACAAGCTGCAGGGCGGAGGAGGCAGCGCCTGCTCGCG
ATGCAAGAGGGCTCTGCCAGTGAGCAAGGGCGAGGAGCTGTTCACCGGGG
TGGTGCCCATC (SEQ ID NO: 16)

CAT:

ATGGACGAGCTGTACAAGCTGCAGGGCGGAGGAGGCAGCGCCTGCTCGCG

ATGCCATAGGGCTCTGCCAGTGAGCAAGGGCGAGGAGCTGTTCACCGGGG

TGGTGCCCATC (SEQ ID NO: 17)

CAA:

ATGGACGAGCTGTACAAGCTGCAGGGCGGAGGAGGCAGCGCCTGCTCGCG

ATGCCAAAGGGCTCTGCCAGTGAGCAAGGGCGAGGAGCTGTTCACCGGGG

TGGTGCCCATC (SEQ ID NO: 18)

CAC:

ATGGACGAGCTGTACAAGCTGCAGGGCGGAGGAGGCAGCGCCTGCTCGCG

ATGCCACAGGGCTCTGCCAGTGAGCAAGGGCGAGGAGCTGTTCACCGGGG

TGGTGCCCATC (SEQ ID NO: 19)

CAG:

ATGGACGAGCTGTACAAGCTGCAGGGCGGAGGAGGCAGCGCCTGCTCGCG

ATGCCAGAGGGCTCTGCCAGTGAGCAAGGGCGAGGAGCTGTTCACCGGGG

TGGTGCCCATC (SEQ ID NO: 20)

GAT:

ATGGACGAGCTGTACAAGCTGCAGGGCGGAGGAGGCAGCGCCTGCTCGCG

ATGCGATAGGGCTCTGCCAGTGAGCAAGGGCGAGGAGCTGTTCACCGGGG

TGGTGCCCATC (SEQ ID NO: 21)

GAA:

ATGGACGAGCTGTACAAGCTGCAGGGCGGAGGAGGCAGCGCCTGCTCGCG

ATGCGAAAGGGCTCTGCCAGTGAGCAAGGGCGAGGAGCTGTTCACCGGGG

TGGTGCCCATC (SEQ ID NO: 22)

GAC:

ATGGACGAGCTGTACAAGCTGCAGGGCGGAGGAGGCAGCGCCTGCTCGCG

ATGCGACAGGGCTCTGCCAGTGAGCAAGGGCGAGGAGCTGTTCACCGGGG

TGGTGCCCATC (SEQ ID NO: 23)

GAG:

ATGGACGAGCTGTACAAGCTGCAGGGCGGAGGAGGCAGCGCCTGCTCGCG ATGCGAGAGGGCTCTGCCAGTGAGCAAGGGCGAGGAGCTGTTCACCGGGG TGGTGCCCATC (SEQ ID NO: 24)

dRNAs were kept same 111 bp length and designed a mismatch C at the center towards the target A.

In 12-well cell culture cluster, $2\times10^5$ cells HEK293T were plated to the each well and each experiment was performed for three replicates. 24 hrs later, 0.5 µg dRNA plasmid and 0.5 µg reporter target plasmid were co-transfected to the cells using the X-tremeGENE HP DNA transfection reagent (Roche). 48 hrs later, cells were trypsinized and selected for mCherry positive cells through FACS (BD). A total of $4\times10^5$ cells were harvested and total RNA was extracted using RNAprep pure Cell/Bacteria Kit (TIANGEN DP430). The cDNAs were synthesized from 2 µg of total RNA using Quantscript RT Kit (TIANGEN KR103-04). And the 111 target regions were amplified through PCR and sent for deep sequencing.

We found that all 16 different 3 base motifs can be edited through an exemplary RNA editing method of the present application, albeit with a variable efficiency. In sum, the results indicate the 5' nearest neighbor of A to be edited has the preference U>C≈A>G and 3' nearest neighbor of A to be edited has the preference G >C> A U. Data were presented as bar chart in FIG. 3A or heatmap of FIG. 3B.

**Example 3. Editing RNA transcribed from endogenous genes**

Next, we tested whether dRNA could mediate mRNA transcribed from endogenous genes. We designed dRNA targeting four genes KRAS, PPIB, β-Actin and GAPDH. For KRAS mRNA, we designed 91, 111, 131, 151, 171 and 191 nucleotides long dRNAs (FIG. 4A) with sequences as shown below.

91-nt KRAS-dRNA

UAGCUGUAUCGUCAAGGCACUCUUGCCUACGCCACCAGCUCCAACCACC ACAAGUUUAUAUUCAGUCAUUUUCAGCAGGCCUCUCUCCCGC (SEQ ID NO: 25)

111-nt KRAS-dRNA

GAUUCUGAAUUAGCUGUAUCGUCAAGGCACUCUUGCCUACGCCACCAGC UCCAACUACCACAAGUUUAUAUUCAGUCAUUUUCAGCAGGCCUCUCUCC CGCACCUGGGAGC (SEQ ID NO: 26)

131-nt KRAS-dRNA

UCCACAAAAUGAUUCUGAAUUAGCUGUAUCGUCAAGGCACUCUUGCCU ACGCCACCAGCUCCAACUACCACAAGUUUAUAUUCAGUCAUUUUCAGCA GGCCUCUCUCCCGCACCUGGGAGCCGCUGAGCCU (SEQ ID NO: 27)

151-nt KRAS-dRNA

AUCAUAUUCGUCCACAAAAUGAUUCUGAAUUAGCUGUAUCGUCAAGGC ACUCUUGCCUACGCCACCAGCUCCAACCACCACAAGUUUAUAUUCAGUC AUUUUCAGCAGGCCUCUCUCCCGCACCUGGGAGCCGCUGAGCCUCUGGC

CCCGC (SEQ ID NO: 28)

171-nt KRAS-dRNA

CUAUUGUUGGAUCAUAUUCGUCCACAAAAUGAUUCUGAAUUAGCUGUA
UCGUCAAGGCACUCUUGCCUACGCCACCAGCUCCAACCACCACAAGUUU
AUAUUCAGUCAUUUUCAGCAGGCCUCUCUCCCGCACCUGGGAGCCGCUG
AGCCUCUGGCCCCGCCGCCGCCUUC (SEQ ID NO: 29)

191-nt KRAS-dRNA

UAGGAAUCCUCUAUUGUUGGAUCAUAUUCGUCCACAAAAUGAUUCUGA
AUUAGCUGUAUCGUCAAGGCACUCUUGCCUACGCCACCAGCUCCAACCA
CCACAAGUUUAUAUUCAGUCAUUUUCAGCAGGCCUCUCUCCCGCACCUG
GGAGCCGCUGAGCCUCUGGCCCCGCCGCCGCCUUCAGUGCCUGCG　(SEQ
ID NO: 30)

The Next-generation sequencing results showed that the dRNAs could edit the targeted KRAS mRNA with up to 11.7% editing efficiency (FIG. 4B). For endogenous PPIB mRNA, the targeted three sites: site1, site2 and site3. We designed 151 nucleotides long dRNA for each site (FIG. 4C) with sequences as shown below.

151-nt PPIB-dRNA (site 1)

GAGGCGCAGCAUCCACAGGCGGAGGCGAAAGCAGCCCGGACAGCUGAGG
CCGGAAGAGGGUGGGGGCCGCGGUGGCCAGGGAGCCGGCGCCGCCACGCG
CGGGUGGGGGGGACUGGGGUUGCUCGCGGGCUCCGGGCGGGCGGCGGG
CGCCG (SEQ ID NO: 31)

151-nt PPIB-dRNA (site 2)

UCCUGUAGCUAAGGCCACAAAAUUAUCCACUGUUUUUGGAACAGUCUU
UCCGAAGAGACCAAAGAUCACCCGGCCCACAUCUUCAUCUCCAAUUCGU
AGGUCAAAAUACACCUUGACGGUGACUUUGGGCCCCUUCUUCUUCUCAU
CGGCC (SEQ ID NO: 32)

151-nt PPIB-dRNA (site 3)

GCCCUGGAUCAUGAAGUCCUUGAUUACACGAUGGAAUUUGCUGUUUUU
GUAGCCAAAUCCUUUCUCUCCUGUAGCCAAGGCCACAAAAUUAUCCACU
GUUUUUGGAACAGUCUUUCCGAAGAGACCAAAGAUCACCCGGCCUACA
UCUUCA (SEQ ID NO: 33)

The Next-generation sequencing results showed that the dRNA could edit PPIB mRNA site1 efficiently with up to 14% editing rate (FIG. 4D). For PPIB mRNA site2 and site3, the editing efficiency was 1.5% and 0.6% (FIG. 4E and 4F). For endogenous β-Actin mRNA, we selected two targeted site and designed dRNA for each site (FIG. 4G) with sequences as shown below.

72-nt β-Actin-dRNA (site 1)

GCGCAAGUUAGGUUUUGUCAAGAAAGGGUGUAACGCAACCAAGUCAUA GUCCGCCUAGAAGCAUUUGCGGUG (SEQ ID NO: 34)

131-nt β-Actin-dRNA (site 1)

GCCAUGCCAAUCUCAUCUUGUUUUCUGCGCAAGUUAGGUUUUGUCAAG AAAGGGUGUAACGCAACCAAGUCAUAGUCCGCCUAGAAGCAUUUGCGG UGGACGAUGGAGGGGCCGGACUCGUCAUACUCCUG (SEQ ID NO: 35)

70-nt β-Actin-dRNA (site 2) GGACUUCCUGUAACAACGCAUCUCAUAUUUGGAAUGACCAUUAAAAAA ACAACAAUGUGCAAUCAAAGUC (SEQ ID NO: 36)

We found that dRNA could edit β-Actin mRNA both site1 and site2, with up to 1.4% editing efficiency for each site (FIG. 4H and FIG. 8A). We also observed longer dRNA conferred higher editing efficiency, with 0.6% for dRNA-71nt and 1.4% for dRNA-131nt (FIG. 3H). For another housekeeping gene GAPDH, we used 71nt dRNA (CAAGGUGCGGCUCCGGCCCCUCCCCUCUUCAAGGGGUCCACAUGGCAAC UGUGAGGAGGGGAGAUUCAGUG (SEQ ID NO: 37)), and the editing efficiency is 0.3%, maybe due to the short dRNA length (FIG. 8B).

**Example 4. Off-targeting analysis on an exemplary LEAPER method**

For therapeutic application, the precision of editing is pivotal. Next, we tried to characterize the specificity of an exemplary RNA editing system of the present application. We selected endogenous PPIB site1 and KRAS site for analysis. For PPIB site1, we could see during the dRNA covered regions, there were several A bases flanking the targeted A76, such as A22, A30, A33, A34, A39, A49, A80, A91, A107 and A140. It revealed that those flanking A bases were barely edited, while the targeted A76 base (A-C mismatch) showed up to 14% editing efficiency (FIG. 5A and 5B).
As for KRAS site, we could see in the dRNA covered region, there are many adenines flanking the targeted A56 base, up to 29 flanking A bases. From the KRAS mRNA editing results, we found that while the targeted A56 base (A-C mismatch) showed up to 11.7% editing efficiency, the flanking adenine could be edited (FIG. 5C and 5D). A variety of the off-targeted adenines were edited, while adenines such as A41, A43, A45, A46, A74, A79 showed more editing. We found the 5' nearest neighbor of those unedited A bases were G or C, whereas the 5' nearest neighbor of those efficiently edited adenines was T or A. Based on this observation, we set out to design dRNA to minimize the off-target editing of those adenines that are prone to be edited. In our study, we have found ADAR preferred A-C mismatch to A-A, A-U, and, the A-G mismatch was the least preferred. So, we proposed that for the off-targeting A bases to which the 5' nearest neighbor was U or A, A-G mismatch might reduce or diminish the off-targeting effects. Previous study has reported A-G mismatch could block the deamination editing by ADAR.
So next we designed three kinds of 91-nt dRNA variants and four kinds of 111-nt dRNA variants (with sequences as shown below) containing different A-G mismatch combinations based on the statistical results in FIG. 5D and existing knowledge: dRNA-AG1 (A41, A46, A74); dRNA-AG2 (A41, A43, A45, A46, A74, A79); dRNA-AG3 (A31, A32, A33, A41, A43, A45, A46, A47, A74, A79); dRNA-AG4 (A7, A31, A32, A33, A40, A41, A43, A45, A46, A47, A74, A79, A95) (FIG. 4E).

KRAS-dRNA-91-AG2

UAGCUGUAUCGUCAAGGCACUCgUGCCgACGCCACCAGCUCCAACcACCA CAAGgggAgAgUCAGUCAgggUCAGCAGGCCUCUCUCCCGC (SEQ ID NO: 38)

KRAS-dRNA-91-AG3

UAGCUGUAUCGUCAAGGCACUCUUGCCgACGCCACCAGCUCCAACcACCA
CAAGUgUAUAgUCAGUCAUUUCAGCAGGCCUCUCUCCCGC (SEQ ID NO: 39)

KRAS-dRNA-91-AG4

UAGCUGGAUCGUCAAGGCACUCGUGCCGACGCCACCAGCUCCAACCACC
ACAAGGGGAGAGGCAGUCAGGGUCAGCAGGCCUCUCUCCCGC (SEQ ID NO: 40)

KRAS-dRNA-111-AG 1

GAUUCUGAAUUAGCUGUAUCGUCAAGGCACUCUUGCCgACGCCACCAGC
UCCAACcACCACAAGUgUAUAgUCAGUCAUUUCAGCAGGCCUCUCUCCC

GCACCUGGGAGC (SEQ ID NO:41)

KRAS-dRNA-111-AG2

GAUUCUGAAUUAGCUGUAUCGUCAAGGCACUCgUGCCgACGCCACCAGC
UCCAACcACCACAAGUggAgAgUCAGUCAUUUCAGCAGGCCUCUCUCCC
GCACCUGGGAGC (SEQ ID NO:42)

KRAS-dRNA-111-AG3

GAUUCUGAAUUAGCUGUAUCGUCAAGGCACUCgUGCCgACGCCACCAGC
UCCAACcACCACAAGgggAgAgUCAGUCAgggUCAGCAGGCCUCUCUCCCGC
ACCUGGGAGC (SEQ ID NO:43)

KRAS-dRNA-111-AG4

GCUCCCCGGUGCGGGAGAGAGGCCUGCUGACCCUGACUGCCUCUCCCCU
UGUGGUGGUUGGAGCUGGUGGCGUCGGCACGAGUGCCUUGACGAUCCA
GCUAAUUCAGAAUC (SEQ ID NO: 44)

Then these dRNAs were transfected into HEK293T cells, and empty vector and 71-nt non-targeting dRNA control: (TCTCAGTCCAATGTATGGTCCGAGCACAAGCTCTAATCAAAGTCCGCGGGT GTAGACCGGTTGCCATAGGA (SEQ ID NO: 45)) were used as negative controls. For 91-nt dRNAs, the deep sequencing results showed that the on-target editing (A56) was reduced to 2.8% for dRNA-91-AG2, 2.3% for dRNA-91-AG3 and 0.7% for dRNA-91-AG4, compared to the on-target editing (A56) efficiency 7.9% for dRNA-91 without A-G mismatch (FIG. 4F). For 91-nt dRNAs, the on-target editing (A56) was reduced to 5.1% for dRNA-111-AG2 and 4.9% for dRNA-111-AG3 compared to the on-target editing (A56) efficiency 15.1% for dRNA-111 without A-G mismatch (FIG. 4F), which indicating longer dRNA could bear more A-G mismatch. So next we selected 111-nt dRNA for detailed off-target analysis. The flanking A bases editing were wiped out dramatically except for A7 and A79 (FIG. 4G). For A7 base, the off-target effect could be prevented by a further A-G mismatch design at this site, which is absent in the current dRNA design. For A79 base, introducing adjacent two A-G mismatch A78/A79 might help to wipe out the off-target effects. Based on such results, applying the RNA editing systems of the present application to cure genetic diseases is very promising and encouraging.

**Example 5. Testing an exemplary LEAPER method in multiple cell lines**

Through the results in HEK293T cells, we supposed that the double strand RNA formed by linear dRNA and its target RNA could recruit endogenous ADAR protein for A-I editing. To confirm the hypothesis, we chose more cell lines to test our RNA editing method. The results are shown in FIG. 9. Those results in multiple cell lines proved the universality of our RNA editing method. Firstly, despite of the various editing efficiency, using dRNA to recruit endogenous ADAR was suitable for multiple human cell lines, which was originated from 7 different tissues and organs. Furthermore, this method could not only work in human cells, but also in mouse cells, providing the possibility to conduct experiments on a mouse.

**Example 6. Leveraging endogenous ADAR for RNA editing**

In an attempt to explore an efficient RNA editing platform, we fused the deaminase domain of the hyperactive E1008Q mutant ADAR1 ($ADAR1_{DD}$)[40] to the catalytic inactive LbuCas13 (dCas13a), an RNA-guided RNA-targeting CRISPR effector[41] (Fig. 10A). To assess RNA editing efficiency, we constructed a surrogate reporter harbouring *mCherry* and *EGFP* genes linked by a sequence comprising a $3\times$ GGGGS-coding region and an in-frame UAG stop codon (Reporter-1, Fig. 10B). The reporter-transfected cells only expressed mCherry protein, while targeted editing on the UAG of the reporter transcript could convert the stop codon to UIG and consequently permit the downstream EGFP expression. Such a reporter allows us to measure the A-to-I editing efficiency through monitoring EGFP level. We then designed hU6 promoter-driven crRNAs (CRISPR RNAs) containing 5' scaffolds subjected for Cas13a recognition and variable lengths of spacer sequences for targeting (crRNA$^{Cas13a}$, following LbuCas13 crRNA sequences). LbuCas13 crRNA sequences

| Name | Sequence | Source |
|---|---|---|
| LbuCas13/Cas13a crRNA scaffold | Ggaccaccccaaaaaugaaggggacuaaaac (SEQ ID NO: 46) | FIG. 10 |
| Ctrl crRNA$_{70}$ | Aaaccgagggaucauaggggacugaauccaccauucuucucc caaucccugcaacuccuucuuccccugc (SEQ ID NO: 47) | FIG. 10 |
| Spacer of crRNA$_{15}$ | gcagagccucCagc (SEQ ID NO: 48) | FIG. 10 |
| Spacer of crRNA$_{22}$ | cucacuggcagagccucCagc (SEQ ID NO: 49) | FIG. 10 |
| Spacer of crRNA$_{28}$ | cccuugcucacuggcagagccucCagc (SEQ ID NO: 50) | FIG. 10 |
| Spacer of crRNA$_{35}$ | cucucgcccuugcucacuggcagagccucCagc (SEQ ID NO: 51) | FIG. 10 |
| Spacer of crRNA$_{40}$ | cucucgcccuugcucacuggcagagccucCagcaucgc (SEQ ID NO: 52) | FIG. 10 |
| Spacer of crRNA$_{47}$ | ugaacagcucucgcccuugcucacuggcagagccucCagcauc gc (SEQ ID NO: 53) | FIG. 10 |
| Spacer of crRNA$_{70}$ | ugaacagcuccucgcccuugcucacuggcagagcccucCagca ucgcgagcaggcgcugccuccuccgcc (SEQ ID NO: 54) | FIG. 10 |

The sequences complementary to the target transcripts all contain CCA opposite to the UAG codon so as to introduce a cytidine (C) mis-pairing with the adenosine (A) (Fig. 10B) because adenosine deamination preferentially occurs in the A-C mismatch site[13,14]. To test the optimal length of the crRNA, non-targeting or targeting crRNAs of different lengths were co-expressed with dCas13a-ADAR1$_{DD}$ proteins in HEK293T cells stably expressing the Reporter-1. Evident RNA editing effects indicated by the appearance of EGFP expression were observed with crRNAs containing matching sequences at least 40-nt long, and the longer the crRNAs the higher the EGFP positive percentage (Fig. 10C).

Surprisingly, expression of long crRNA[Cas13a] alone appeared sufficient to activate EGFP expression, and the co-expression of dCas13a-ADAR1$_{DD}$ rather decreased crRNA activity (Figs. 10C, 10d). The EGFP expression was clearly sequence-dependent because the 70-nt (exclusive of the 5' scaffold for the length calculation) control RNA could not activate EGFP expression (Figs. 10C, 10D).

With the surprising finding that certain long engineered crRNA[Cas13a] enabled RNA editing independent of dCas13a-ADAR1$_{DD}$, we decided to remove the Cas13a-recruiting scaffold sequence from the crRNA. Because the crRNA$_{70}$ had the highest activity to trigger EGFP expression (Fig. 10C, 10D), we chose the same 70-nt long guide RNA without the Cas13a-recruiting scaffold for further test (Fig. 11A and the following Sequences of arRNAs and control RNAs used in the examples).

| Name | Sequence (5' ---> 3') | Source |
|---|---|---|
| Ctrl RNA$_{70}$ | Aaaccgagggaucauaggggacugaauccaccauucuucucccaaucccugcaacuccuucu uccccugc (SEQ ID NO: 55) | Fig. 11 |
| arRNA$_{70}$ | ugaacagcuccucgcccuugcucacuggcagagcccucCagcaucgcgagcaggcgcugccu ccuccgcc (SEQ ID NO: 56) | |
| Ctrl RNA$_{71}$ | Ucucaguccaauguaugguccgagcacaagcucuaaucaaaguccgcggguguagaccggu ugccauagga (SEQ ID NO: 57) | Fig. 14 and Fig. 16 |
| arRNA$_{71}$ | acagcuccucgcccuugcucacuggcagagcccucCagcaucgcgagcaggcgcugccuccu ccgccgcug (SEQ ID NO: 58) | |
| arRNA$_{71}$-CAA | acagcuccucgcccuugcucacuggcagagcccucAagcaucgcgagcaggcgcugccuccu ccgccgcug (SEQ ID NO: 59) | Fig. 16A |
| arRNA$_{71}$-CUA | acagcuccucgcccuugcucacuggcagagcccucUagcaucgcgagcaggcgcugccuccu ccgccgcug (SEQ ID NO: 60) | |
| arRNA$_{71}$-CGA | acagcuccucgcccuugcucacuggcagagcccucGagcaucgcgagcaggcgcugccuccu ccgccgcug (SEQ ID NO: 61) | |

(continued)

| Name | Sequence (5' ---> 3') | Source |
|------|------------------------|--------|
| arRNA$_{71}$-GCA | acagcuccucgcccuugcucacuggcagagcccuGCAgcaucgcgagcaggcgcugccucc uccgccgcug (SEQ ID NO: 62) | |
| arRNA$_{71}$-UCA | acagcuccucgcccuugcucacuggcagagcccuUCAgcaucgcgagcaggcgcugccuccu ccgccgcug (SEQ ID NO: 63) | |
| arRNA$_{71}$-ACA | acagcuccucgcccuugcucacuggcagagcccuACAgcaucgcgagcaggcgcugccuccu ccgccgcug (SEQ ID NO: 64) | |
| arRNA$_{71}$-CCU | acagcuccucgcccuugcucacuggcagagcccuCCUgcaucgcgagcaggcgcugccucc uccgccgcug (SEQ ID NO: 65) | |
| arRNA$_{71}$-GCU | acagcuccucgcccuugcucacuggcagagcccuGCUgcaucgcgagcaggcgcugccucc uccgccgcug (SEQ ID NO: 66) | |
| arRNA$_{71}$-UCU | acagcuccucgcccuugcucacuggcagagcccuUCUgcaucgcgagcaggcgcugccucc uccgccgcug (SEQ ID NO: 67) | |
| arRNA$_{71}$-ACU | acagcuccucgcccuugcucacuggcagagcccuACUgcaucgcgagcaggcgcugccuccu ccgccgcug (SEQ ID NO: 68) | Fig. 16B, C |
| arRNA$_{71}$-CCC | acagcuccucgcccuugcucacuggcagagcccuCCCgcaucgcgagcaggcgcugccucc uccgccgcug (SEQ ID NO: 69) | |
| arRNA$_{71}$-GCC | acagcuccucgcccuugcucacuggcagagcccuGCCgcaucgcgagcaggcgcugccucc uccgccgcug (SEQ ID NO: 70) | |
| arRNA$_{71}$-UCC | acagcuccucgcccuugcucacuggcagagcccuUCCgcaucgcgagcaggcgcugccucc uccgccgcug (SEQ ID NO: 71) | |
| arRNA$_{71}$-ACC | acagcuccucgcccuugcucacuggcagagcccuACCgcaucgcgagcaggcgcugccuccu ccgccgcug (SEQ ID NO: 72) | |
| arRNA$_{71}$-CCG | acagcuccucgcccuugcucacuggcagagcccuCCGgcaucgcgagcaggcgcugccucc uccgccgcug (SEQ ID NO: 73) | |
| arRNA$_{71}$-GCG | acagcuccucgcccuugcucacuggcagagcccuGCUgcaucgcgagcaggcgcugccucc uccgccgcug (SEQ ID NO: 74) | |
| arRNA$_{71}$-UCG | acagcuccucgcccuugcucacuggcagagcccuUCGgcaucgcgagcaggcgcugccucc uccgccgcug (SEQ ID NO: 75) | |
| arRNA$_{71}$-ACG | acagcuccucgcccuugcucacuggcagagcccuACGgcaucgcgagcaggcgcugccucc uccgccgcug (SEQ ID NO: 76) | |

(continued)

| Name | Sequence (5' ---> 3') | Source |
|------|----------------------|--------|
| arRNAs -Reporter-1 | acuggcagagcccucCagcaucgcgagcagg (SEQ ID NO: 77) | Fig. 16D and Fig. 27 |
| arRNA$_{51}$-Reporter-1 | gcccuugcucacuggcagagcccucCagcaucgcgagcaggcgcugccucc (SEQ ID NO: 78) | |
| arRNA$_{91}$-Reporter-1 | acagcuccucgcccuugcucacuggcagagcccucCagcaucgcgagcaggcgcugccuccuccgccgcug( SEQ ID NO: 79) | |
| arRNA$_{111}$-Reporter-1 | accccggugaacagcuccucgcccuugcucacuggcagagcccucCagcaucgcgagcaggcgcugccuccuccgccgcugccuccuccgc (SEQ ID NO: 80) | |
| arRNA$_{131}$-Reporter-1 | gcucgaccaggaugggcaccaccccggugaacagcuccucgcccuugcucacuggcagagcccucCagcaucgcgagcaggcgcugccuccuccgccgcugccuccuccgccgcugccuccuccgcccugc (SEQ ID NO: 81) | |
| arRNA1$_{51}$-Reporter-1 | ucgccguccagcucgaccaggaugggcaccaccccggugaacagcuccucgcccuugcucacuggcagagcccucCagcaucgcgagcaggcgcugccuccuccgccgcugccuccuccgccgcugccuccuccgcccugcagcuuguaca (SEQ ID NO: 82) | |
| arRNA$_{171}$-Reporter-1 | gccguuuacgucgccguccagcucgaccaggaugggcaccaccccggugaacagcuccucgcccuugcucacuggcagagcccucCagcaucgcgagcaggcgcugccuccuccgccgcugccuccuccgccgcugccuccuccgcccugcagcuuguacagcucguccau (SEQ ID NO: 83) | |

(continued)

| Name | Sequence (5' ---> 3') | Source |
|---|---|---|
| arRNA$_{191}$-Reporter-1 | ugaacuugugggccguuuacgucgccguccagcucgaccaggauggcaccaccccggugaacagcuccucgc ccuugcucacuggcagagcccucCagcaucgcgagcaggcgcugccuccuccgccgcugccuccuccgccgc ugccuccuccgcccugcagcuuguacagcucguccaugccgccggug<br>(SEQ ID NO: 84) | |
| arRNA$_{211}$-Reporter-1 | ccggacacgcugaacuugugggccguuuacgucgccguccagcucgaccaggauggcaccaccccggugaac agcuccucgcccuugcucacuggcagagcccucCagcaucgcgagcaggcgcugccuccuccgccgcugccu ccuccgccgcugccuccuccgcccugcagcuuguacagcucguccaugccgccgguggaguggcggc<br>(SEQ ID NO: 85) | |
| arRNA$_{31}$-Reporter-2 | gcgaccggggaucucCacagauucuuccggc (SEQ ID NO: 86) | |
| arRNA$_{51}$-Reporter-2 | gcucacgguggcgaccggggaucucCacagauucuuccggcguguauaccu (SEQ ID NO: 87) | |
| arRNA$_{71}$-Reporter-2 | ccucgcccuugcucacgguggcgaccggggaucucCacagauucuuccggcguguauaccuu cugcugccu( SEQ ID NO: 88) | |
| arRNA$_{91}$-Reporter-2 | gugaacagcuccucgcccuugcucacgguggcgaccggggaucucCacagauucuuccggcguguauaccu ucugcugccuccuccgccgc<br>(SEQ ID NO: 89) | |
| arRNA$_{111}$-Reporter-2 | caccaccccggugaacagcuccucgcccuugcucacgguggcgaccggggaucucCacagauucuuccggcg uguauaccuucugcugccuccuccgccgcugccuccucc<br>(SEQ ID NO: 90) | |
| arRNA$_{131}$-Reporter-2 | ccaggaugggcaccaccccggugaacagcuccucgcccuugcucacgguggcgaccggggaucucCacagau ucuuccggcguguauaccuucugcugccuccuccgccgcugccuccuccgccgcugccu<br>(SEQ ID NO: 91) | |
| arRNA$_{151}$-Reporter-2 | uccagcucgaccaggaugggcaccaccccggugaacagcuccucgcccuugcucacgguggcgaccggggau cucCacagauucuuccggcguguauaccuucugcugccuccuccgccgcugccuccuccgccgcugccuccu ccgcccu<br>(SEQ ID NO: 92) | |
| arRNA$_{171}$-Reporter-2 | cggcgacguaccagcucgaccaggaugggcaccaccccggugaacagcuccucgcccuugcucacgguggc gaccggggaucucCacagauucuuccggcguguauaccuucugcugccuccuccgccgcugccuccuccgcc gcugccuccuccgcccugcagcuugua<br>(SEQ ID NO: 93) | |
| arRNA$_{191}$-Reporter-2 | uguggccguuuacgucgccguccagcucgaccaggaugggcaccaccccggugaacagcuccucgcccuugc ucacgguggcgaccggggaucucCacagauucuuccggcguguauaccuucugcugccuccuccgccgcug ccuccuccgccgcugccuccuccgcccugcagcuuguacagcucgucc<br>(SEQ ID NO: 94) | |
| arRNA$_{211}$-Reporter-2 | acgcugaacuugugggccguuuacgucgccguccagcucgaccaggaugggcaccaccccggugaacagcucc ucgcccuugcucacgguggcgaccggggaucucCacagauucuuccggcguguauaccuucugcugccucc uccgccgcugccuccuccgccgcugccuccuccgcccugcagcuuguacagcucguccaugccgccgg<br>(SEQ ID NO: 95) | |
| arRNA$_{71}$(C+70)-Reporter-1 | Cagcaucgcgagcaggcgcugccuccuccgccgcugccuccuccgccgcugccuccuccgccc ugcagcuu (SEQ ID NO: 96) | Fig. 16E |
| arRNA$_{71}$(5+C+65)-Reporter-1 | cccucCagcaucgcgagcaggcgcugccuccuccgccgcugccuccuccgccgcugccuccuc cgcccugc (SEQ ID NO: 97) | |
| arRNA$_{71}$(10+C+60)-Reporter-1 | cagagcccucCagcaucgcgagcaggcgcugccuccuccgccgcugccuccuccgccgcugcc uccuccgc (SEQ ID NO: 98) | |
| arRNA$_{71}$(15+C+55)-Reporter 1 | acuggcagagcccuccCagcaucgcgagcaggcgcugccuccuccgccgcugccuccuccgcc gcugccucc (SEQ ID NO: 99) | |

(continued)

| Name | Sequence (5' ---> 3') | Source |
|---|---|---|
| arRNA$_{71}$(20+C+50)-Reporter-1 | ugcucacuggcagagcccucCagcaucgcgagcaggcgcugccuccuccgccgcugccuccuccgccgcug (SEQ ID NO: 100) | |
| arRNA$_{71}$(25+C+45)-Reporter-1 | gcccuugcucacuggcagagcccucCagcaucgcgagcaggcgcugccuccuccgccgcgcuccuccgc (SEQ ID NO: 101) | |
| arRNA$_{71}$(30+C+40)-Reporter-1 | uccucgcccuugcucacuggcagagcccucCagcaucgcgagcaggcgcugccuccuccgccgcugccucc (SEQ ID NO: 102) | |
| arRNA$_{71}$(40+C+30)-Reporter-1 | ggugaacagcuccucgcccuugcucacuggcagagcccucCagcaucgcgagcaggcgcugccuccuccgc (SEQ ID NO: 103) | |
| arRNA$_{71}$(45+C+25)-Reporter-1 | accccggugaacagcuccucgcccuugcucacuggcagagcccucCagcaucgcgagcaggcgcugccucc (SEQ ID NO: 104) | |
| arRNA$_{71}$(50+C+20)-Reporter-1 | gcaccaccccggugaacagcuccucgcccuugcucacuggcagagcccucCagcaucgcgagcaggcgcug (SEQ ID NO: 105) | |
| arRNA$_{71}$(55+C+15)-Reporter-1 | gaugggcaccaccccggugaacagcuccucgcccuugcucacuggcagagcccucCagcaucgcgagcagg (SEQ ID NO: 106) | |
| arRNA$_{71}$(60+C+10)-Reporter-1 | accaggaugggcaccaccccggugaacagcuccucgcccuugcucacuggcagagcccucCagcaucgcga (SEQ ID NO: 107) | |
| arRNA$_{71}$(65+C+5)-Reporter-1 | gcucgaccaggaugggcaccaccccggugaacagcuccucgcccuugcucacuggcagagcccucCagcau (SEQ ID NO: 108) | |
| arRNA$_{71}$(70+C)-Reporter-1 | guccagcucgaccaggaugggcaccaccccggugaacagcuccucgcccuugcucacuggcagagcccucC (SEQ ID NO: 109) | |
| arRNA$_{71}$(C+70)-Reporter-2 | Cacagauucuuccggcguguauaccuucugcugccuccuccgccgcugccuccuccgccgcugccuccucc (SEQ ID NO: 110) | |
| arRNA$_{71}$(5+C+65)-Reporter-2 | aucucCacagauucuuccggcguguauaccuucugcugccuccuccgccgcugccuccuccgccgcugccu (SEQ ID NO: 111) | |
| arRNA$_{71}$(10+C+60)-Reporter-2 | cggggaucucCacagauucuuccggcguguauaccuucugcugccuccuccgccgcugccuccuccgccgc (SEQ ID NO: 112) | |
| arRNA$_{71}$(15+C+55)-Reporter-2 | gcgaccggggaucucCacagauucuuccggcguguauaccuucugcugccuccuccgccgcugccuccucc (SEQ ID NO: 113) | |
| arRNA$_{71}$(20+C+50)-Reporter-2 | cgguggcgaccggggaucucCacagauucuuccggcguguauaccuucugcugccuccuccgccgcugccu (SEQ ID NO: 114) | |
| arRNA$_{71}$(25+C+45)-Reporter-2 | gcucacgguggcgaccggggaucucCacagauucuuccggcguguauaccuucugcugccuccuccgccgc (SEQ ID NO: 115) | |
| arRNA$_{71}$(30+C+40)-Reporter-2 | cccuugcucacgguggcgaccggggaucucCacagauucuuccggcguguauaccuucugcugccuccucc (SEQ ID NO: 116) | |
| arRNA$_{71}$(40+C+30)-Reporter-2 | cagcuccucgcccuugcucacgguggcgaccggggaucucCacagauucuuccggcguguauaccuucugc (SEQ ID NO: 117) | |

(continued)

| Name | Sequence (5' ---> 3') | Source |
|---|---|---|
| arRNA$_{71}$(45+C +25)-Reporter-2 | gugaacagcuccucgcccuugcucacggguggcgaccggggaucucCacagauucuuccggcg uguauaccu (SEQ ID NO: 118) | |
| arRNA$_{71}$(50+C +20)-Reporter-2 | ccccggugaacagcuccucgcccuugcucacgguggcgaccggggaucucCacagauucuuc cggcgugua (SEQ ID NO: 119) | |
| arRNA$_{71}$(55+C +15)-Reporter-2 | caccaccccggugaacagcuccucgcccuugcucacgguggcgaccggggaucucCacagau ucuuccggc (SEQ ID NO: 120) | |
| arRNA$_{71}$(60+C +10)-Reporter-2 | augggcaccaccccggugaacagcuccucgcccuugcucacgguggcgaccggggaucucCa cagauucuu (SEQ ID NO: 121) | |
| arRNA$_{71}$(65+C +5)-Reporter-2 | ccaggaugggcaccaccccggugaacagcuccucgcccuugcucacgguggcgaccgggggau cucCacaga (SEQ ID NO: 122) | |
| arRNA$_{71}$(70+C) -Reporter-2 | cucgaccaggaugggcaccaccccggugaacagcuccucgcccuugcucacgguggcgaccg gggaucucC (SEQ ID NO: 123) | |

(continued)

| Name | Sequence (5' ---> 3') | Source |
|---|---|---|
| arRNA$_{111}$-CCA-Reporter-3 (UAG) | gaugggcaccaccccggugaacagcuccucgcccuugcucacuggcagagcccucCagcaucgcgagcaggc gcugccuccuccgcccugcagcuuguacagcucguccau (SEQ ID NO: 124) | |
| arRNA$_{111}$-GCA-Reporter-3 (UAC) | gaugggcaccaccccggugaacagcuccucgcccuugcucacuggcagagcccugCagcaucgcgagcaggc gcugccuccuccgcccugcagcuuguacagcucguccau (SEQ ID NO: 125) | |
| arRNA$_{111}$-UCA-Reporter-3 (UAA) | gaugggcaccaccccggugaacagcuccucgcccuugcucacuggcagagcccuuCagcaucgcgagcaggc gcugccuccuccgcccugcagcuuguacagcucguccau (SEQ ID NO: 126) | |
| arRNA$_{111}$-ACA-Reporter-3 (UAU) | gaugggcaccaccccggugaacagcuccucgcccuugcucacuggcagagcccuaCagcaucgcgagcaggc gcugccuccuccgcccugcagcuuguacagcucguccau (SEQ ID NO: 127) | |
| arRNA$_{111}$-CCG-Reporter-3 (CAG) | gaugggcaccaccccggugaacagcuccucgcccuugcucacuggcagagcccucCggcaucgcgagcaggc gcugccuccuccgcccugcagcuuguacagcucguccau (SEQ ID NO: 128) | |
| arRNA$_{111}$-GCG-Reporter-3 (CAC) | gaugggcaccaccccggugaacagcuccucgcccuugcucacuggcagagcccugCggcaucgcgagcaggc gcugccuccuccgcccugcagcuuguacagcucguccau (SEQ ID NO: 129) | Fig. 16F, G |
| arRNA$_{111}$-UCG-Reporter-3 (CAA) | gaugggcaccaccccggugaacagcuccucgcccuugcucacuggcagagcccuuCggcaucgcgagcaggc gcugccuccuccgcccugcagcuuguacagcucguccau (SEQ ID NO: 130) | |
| arRNA$_{111}$-ACG-Reporter-3 (CAU) | gaugggcaccaccccggugaacagcuccucgcccuugcucacuggcagagcccuaCggcaucgcgagcaggc gcugccuccuccgcccugcagcuuguacagcucguccau (SEQ ID NO: 131) | |
| arRNA$_{111}$-CCU-Reporter-3 (AAG) | gaugggcaccaccccggugaacagcuccucgcccuugcucacuggcagagcccucCugcaucgcgagcaggc gcugccuccuccgcccugcagcuuguacagcucguccau (SEQ ID NO: 132) | |
| arRNA$_{111}$-GCU-Reporter-3 (AAC) | gaugggcaccaccccggugaacagcuccucgcccuugcucacuggcagagcccugCugcaucgcgagcaggc gcugccuccuccgcccugcagcuuguacagcucguccau (SEQ ID NO: 133) | |
| arRNA$_{111}$-ACU-Reporter-3 (AAA) | gaugggcaccaccccggugaacagcuccucgcccuugcucacuggcagagcccuaCugcaucgcgagcaggc gcugccuccuccgcccugcagcuuguacagcucguccau (SEQ ID NO: 134) | |
| arRNA$_{111}$-UCU-Reporter-3 (AAU) | gaugggcaccaccccggugaacagcuccucgcccuugcucacuggcagagcccuuCugcaucgcgagcaggc gcugccuccuccgcccugcagcuuguacagcucguccau (SEQ ID NO: 135) | |

(continued)

| Name | Sequence (5' ---> 3') | Source |
|---|---|---|
| arRNA$_{111}$-CCC-Reporter-3 (GAG) | gaugggcaccacccccggugaacagcuccucgcccuugcucacuggcagagcccucCcgcaucgcgagcaggcgcugccuccuccgcccugcagcuuguacagcucguccau (SEQ ID NO: 136) | |
| arRNA$_{111}$-GCC-Reporter-3 (GAC) | gaugggcaccacccccggugaacagcuccucgcccuugcucacuggcagagcccugCcgcaucgcgagcaggcgcugccuccuccgcccugcagcuuguacagcucguccau (SEQ ID NO: 137) | |
| arRNA$_{111}$-UCC-Reporter-3 (GAA) | gaugggcaccacccccggugaacagcuccucgcccuugcucacuggcagagcccuuCcgcaucgcgagcaggcgcugccuccuccgcccugcagcuuguacagcucguccau (SEQ ID NO: 138) | |
| arRNA$_{111}$-ACC-Reporter-3 (GAU) | gaugggcaccacccccggugaacagcuccucgcccuugcucacuggcagagcccuaCcgcaucgcgagcaggcgcugccuccuccgcccugcagcuuguacagcucguccau (SEQ ID NO: 139) | |
| Ctrl RNA$_{111}$ | Uaccgcuacagccacgcugauuucagcuauaccugcccgguauaaagggacguucacaccgcgauguucucugcuggggaauugcgcgauauucaggauuaaaagaagugc (SEQ ID NO: 140) | Fig.17 |
| Ctrl RNA$_{151}$ | Acuacaguugcuccgauauuuaggcuacgucaauaggcacuaacuuauuggcgcuggugaacggacuuccucucgaguaccagaagaugacuacaaaacuccuuuccauugcgaguaucggagucuggcucaguuuggccagggaggcacu (SEQ ID NO: 141) | |

(continued)

| Name | Sequence (5' ---> 3') | Source |
|------|----------------------|--------|
| arRNA$_{51}$-PPIB | cggaagagggguggggccgcggguggcCagggagccggcgccgccacgcgcgg<br>(SEQ ID NO: 142) | |
| arRNA$_{71}$-PPIB | cagcugaggccggaagagggguggggccgcggguggcCagggagccggcgccgccacgcgcgggugggggggga<br>(SEQ ID NO: 143) | |
| arRNA$_{111}$-PPIB | ggaggcgaaagcagcccggacagcugaggccggaagagggguggggccgcggguggcCagggagccggcgccg<br>ccacgcgcgggugggggggacugggguugcucgcgggcuc<br>(SEQ ID NO: 144) | |
| arRNA$_{151}$-PPIB | gaggcgcagcauccacaggcggaggcgaaagcagcccggacagcugaggccggaagagggguggggccgcggu<br>ggcCagggagccggcgccgccacgcgcgggugggggggacuggggguugcucgcgggcuccgggcgggcgg<br>cgggcgccg<br>(SEQ ID NO: 145) | |
| arRNA$_{51}$-KRAS | ucuugccuacgccaccagcuccaacCaccacaaguuuauauucagucauuu (SEQ ID NO: 146) | |
| arRNA$_{71}$-KRAS | gucaaggcacucuugccuacgccaccagcuccaacCaccacaaguuuauauucagucauuuu<br>cagcaggcc | |
| arRNA$_{111}$-KRA S | GauucugaauuagcuguaucgucaaggcacucuugccuacgccaccagcuccaacCaccacaaguuuauauu<br>cagucauuuucagcaggccucucucccgcaccugggagc<br>(SEQ ID NO: 147) | Fig. 17B |
| arRNA$_{151}$-KRA S | aucauauucguccacaaaaugauucugaauuagcuguaucgucaaggcacucuugccuacgccaccagcucca<br>acCaccacaaguuuauauucagucauuuucagcaggccucucucccgcaccugggagccgcugagccucugg<br>ccccgc<br>(SEQ ID NO: 148) | |
| arRNA$_{51}$-SMA D4 | ucggcaugguaugaaguacuucgucCaggagcuggagggcccgguguaagu (SEQ ID NO: 149) | |
| arRNA$_{71}$-SMA D4 | gggucugcaaucggcaugguaugaaguacuucgucCaggagcuggagggcccgguguaagu<br>gaauuucaau (SEQ ID NO: 150) | |
| arRNA$_{111}$-SMA D4 | gaccucagucuaaagguugugggucugcaaucggcaugguaugaaguacuucgucCaggagcuggagggcc<br>cgguguaagugaauuucaauccagcaaggguguuucuuuga<br>(SEQ ID NO: 151) | |
| arRNA$_{151}$-SMA D4 | uaagggccccaacgguaaaagaccucagucuaaagguugugggucugcaaucggcaugguaugaaguacuuc<br>gucCaggagcuggagggcccgguguaagugaauuucaauccagcaagguguuucuuugaugcucugucuug<br>gguaaucc<br>(SEQ ID NO: 152) | |
| arRNA$_{51}$-FANC C (TAC site) | uggggggguucggcugccgacaucagCaauugcucugccaccaucucagccc (SEQ ID NO: 153) | |
| arRNA$_{71}$-FANC C (TAC site) | agcagggccgugggggguucggcugccgacaucagCaauugcucugccaccaucucagccca<br>uccuccgaa (SEQ ID NO: 154) | |
| arRNA$_{111}$-FAN CC (TAC site) | aguagaaggccaagagccacagcagggccgugggggguucggcugccgacaucagCaauugcucugccacca<br>ucucagcccauccuccgaagugaaugaacaggaaccagc<br>(SEQ ID NO: 155) | |
| arRNA$_{151}$-FAN CC (TAC site) | ccucccaucacggggggccguaguagaaggccaagagccacagcagggccgugggggguucggcugccgacau<br>cagCaauugcucugccaccaucucagcccauccuccgaagugaaugaacaggaaccagcucucaaagggaccu<br>ccgcag<br>(SEQ ID NO: 156) | |

(continued)

| Name | Sequence (5' ---> 3') | Source |
|------|----------------------|--------|
| arRNA$_{151}$-PPIB (AAG site) | gccaaacaccacatgcttgccatctagccaggctgtcttgactgtcgtgatgaagaactgggagccgttggtgtcCttgcc tgcgttggccatgctcacccagccaggcccgtagtgcttcagtttgaagttctcatcggggaagcgctca (SEQ ID NO: 157) | |
| arRNA$_{151}$-PPIB (CAG site) | gggagtgggtccgctccaccagatgccagcaccggggccagtgcagctcagagccctgtggcggactacagggccC gcacagacggtcactcaaagaaagatgtccctgtgccctactccttggcgatggcaaagggcttctccacctcga (SEQ ID NO: 158) | Fig. 17C |
| arRNA$_{151}$-FAN CC (AAG site) | tgcattttgtaaaatagatactagcagattgtcccaagatgtgtacagctcattctcacagcccagcgagggcacCtactcc acaaatgcgtggccacaggtcatcacctgtcctgtggccctggcgagcctgatccctcacgccgggcac (SEQ ID NO: 159) | |
| arRNA$_{151}$-FAN CC (CAG site) | gctcattctcacagcccagcgagggcacttactccacaaatgcgtggccacaggtcatcacctgtcctgtggcccCggc gagcctgatccctcacgccgggcacccacacggcctgcgtgccttctagacttgagttcgcagctctttaag (SEQ ID NO: 160) | |
| arRNA$_{151}$-IDU A (CAG site) | tcggccgggccctgggggcggtgggcgctggccaggacgcccaccgtgtggttgctgtccaggacggtcccggccC gcgacacttcggcccagagctgctcctcatccagcagcgccagcagccccatggccgtgagcaccggcttgcgca (SEQ ID NO: 161) | |
| arRNA$_{111}$-TAR DBP | ugaccagucuuaagaaucuuucuugaccugcaccauaagaacuucuccaaagguacCaaaauacucuuucagg uccuguucgguuguuuuccaugggagacccaacacuauu (SEQ ID NO: 162) | Fig. 17D |
| arRNA$_{111}$-CGA -Reporter-3 (UAG) | gaugggcaccaccccggugaacagcuccucgcccuugcucacuggcagagcccucGagcaucgcgagcaggc gcugccuccuccgcccugcagcuuguacagcucguccau (SEQ ID NO: 163) | |
| arRNA$_{111}$-GGA -Reporter-3 (UAC) | gaugggcaccaccccggugaacagcuccucgcccuugcucacuggcagagcccugGagcaucgcgagcaggc gcugccuccuccgcccugcagcuuguacagcucguccau (SEQ ID NO: 164) | |
| arRNA$_{111}$-UGA -Reporter-3 (UAA) | gaugggcaccaccccggugaacagcuccucgcccuugcucacuggcagagcccuuGagcaucgcgagcaggc gcugccuccuccgcccugcagcuuguacagcucguccau (SEQ ID NO: 165) | |
| arRNA$_{111}$-AGA -Reporter-3 (UAU) | gaugggcaccaccccggugaacagcuccucgcccuugcucacuggcagagcccuaGagcaucgcgagcaggc gcugccuccuccgcccugcagcuuguacagcucguccau (SEQ ID NO: 166) | |
| arRNAtti-CGG -Reporter-3 (CAG) | gaugggcaccaccccggugaacagcuccucgcccuugcucacuggcagagcccucGggcaucgcgagcaggc gcugccuccuccgcccugcagcuuguacagcucguccau (SEQ ID NO: 167) | Fig. 17G |
| arRNA$_{111}$-GGG -Reporter-3 (CAC) | gaugggcaccaccccggugaacagcuccucgcccuugcucacuggcagagcccugGggcaucgcgagcaggc gcugccuccuccgcccugcagcuuguacagcucguccau (SEQ ID NO: 168) | |
| arRNA$_{111}$-UGG -Reporter-3 (CAA) | gaugggcaccaccccggugaacagcuccucgcccuugcucacuggcagagcccuuGggcaucgcgagcaggc gcugccuccuccgcccugcagcuuguacagcucguccau (SEQ ID NO: 169) | |
| arRNA$_{111}$-AGG -Reporter-3 (CAU) | gaugggcaccaccccggugaacagcuccucgcccuugcucacuggcagagcccuaGggcaucgcgagcaggc gcugccuccuccgcccugcagcuuguacagcucguccau (SEQ ID NO: 170) | |
| arRNA$_{111}$-CGU -Reporter-3 (AAG) | gaugggcaccaccccggugaacagcuccucgcccuugcucacuggcagagcccucGugcaucgcgagcaggc gcugccuccuccgcccugcagcuuguacagcucguccau (SEQ ID NO: 171) | |

(continued)

| Name | Sequence (5' ---> 3') | Source |
|---|---|---|
| arRNA$_{111}$-GGU -Reporter-3 (AAC) | gaugggcaccaccccggugaacagcuccucgcccuugcucacuggcagagcccugGugcaucgcgagcaggc gcugccuccuccgcccugcagcuuguacagcucguccau (SEQ ID NO: 172) | |
| arRNA$_{111}$-AGU -Reporter-3 (AAA) | gaugggcaccaccccggugaacagcuccucgcccuugcucacuggcagagcccuaGugcaucgcgagcaggc gcugccuccuccgcccugcagcuuguacagcucguccau (SEQ ID NO: 173) | |
| arRNA$_{111}$-UGU -Reporter-3 (AAU) | gaugggcaccaccccggugaacagcuccucgcccuugcucacuggcagagcccuuGugcaucgcgagcaggc gcugccuccuccgcccugcagcuuguacagcucguccau (SEQ ID NO: 174) | |
| arRNA$_{111}$-CGC- Reporter-3 (GAG) | gaugggcaccaccccggugaacagcuccucgcccuugcucacuggcagagcccucGcgcaucgcgagcaggc gcugccuccuccgcccugcagcuuguacagcucguccau (SEQ ID NO: 175) | |
| arRNA$_{111}$-GGC -Reporter-3 (GAC) | gaugggcaccaccccggugaacagcuccucgcccuugcucacuggcagagcccugGcgcaucgcgagcaggc gcugccuccuccgcccugcagcuuguacagcucguccau (SEQ ID NO: 176) | |
| arRNA$_{111}$-UGC -Reporter-3 (GAA) | gaugggcaccaccccggugaacagcuccucgcccuugcucacuggcagagcccuuGcgcaucgcgagcaggc gcugccuccuccgcccugcagcuuguacagcucguccau (SEQ ID NO: 177) | |
| arRNA$_{111}$-AGC -Reporter-3 (GAU) | gaugggcaccaccccggugaacagcuccucgcccuugcucacuggcagagcccuaGcgcaucgcgagcaggc gcugccuccuccgcccugcagcuuguacagcucguccau (SEQ ID NO: 178) | |

(continued)

| Name | Sequence (5' ---> 3') | Source |
|------|----------------------|--------|
| arRNA$_{111}$-CGA -Reporter-3 (UAG) | gaugggcaccaccccggugaacagcuccucgcccuugcucacuggcagagcccucGagcaucgcgagcaggc gcugccuccuccgcccugcagcuuguacagcucguccau (SEQ ID NO: 179) | |
| arRNA$_{111}$-GGA -Reporter-3 (UAG) | gaugggcaccaccccggugaacagcuccucgcccuugcucacuggcagagcccuGGagcaucgcgagcaggc gcugccuccuccgcccugcagcuuguacagcucguccau (SEQ ID NO: 180) | |
| arRNA$_{111}$-UGA -Reporter-3 (UAG) | gaugggcaccaccccggugaacagcuccucgcccuugcucacuggcagagcccuUGagcaucgcgagcaggc gcugccuccuccgcccugcagcuuguacagcucguccau (SEQ ID NO: 181) | |
| arRNA$_{111}$-AGA -Reporter-3 (UAG) | gaugggcaccaccccggugaacagcuccucgcccuugcucacuggcagagcccuAGagcaucgcgagcaggc gcugccuccuccgcccugcagcuuguacagcucguccau (SEQ ID NO: 182) | |
| arRNA$_{111}$-CGU -Reporter-3 (UAG) | gaugggcaccaccccggugaacagcuccucgcccuugcucacuggcagagcccucGUgcaucgcgagcaggc gcugccuccuccgcccugcagcuuguacagcucguccau (SEQ ID NO: 183) | |
| arRNA$_{111}$-CGG -Reporter-3 (UAG) | gaugggcaccaccccggugaacagcuccucgcccuugcucacuggcagagcccucGGgcaucgcgagcaggc gcugccuccuccgcccugcagcuuguacagcucguccau (SEQ ID NO: 184) | Fig. 17H |
| arRNA$_{111}$-CGC- Reporter-3 (UAG) | gaugggcaccaccccggugaacagcuccucgcccuugcucacuggcagagcccucGCgcaucgcgagcaggc gcugccuccuccgcccugcagcuuguacagcucguccau (SEQ ID NO: 185) | |
| arRNA$_{111}$-CGU -Reporter-3 (AAG) | gaugggcaccaccccggugaacagcuccucgcccuugcucacuggcagagcccucGugcaucgcgagcaggc gcugccuccuccgcccugcagcuuguacagcucguccau (SEQ ID NO: 186) | |
| arRNA$_{111}$-GGU -Reporter-3 (AAG) | gaugggcaccaccccggugaacagcuccucgcccuugcucacuggcagagcccuGGugcaucgcgagcagg cgcugccuccuccgcccugcagcuuguacagcucguccau (SEQ ID NO: 187) | |
| arRNA$_{111}$-UGU -Reporter-3 (AAG) | gaugggcaccaccccggugaacagcuccucgcccuugcucacuggcagagcccuUGugcaucgcgagcagg cgcugccuccuccgcccugcagcuuguacagcucguccau (SEQ ID NO: 188) | |
| arRNA$_{111}$-AGU -Reporter-3 (AAG) | gaugggcaccaccccggugaacagcuccucgcccuugcucacuggcagagcccuAGugcaucgcgagcagg cgcugccuccuccgcccugcagcuuguacagcucguccau (SEQ ID NO: 189) | |
| arRNA$_{111}$-CGA - Reporter-3 (AAG) | gaugggcaccaccccggugaacagcuccucgcccuugcucacuggcagagcccucGAgcaucgcgagcaggc gcugccuccuccgcccugcagcuuguacagcucguccau (SEQ ID NO: 190) | |
| arRNA$_{111}$-CGC- Reporter-3 (AAG) | gaugggcaccaccccggugaacagcuccucgcccuugcucacuggcagagcccucGCgcaucgcgagcaggc gcugccuccuccgcccugcagcuuguacagcucguccau (SEQ ID NO: 191) | |
| arRNA$_{111}$-CGG -Reporter-3 (AAG) | gaugggcaccaccccggugaacagcuccucgcccuugcucacuggcagagcccucGGgcaucgcgagcaggc gcugccuccuccgcccugcagcuuguacagcucguccau (SEQ ID NO: 192) | |

(continued)

| Name | Sequence (5' ---> 3') | Source |
|------|----------------------|--------|
| arRNA<sub>111</sub>-KRAS-AG6 | gauucugaauuagcuguaucgucaaggcacucgugccgacgccaccagcuccaacCaccacaaguggagagu cagucauuuucagcaggccucucucucccgcaccugggagc (SEQ ID NO: 193) | Fig. 17I |
| arRNA<sub>111</sub>-KRAS-AG9 | gauucugaauuagcuggaucgucaaggcacucgggccgacgccaccagcuccaacCaccacaaguggagagu cagucauuuucagcaggccucucucucccgcaccggggagc (SEQ ID NO: 194) | |
| arRNA<sub>111</sub>-TP53 | gggagcagccucuggcauucugggagcuucaucuggaccugggucuucagugaacCauuguucaauaucgu ccggggacagcaucaaaucauccauugcuugggacggcaa (SEQ ID NO: 195) | Fig. 23 |
| arRNA<sub>111</sub>-TP53-AG1 | gggagcagccucuggcauucugggagcuucaucuggaccugggucuucagugaacCauuguucaagaucgu ccggggacagcaucaaaucauccauugcuugggacggcaa (SEQ ID NO: 196) | |
| arRNA<sub>111</sub>-TP53-AG4 | gggagcagccucuggcagucggggagcuucaucuggaccugggucuucagugaacCauuguucaagaucgu ccggggacagcaucaaaucauccagugcuugggacggcaa (SEQ ID NO: 197) | |
| arRNA<sub>111</sub>-COL3A1 | cauauuacagaauaccuugauagcauccaauuugcauccuugguuagggucaaccCaguauucuccacucuu gaguucaggauggcagaauuucaggucucugcaguuucu (SEQ ID NO: 198) | Fig.26 |
| arRNA<sub>111</sub>-BMPR2 | gugaagauaagccaguccucuaguaacagaaugagcaagacggcaagagcuuaccCagucacuuguguggag acuuaaauacuugcauaaagauccauugggauaguacuc (SEQ ID NO: 199) | |
| arRNA<sub>111</sub>-AHI1 | gugaacgucaaacugucggaccaauauggcagaaucuucucucaucucaacuuucCauauccguaucaugga aucauagcauccuguaacuacuagcucucuuacagcugg (SEQ ID NO: 200) | |
| arRNA<sub>111</sub>-FANCC (Site 2) | gccaaugaucucgugaguuaucucagcagugugagccaucagggugaugacauccCaggcgaucguguggc cuccaggagcccagagcaggaaguugaggagaaggugccu (SEQ ID NO: 201) | |
| arRNA<sub>111</sub>-MYBPC3 | caagacggugaaccacuccauggucuucuugucggcuuucugcacugugguaccccCagagcuccguguugc cgacauccuggggguggcuuccacuccagagccacauuaag (SEQ ID NO: 202) | |
| arRNA<sub>111</sub>-IL2RG | aggauucucuuuugaaguauuugcucccccagugggauuggguggcuccauucacucCaaugcugagcacuuc cacagaguggguuaaagcggcuccgaacacgaaacgugua (SEQ ID NO: 203) | |
| arRNA<sub>111</sub>-IDUA-V1 | gacgcccaccgugugguugcuguccaggacggucccggccugcgacacuucggccCagagcugcuccucauc cagcagcgccagcagccccauggccgugagcaccggcuu (SEQ ID NO: 204) | Fig.29 |
| arRNA<sub>111</sub>-IDUA-V2 | gacgcccaccgugugguugcuguccaggacggucccggccugcgacacuucggccCagagcugcuccucauc ugcggggcggggggggggccgucgccgcgugggggucguug (SEQ ID NO: 205) | |

It turned out that this linear guide RNA induced strong EGFP expression in close to 40% of total cells harboring the Reporter-1 (Fig. 11B, upper). Because endogenous ADAR proteins could edit double-stranded RNA (dsRNA) substrates[12], we reasoned that the long guide RNAs could anneal with the target transcripts to form dsRNA substrates that in turn recruit endogenous ADAR proteins for targeted editing. We thus designated such guide RNA as arRNA (ADAR-recruiting RNA).

To verify if endogenous ADAR proteins are indeed responsible for above observation, we set out to examine the arRNA-mediated RNA editing in ADAR-deficient cells. Since *ADAR2* mRNA was barely detectable in HEK293T cells (Fig. 12A), we generated HEK293T *ADAR1*<sup>-/-</sup> cells, rendering this cell line deficient in both ADAR1 and ADAR2 (Fig. 11C, D). Indeed, the depletion of ADAR1 abrogated arRNA<sub>70</sub>-induced EGFP signals (Fig. 11B, lower). Moreover, exogenous expression of ADAR1$^{p110}$, ADAR1$^{p150}$ or ADAR2 in HEK293T *ADAR1$^{-/-}$* cells (Fig. 11C, D) successfully rescued the loss of EGFP induction by arRNA$_{70}$ (Fig. 11E, Fig. 12B), demonstrating that arRNA-induced EGFP reporter expression solely depended on native ADAR1, whose activity could be reconstituted by its either isoforms (p110 and p150) or ADAR2. Sanger sequencing analysis on the arRNA$_{70}$-targeting region showed an A/G overlapping peak at the predicted adenosine site within UAG, indicating a significant A to I (G) conversion (Fig. 11F). The next-generation sequencing (NGS) further confirmed that the A to I conversion rate was about 13% of total reporter transcripts (Fig. 11G). The quantitative PCR analysis showed that arRNA$_{70}$ did not reduce the expression of targeted transcripts (Fig. 13), ruling out the possible RNAi effect of the arRNA. Collectively, our data demonstrated that the arRNA is capable of generating significant level of editing on the targeted transcripts through the engineered A-C mismatch.

**Example 7. LEAPER enables RNA editing in multiple cell lines**

Because the expression of endogenous ADAR proteins is a prerequisite for LEAPER-mediated RNA editing, we tested the performance of LEAPER in a panel of cell lines originated from distinct tissues, including HT29, A549, HepG2, RD, SF268, SW13 and HeLa. We first examined the endogenous expression of all three kinds of ADAR proteins using Western blotting analyses. ADAR1 was highly expressed in all tested cell lines, and its identity in the Western blots was confirmed by the negative control, HEK293T *ADAR1$^{-/-}$* line (Fig. 14A, B). ADAR3 was detected only in HepG2 and HeLa cells (Fig. 14A, B). ADAR2 was non-detectable in any cells, a result that was not due to the failure of Western blotting because ADAR2 protein could be detected from ADAR2-overexpressing HEK293T cells (Fig. 14A, B). These findings are in consistent with previous reports that ADAR1 is ubiquitously expressed, while the expressions of ADAR2 and ADAR3 are restricted to certain tissues[11].

We then set out to test the editing efficiencies of a re-designed 71-nt arRNA (arRNA$_{71}$) targeting the Reporter-1 (Fig. 15A and Sequences of arRNAs and control RNAs used in this study listed above) in these cell lines.

LEAPER worked in all tested cells for this arRNA$_{71}$, albeit with varying efficiencies (Fig. 14C). These results were in agreement with the prior report that the ADAR1/2 protein levels correlate with the RNA editing yield[42], with the exception of HepG2 and HeLa cells. The suboptimal correlations of editing efficiencies with ADAR1 levels were likely due to the abundant ADAR3 expressions in these two lines (Fig. 14A, B) because it has been reported that ADAR3 plays an inhibitory role in RNA editing. Importantly, LEAPER also worked in three different cell lines of mouse origin (NIH3T3, Mouse Embryonic Fibroblast (MEF) and B16) (Fig. 14D), paving the way for testing its therapeutics potential through animal and disease models. Collectively, we conclude that LEAPER is a versatile tool for wide-spectrum of cell types, and for different organisms.

**Example 8. Characterization and optimization of LEAPER**

To better characterize and optimize LEAPER, we investigated the choices of nucleotide opposite to the adenosine within the UAG triplet of the targeted transcript. In HEK293T cells, Reporter-1-targeting arRNA$_{71}$ showed variable editing efficiencies with a changed triplet (5'-CNA, N denotes one of A/U/C/G) opposite to the targeted UAG (Sequences of arRNAs and control RNAs used in this study listed above). A-C mismatch resulted in the highest editing efficiency, and the A-G mismatch yielded the least but evident edits (Fig. 16A). We then investigated the preference of nucleotides flanking the A-C mismatch in arRNA. We tested all 16 combinations of 5' and 3' neighbor sites surrounding the cytidine (5'-N$^1$CN$^2$) (Sequences of arRNAs and control RNAs used in this study listed above), and found that the 3' neighboring adenosine was required for the efficient editing, while adenosine is the least favorable nucleotide at the 5' site (Fig. 16B, C). We thus concluded that CCA motif on the arRNA confers the highest editing efficiency targeting the UAG site. It is worthwhile to note that the 3' neighboring guanosine (5'-N$^1$CG) in arRNA showed a dramatic inhibitory effect (Fig. 16B, C).

Length of RNA appeared relevant to arRNA efficiency in directing the editing on the targeted transcripts (Fig. 10C), consistent with a previous report[42]. To fully understand this effect, we tested arRNAs with variable lengths targeting two different reporter transcripts -Reporter-1 and Reporter-2 (Fig. 15A, B). For either reporter targeting, arRNAs of 10 different sizes were designed and tested, ranging from 31-nt to 211-nt, with CCA triplet (for UAG targeting) right in the middle (Sequences of arRNAs and control RNAs used in this study listed above). Based on the reporter EGFP activities, the length of arRNA correlated positively with the editing efficiency, for both reporters, peaking at 111- to191-nt (Fig. 16D). Although one arRNA$_{51}$ appeared working, 71-nt was the minimal length for arRNA to work for both reporters (Fig. 16D).

Next, we investigated the effect of the A-C mismatch position within an arRNA on editing efficiency. We fixed the lengths of all arRNAs for testing to 71-nt, and slid the UAG-targeting ACC triplet from 5' to 3' within arRNAs (Sequences of arRNAs and control RNAs used in this study listed above). It turned out that placing the A-C mismatch in the middle region resulted in high editing yield, and arRNAs with the mismatch sites close to the 3' end outperformed those close to the 5' end in both reporters (Fig. 16E). For convenience, we placed the A-C mismatch at the center of arRNAs for all of our subsequent studies.

We also tested the targeting flexibility of LEAPER and tried to determine whether UAG on target is the only motif subjected to RNA editing. For all 16 triplet combinations ($5'-N^1AN^2$) on Reporter-3 (Fig. 15C), we used the corresponding arRNAs with the fixed lengths (111-nt) and ensured the perfect sequencing match for arRNA and the reporter except for the editing site (A-C mismatch) (Fig. 16F and Sequences of arRNAs and control RNAs used in this study listed above). NGS results showed that all $N^1AN^2$ motifs could be edited. The $UAN^2$ and $GAN^2$ are the most and the least preferable motifs, respectively (Fig. 16F, G). Collectively, the nearest neighbor preference of the target adenosine is 5' U>C≈A>G and 3' G>C>A≈U (Fig. 16G).

## Example 9. Editing endogenous transcripts using LEAPER

Next, we examined if LEAPER could enable effective editing on endogenous transcripts. Using arRNAs of different lengths, we targeted the UAG motifs in the transcripts of *PPIB, KRAS* and *SMAD4* genes, and an UAC motif in *FANCC* gene transcript (Fig. 17A, Sequences of arRNAs and control RNAs used in this study listed above). Encouragingly, targeted adenosine sites in all four transcripts were edited by their corresponding arRNAs with all four sizes, albeit with variable efficiencies according to NGS results (Fig. 17B). In consistent with our prior observation, longer arRNAs tended to yield higher editing rates. Of note, the 151-nt arRNA$^{PPIB}$ edited ~50% of total transcripts of *PPIB* gene (Fig. 17B). No arRNAs showed RNAi effects on their targeted transcripts (Fig. 18A) or ultimate protein level (e.g. KRAS, Fig. 18B). Besides, LEAPER is able to achieve desirable editing rate on non-UAN sites (Fig. 17C and Sequences of arRNAs and control RNAs used in this study listed above), showing the flexibility of LEAPER on editing endogenous transcripts. To further explore the power of LEAPER, we tested whether it could simultaneously target multiple sites. We observed multiplex editing of both *TARDBP* and *FANCC* transcripts by co-expression of two arRNAs (Sequences of arRNAs and control RNAs used in this study listed above), with the efficiency even higher than those with individual arRNAs (Fig. 17D), indicating that LEAPER is well suited for editing multiple targets in parallel.

It is noteworthy that ADAR1/2 tend to promiscuously deaminate multiple adenosines in an RNA duplex[44] and the A-C mismatch is not the only motif to guide the A-to-I switch (Fig. 16A). It is therefore reasonable to assume that all adenosines on target transcripts within the arRNA coverages are subjected to variable levels of editing, major sources of unwanted modifications. The longer the arRNA, the higher the possibility of such off-targets. We therefore examined all adenosine sites within the arRNA covering regions in these targeted transcripts. For *PPIB* transcripts, very little off-target editing was observed throughout the sequencing window for variable sizes of arRNAs (Fig. 17E, F). However, in the cases of targeting *KRAS, SMAD4* and *FANCC* genes, multiple off-target edits were detected (Fig. 19A-F). For *KRAS* in particular, 11 out of 30 adenosines underwent substantial A to I conversions in the sequencing window of arRNA$_{111}$ (Fig. 19A, B).

We next attempted to develop strategies to minimize such unwanted off-target effects. Because an A-G mismatch suppressed editing for UAG targeting (Fig. 16A), we postulated that pairing a guanosine with a non-targeting adenosine might reduce undesirable editing. We then tested the effect of the A-G mismatch on adenosine in all possible triplet combinations ($5'-N^1AN^2$) as in Reporter-3 (Fig. 15C and Sequences of arRNAs and control RNAs used in this study listed above). A-G mismatch indeed decreased the editing on adenosine in all tested targets, except for UAG or AAG targeting (~2%) (Fig. 17G), in comparison with A-C mismatch (Fig. 16F). To further reduce editing rates at unwanted sites, we went on testing the effect of two consecutive mismatches. It turned out that the additional mismatch at the 3' end nucleotide of the triplet opposite to either UAG or AAG, abolished its corresponding adenosine editing (Fig. 17H and Sequences of arRNAs and control RNAs used in this study listed above). In light of these findings, we attempted to apply this rule to reduce off-targets in *KRAS* transcripts (Fig. 19A). We first designed an arRNA (arRNA$_{111}$-AG6) that created A-G mismatches on all "editing-prone" motifs covered by arRNA$_{111}$ (Fig. 17I, Fig. 19A and Sequences of arRNAs and control RNAs used in this study listed above), including AAU (the 61st), UAU (the 63rd), UAA (the 65th), AAA (the 66th), UAG (the 94th) and AAG (the 99th). This arRNA$_{111}$-AG6 eliminated most of the off-target editing, while maintained an on-target editing rate of ~ 5%. In consistent with the findings in Fig. 17G, the single A-G mismatch could not completely minimize editing in AAG motif (99th) (Fig. 17I and Fig. 19A). We then added more mismatches on arRNA$_{111}$-AG6, including a dual mismatch (5'-CGG opposite to the targeted motif 5'-AAG), plus three additional A-G mismatches to mitigate editing on the 27th, 98th and the 115th adenosines (arRNA$_{111}$-AG9) (Sequences of arRNAs and control RNAs used in this study listed above). Consequently, we achieved a much improved specificity for editing, without additional loss of editing rate on the targeted site (A76) (Fig. 17I). In summary, engineered LEAPER incorporating additional rules enables efficient and more precise RNA editing on endogenous transcripts.

## Example 10. RNA editing specificity of LEAPER

In addition to the possible off-target effects within the arRNA-covered dsRNA region, we were also concerned about the potential off-target effects on other transcripts through partial base pairing of arRNA. We then performed a transcriptome-wide RNA-sequencing analysis to evaluate the global off-target effects of LEAPER. Cells were transfected

with a Ctrl RNA$_{151}$ or a *PPIB*-specific arRNA (arRNAisi-PPIB) expressing plasmids before subjected to RNA-seq analysis. We identified six potential off-targets in the Ctrl RNA$_{151}$ group (Fig. 20A) and five in the arRNAisi-PPIB group (Fig. 20B), and the *PPIB* on-target rate based on NGS analysis was ~37% (Fig. 20B). Further analysis revealed that all sites, except for the two sites from *EIF2AK2* transcripts, were located in either SINE (Alu) or LINE regions (Fig. 20A, B), both are prone to ADAR-mediated editing[45], suggesting that these off-targets may not be derived from pairing between the target transcripts and the arRNA or control RNA. Of note, two off-targeting transcripts, *WDR73* and *SMYD4,* appeared in both groups, suggesting they are unlikely sequence-dependent RNA editing. Indeed, minimum free energy analysis suggested that all these possible off-target transcripts failed to form a stable duplex with either Ctrl RNA$_{151}$ or arRNAisi-PPIB (Fig. 20C). To further test if arRNA generates sequence-dependent off-targets, we selected potential off-target sites by comparing sequence similarity using NCBI BLAST for both arRNA$_{151}$-PPIB and arRNA$_{111}$-FANCC. *TRAPPC12* transcripts for arRNA$_{151}$-PPIB and three sites in the *ST3GAL1, 0STM1-AS1* and *EHD2* transcripts for arRNA$_{111}$-FANCC were top candidates (Fig. 20D and Fig. 21A). NGS analysis revealed that no editing could be detected in any of these predicted off-target sites (Fig. 20D and Fig. 21B). These results indicate that LEAPER empowers efficient editing at the targeted site, while maintaining transcriptome-wide specificity without detectable sequence-dependent off-target edits.

## Example 11. Safety assessment of LEAPER in mammalian cells

Because arRNAs rely on endogenous ADAR proteins for editing on target transcripts, we wondered if the addition of exogenous arRNAs affects native RNA editing events by occupying too much of ADAR1 or ADAR2 proteins. Therefore, we analyzed the A-to-I RNA editing sites shared by mock group and arRNA$_{151}$-PPIB group from the transcriptome-wide RNA-sequencing results, and the comparison between the mock group and Ctrl RNA$_{151}$ group was also analyzed. Neither Ctrl RNA$_{151}$ group nor arRNAisi-PPIB group showed a significant difference compared to the mock group (Fig. 22A, B), indicating that LEAPER had little impact on the normal function of endogenous ADAR1 to catalyze the native A-to-I editing events.

Meanwhile, we performed differential gene expression analysis using RNA-seq data to verify whether arRNA affects global gene expression. We found that neither Ctrl RNA$_{151}$ nor arRNAisi-PPIB affected the global gene expression in comparison with the mock group (Fig. 22C, D). In consistent with our prior observation (Fig. 18A), arRNAs did not show any RNAi effect on the expression of *PPIB* (Fig. 22C, D).

Considering that the arRNA forms RNA duplex with the target transcript and that RNA duplex might elicit innate immune response, we investigated if the introduction of arRNA has such an effect. To test this, we selected arRNAs targeting four gene transcripts that had been proven effective. We did not observe any mRNA induction of interferon-β (IFN-β) (Fig. 22E) or interleukin-6 (IL-6) (Fig. 22F), which are two hallmarks of innate immune activation. As a positive control, a synthetic analog of double-stranded RNA - poly(I:C) induced strong IFN-β and IL-6 expression (Fig. 22E, F). LEAPER does not seem to induce immunogenicity in target cells, a feature important for safe therapeutics.

## Example 12. Recovery of transcriptional regulatory activity of p53 by LEAPER

Now that we have established a novel method for RNA editing without the necessity of introducing foreign proteins, we attempted to demonstrate its therapeutic utility. We first targeted the tumor suppressor gene *TP53,* which is known to play a vital role in the maintenance of cellular homeostasis, but undergo frequent mutations in >50% of human cancers[46]. The c.158G>A mutation in *TP53* is a clinically-relevant nonsense mutation (Trp53Ter), resulting in a non-functional truncated protein[46]. We designed one arRNA$_{111}$ and two alternative arRNAs (arRNA$_{111}$-AG1 and arRNA$_{111}$-AG4) (Sequences of arRNAs and control RNAs used in this study listed above), all targeting *TP53*$^{W53X}$ transcripts (Fig. 23A), with the latter two being designed to minimize potential off-targets. We generated HEK293T *TP53*$^{-/-}$ cell line to eliminate the effects of native p53 protein. All three forms of *TP53*$^{W53X}$-targeting arRNAs converted ~25-35% of *TP53*$^{W53X}$ transcripts on the mutated adenosine site (Fig. 23B), with variable reductions of unwanted edits for arRNA$_{111}$-AG1 and arRNA$_{111}$-AG4 (Fig. 24). Western blot showed that arRNA$_{111}$, arRNA$_{111}$-AG1 and arRNA$_{111}$-AG4 could all rescue the production of full-length p53 protein based on the *TP53*$^{W53X}$ transcripts in HEK293T *TP53*$^{-/-}$ cells, while the Ctrl RNA$_{111}$ could not (Fig. 23C).

To verify whether the repaired p53 proteins are fully functional, we tested the transcriptional regulatory activity of p53 with a p53-luciferase *cis*-reporting system[47,48]. All three versions of arRNAs could restore p53 activity, and the optimized version arRNA$_{111}$-AG1 performed the best (Fig. 23D). In conclusion, we demonstrated that LEAPER is capable of repairing the cancer-relevant pre-mature stop codon of *TP53* and restoring its function.

## Example 13. Corrections of pathogenic mutations by LEAPER

We next investigated whether LEAPER could be used to correct more pathogenic mutations. Aiming at clinically relevant mutations from six pathogenic genes, *COL3A1* of Ehlers-Danlos syndrome, *BMPR2* of Primary pulmonary hypertension, *AHI1* of Joubert syndrome, *FANCC* of Fanconi anemia, *MYBPC3* of Primary familial hypertrophic cardiomyopathy and *IL2RG* of X-linked severe combined immunodeficiency, we designed 111-nt arRNAs for each of these genes carrying corresponding pathogenic G>A mutations (Fig. 25 and Sequences of arRNAs and control RNAs used in this study listed above, and the following disease-relevant cDNAs used in this study).

Disease-related cDNAs used in this study

| Candidate | Disease | Mutant Adenosine |
|---|---|---|
| NM_000090.3 (*COL3A1*) | Ehlers-Danlos syndrome, type 4 | c.3833G>A (p.Trp1278Ter) |
| NM_001204.6 (*BMPR2*) | Primary pulmonary hypertension | c.893G>A (p.Trp298Ter) |
| NM_017651.4 (*AHI1*) | Joubert syndrome 3 | c.2174G>A (p.Trp725Ter) |
| NM_000136.2 (*FANCC*) | Fanconi anemia, complementation group C | c.1517G>A (p.Trp506Ter) |
| NM_000256.3 (*MYBPC3*) | Primary familial hypertrophic cardiomyopathy | c.3293G>A (p.Trp 1098Ter) |
| NM_000206.2 (*IL2RG*) | X-linked severe combined immunodeficiency | c.710G>A (p.Trp237Ter) |

By co-expressing arRNA/cDNA pairs in HEK293T cells, we identified significant amounts of target transcripts with A>G corrections in all tests (Fig. 24). Because G>A mutations account for nearly half of known disease-causing point mutations in humans[10,49], the A>G conversion by LEAPER may offer immense opportunities for therapeutics.

## Example 14. RNA editing in multiple human primary cells by LEAPER

To further explore the clinical utility of LEAPER, we set out to test the method in multiple human primary cells. First, we tested LEAPER in human primary pulmonary fibroblasts and human primary bronchial epithelial cells with 151-nt arRNA (Sequences of arRNAs and control RNAs used in this study listed above) to edit the Reporter-1 (Fig. 15A). 35-45% of EGFP positive cells could be obtained by LEAPER in both human primary cells (Fig. 27A). We then tested LEAPER in editing endogenous gene *PPIB* in these two primary cells and human primary T cells, and found that arRNA$_{151}$-PPIB could achieve >40%, >80% and >30% of editing rates in human primary pulmonary fibroblasts, primary bronchial epithelial cells (Fig. 27B) and primary T cells (Fig. 27C), respectively. The high editing efficiency of LEAPER in human primary cells is particularly encouraging for its potential application in therapeutics.

## Example 15. Efficient editing by lentiviral expression and chemical synthesis of arRNAs

We then investigated if LEAPER could be delivered by more clinically-relevant methods. We first tested the effect of arRNA through lentivirus-based expression. Reporter-1-targeting arRNAisi induced strong EGFP expression in more than 40% of total cells harboring the Reporter-1 in HEK293T cells 2 days post infection (dpi). At 8 dpi, the EGFP ratio maintained at a comparable level of ~38% (Fig. 28A and Sequences of arRNAs and control RNAs used in this study listed above), suggesting that LEAPER could be tailored to therapeutics that require continuous administration. For native gene editing, we delivered *PPIB*-targeting arRNA$_{151}$ through lentiviral transduction in HEK293T cells and observed over 6% of target editing at 6 dpi (Fig. 28B).

We next tested synthesized arRNA oligonucleotides and electroporation delivery method for LEAPER. The 111-nt arRNA targeting *PPIB* transcripts as well as Ctrl RNA were chemically synthesized with 2'-O-methyl and phosphorothioate linkage modifications at the first three and last three residues of arRNAs (Fig. 28C). After introduced into T cells through electroporation, arRNA$_{111}$-PPIB oligos achieved ~20% of editing on *PPIB* transcripts (Fig. 28D), indicating that LEAPER holds promise for the development of oligonucleotide drugs.

## Example 16. Restoration of α-L-iduronidase activity in Hurler syndrome patient-derived primary fibroblast by LEAPER

Finally, we examined the potential of LEAPER in treating a monogenic disease - Hurler syndrome, the most severe subtype of Mucopolysaccharidosis type I (MPS I) due to the deficiency of α-L-iduronidase (IDUA), a lysosomal metabolic enzyme responsible for the degradation of mucopolysaccharides[50]. We chose a primary fibroblast GM06214 that was originally isolated from Hurler syndrome patient. The GM06214 cells contain a homozygous TGG>TAG mutation in exon 9 of the *IDUA* gene, resulting in a Trp402Ter mutation in the protein. We designed two versions of arRNAs by synthesized RNA oligonucleotides with chemical modifications, arRNA$_{111}$-IDUA-V1 and arRNA$_{111}$-IDUA-V2, targeting the mature mRNA and the pre-mRNA of *IDUA*, respectively (Fig. 29A and Sequences of arRNAs and control RNAs used in this study listed above). After introduction of arRNA$_{111}$-IDUA-V1 or arRNA$_{111}$-IDUA-V2 into GM06214 cells via electroporation, we measured the targeted RNA editing rates via NGS analysis and the catalytic activity of $\alpha$-L-iduronidase with 4-MU-$\alpha$-L-iduronidase substrate at different time points. Both arRNA$_{111}$-IDUA-V1 and arRNA$_{111}$-IDUA-V2 significantly restored the IDUA catalytic activity in *IDUA*-deficient GM06214 cells progressively with time after electroporation, and arRNA$_{111}$-IDUA-V2 performed much better than arRNA$_{111}$-IDUA-V1, while no $\alpha$-L-iduronidase activity could be detected in three control groups (Fig. 29B).

To further evaluate the extent to which the restored IDUA activity in GM06214 by LEAPER relieves the Hurler syndrome, we examined the IDUA activity in GM01323 cells, another primary fibroblasts from patient with Scheie syndrome, a much milder subtype of MPS I than Hurler syndrome due to the remnant IDUA activity resulting from heterozygous genotype on *IDUA* gene. We found that the catalytic activity of IDUA in GM06214 cells harbouring arRNA$_{111}$-IDUA-V2 was higher than GM01323 cells 48 hr post electroporation (Fig. 29B). Consistent with these results, NGS analysis indicated that arRNA$_{111}$-IDUA-V2 converted nearly 30% of A to I editing, a much higher rate than arRNA$_{111}$-IDUA-V1 (Fig. 29C). Further analysis revealed that minimal unwanted edits were detected within the arRNA covered regions of *IDUA* transcripts (Fig. 29D). Importantly, LEAPER did not trigger immune responses in primary cells as we demonstrated that, unlike the RNA duplex poly(I:C) serving as a positive control, neither arRNA$_{111}$-IDUA-V1 nor arRNA$_{111}$-IDUA-V2 induced expressions of a panel of genes involved in type-I interferon and pro-inflammatory responses (Fig. 29E). These results showed the therapeutic potential of LEAPER in targeting certain monogenetic diseases.

## Example 17. Restoration of $\alpha$-L-iduronidase activity in Hurler syndrome mice by LEAPER

As LEAPER providing the possibility to conduct experiments on a mouse, we examined the potential of LEAPER for treating a monogenic disease - Hurler syndrome *in vivo*. Adeno-associated Virus (AAV) is selected as the delivery system. The two sequences (Seq ID NO. 341 and Seq ID NO. 342) were inserted into the plasmid AAV-U6-CMV-GFP vector, and the plasmid was packaged into AAV8 virus by PackGene Biotech (see FIG. 31). The virus titer was as follows:

| name | titer | volume | Total amount | arRNA sequence 5'-3 |
|---|---|---|---|---|
| AAV8-ID UA-adRN A151-KD2 | 9.61E+1 2GC/ml | 590ul | 5.67E+12GC | Seq ID NO. 341: gggtgatgggtgctggccaggacacccactgtatg attgctgtccaacacagccccagcctttgagacctct gcccagagttgttctccatccaacagggccatgag ccccatgactgtgagtactggctttcgcagcaactg cacatggg |
| AAV8-Ran dom151-K D2 | 9.91E+12GC/ml | 600ul | 5.95E+12GC | Seq ID NO. 342: actacagttgctccgatatttaggctacgtcaataggcact aacttattggcgctggtgaacggacttcctctcgagtacc agaagatgactacaaaactcctttccattgcgagtatcgg agtctggctcagtttggccagggaggcact |

Six MPSI (idua W392X mice, B6.129S-Idua$^{tm1.1Kmke/J}$) male mice (4-10 weeks old) were selected and divided into two groups, and each of three mice of the first group was injected with 1E12 GC of AAV8 virus packed with plasmid AAV8-IDUA-adRNA151-KD2 through the tail vein, each of other three mice of the second group were injected with 1E12 GC of AAV8 virus packed with plasmid AAV8-Random151-KD2 through the tail vein as well as a control. Twenty-eight days after the injection, the mice were sacrificed by cervical dislocation and livers were taken.. We measured the enzymatic activity of $\alpha$-L-iduronidase in mouse hepatic cells with 4-MU-$\alpha$-L-iduronidase substrate for each mouse in two groups. The liver is divided into small pieces, one part after grinding is used for enzyme activity measurement (immediately). The average relative enzymatic activity of the first group is higher than that of the second group (See FIG. 32A). The relative enzymatic activity of each mouse of the first group is higher than that of the second group (See FIG. 32B, GM01323 as control and it has 0.3% $\alpha$-L-iduronidase enzyme activity compared with Wildtype cells). It can be seen from the result of enzymatic activity that the α-L-iduronidase activity of the first group of mice is restored. To confirm that TGG> TAG mutation is restored and the LEAPER provides effective RNA editing, we then measured the targeted RNA editing rates in mouse hepatic cells via next-generation sequencing analysis for each mouse in two groups by using other part of grinding liver. The other part is added with trizol for NGS. The average RNA editing efficiency of the first group is higher than that of the second group (See FIG. 33A). The RNA editing efficiency of each mouse of the first group is almost higher than that of the second group (See FIG. 33B). It can be seen from the result of RNA editing efficiency that the LEAPER method provides effective RNA editing up to 7%.

**ADAR1(p110) cDNA**

5'-ATGGCCGAGATCAAGGAGAAAATCTGCGACTATCTCTTCAATGTGTCTGACTCCTCTGCCCTGAATT
TGGCTAAAAATATTGGCCTTACCAAGGCCCGAGATATAAATGCTGTGCTAATTGACATGGAAAGGCAGG
GGGATGTCTATAGACAAGGGACAACCCCTCCCATATGGCATTTGACAGACAAGAAGCGAGAGAGGAT
GCAAATCAAGAGAAATACGAACAGTGTTCCTGAAACCGCTCCAGCTGCAATCCCTGAGACCAAAAGA
AACGCAGAGTTCCTCACCTGTAATATACCCACATCAAATGCCTCAAATAACATGGTAACCACAGAAAA
AGTGGAGAATGGGCAGGAACCTGTCATAAAGTTAGAAAACAGGCAAGAGGCCAGACCAGAACCAGC
AAGACTGAAACCACCTGTTCATTACAATGGCCCCTCAAAAGCAGGGTATGTTGACTTTGAAAATGGCC
AGTGGGCCACAGATGACATCCCAGATGACTTGAATAGTATCCGCGCAGCACCAGGTGAGTTTCGAGCC
ATCATGGAGATGCCCTCCTTCTACAGTCATGGCTTGCCACGGTGTTCACCCTACAAGAAACTGACAGA
GTGCCAGCTGAAGAACCCCATCAGCGGGCTGTTAGAATATGCCCAGTTCGCTAGTCAAACCTGTGAGT
TCAACATGATAGAGCAGAGTGGACCACCCCATGAACCTCGATTTAAATTCCAGGTTGTCATCAATGGCC
GAGAGTTTCCCCCAGCTGAAGCTGGAAGCAAGAAAGTGGCCAAGCAGGATGCAGCTATGAAAGCCAT
GACAATTCTGCTAGAGGAAGCCAAAGCCAAGGACAGTGGAAAATCAGAAGAATCATCCCACTATTCC
ACAGAGAAAGAATCAGAGAAGACTGCAGAGTCCCAGACCCCCACCCCTTCAGCCACATCCTTCTTTT
CTGGGAAGAGCCCCGTCACCACACTGCTTGAGTGTATGCACAAATTGGGGAACTCCTGCGAATTCCGT
CTCCTGTCCAAAGAAGGCCCTGCCCATGAACCCAAGTTCCAATACTGTGTTGCAGTGGGAGCCCAAAC
TTTCCCCAGTGTGAGTGCTCCCAGCAAGAAAGTGGCAAAGCAGATGGCCGCAGAGGAAGCCATGAAG
GCCCTGCATGGGGAGGCGACCAACTCCATGGCTTCTGATAACCAGCCTGAAGGTATGATCTCAGAGTC

ACTTGATAACTTGGAATCCATGATGCCCAACAAGGTCAGGAAGATTGGCGAGCTCGTGAGATACCTGA
ACACCAACCCTGTGGGTGGCCTTTTGGAGTACGCCCGCTCCCATGGCTTTGCTGCTGAATTCAAGTTG
GTCGACCAGTCCGGACCTCCTCACGAGCCCAAGTTCGTTTACCAAGCAAAAGTTGGGGGTCGCTGGT
TCCCAGCCGTCTGCGCACACAGCAAGAAGCAAGGCAAGCAGGAAGCAGCAGATGCGGCTCTCCGTG
TCTTGATTGGGGAGAACGAGAAGGCAGAACGCATGGGTTTCACAGAGGTAACCCCAGTGACAGGGGC
CAGTCTCAGAAGAACTATGCTCCTCCTCTCAAGGTCCCCAGAAGCACAGCCAAAGACACTCCCTCTCA
CTGGCAGCACCTTCCATGACCAGATAGCCATGCTGAGCCACCGGTGCTTCAACACTCTGACTAACAGC
TTCCAGCCCTCCTTGCTCGGCCGCAAGATTCTGGCCGCCATCATTATGAAAAAAGACTCTGAGGACAT
GGGTGTCGTCGTCAGCTTGGGAACAGGGAATCGCTGTGTAAAAGGAGATTCTCTCAGCCTAAAAGGA
GAAACTGTCAATGACTGCCATGCAGAAATAATCTCCCGGAGAGGCTTCATCAGGTTTCTCTACAGTGA
GTTAATGAAATACAACTCCCAGACTGCGAAGGATAGTATATTTGAACCTGCTAAGGGAGGAGAAAAGC
TCCAAATAAAAAAGACTGTGTCATTCCATCTGTATATCAGCACTGCTCCGTGTGGAGATGGCGCCCTCT
TTGACAAGTCCTGCAGCGACCGTGCTATGGAAAGCACAGAATCCCGCCACTACCCTGTCTTCGAGAAT
CCCAAACAAGGAAAGCTCCGCACCAAGGTGGAGAACGGAGAAGGCACAATCCCTGTGGAATCCAGT
GACATTGTGCCTACGTGGGATGGCATTCGGCTCGGGGAGAGACTCCGTACCATGTCCTGTAGTGACAA
AATCCTACGCTGGAACGTGCTGGGCCTGCAAGGGGCACTGTTGACCCACTTCCTGCAGCCCATTTATC
TCAAATCTGTCACATTGGGTTACCTTTTCAGCCAAGGGCATCTGACCCGTGCTATTTGCTGTCGTGTGA
CAAGAGATGGGAGTGCATTTGAGGATGGACTACGACATCCCTTTATTGTCAACCACCCCAAGGTTGGC
AGAGTCAGCATATATGATTCCAAAAGGCAATCCGGGAAGACTAAGGAGACAAGCGTCAACTGGTGTCT
GGCTGATGGCTATGACCTGGAGATCCTGGACGGTACCAGAGGCACTGTGGATGGGCCACGGAATGAAT
TGTCCCGGGTCTCCAAAAAGAACATTTTTCTTCTATTTAAGAAGCTCTGCTCCTTCCGTTACCGCAGGG
ATCTACTGAGACTCTCCTATGGTGAGGCCAAGAAAGCTGCCCGTGACTACGAGACGGCCAAGAACTA
CTTCAAAAAAGGCCTGAAGGATATGGGCTATGGGAACTGGATTAGCAAACCCCAGGAGGAAAAGAAC
TTTTATCTCTGCCCAGTA <u>GATTACAAGGATGACGACGATAAG(Flag tag)</u> TAG-3' (SEQ ID NO:332)

**ADAR1(p150) cDNA**

5'ATGAATCCGCGGCAGGGGTATTCCCTCAGCGGATACTACACCCATCCATTTCAAGGCTATGAGCACA
GACAGCTCAGATACCAGCAGCCTGGGCCAGGATCTTCCCCCAGTAGTTTCCTGCTTAAGCAAATAGAA
TTTCTCAAGGGGCAGCTCCCAGAAGCACCGGTGATTGGAAAGCAGACACCGTCACTGCCACCTTCCC
TCCCAGGACTCCGGCCAAGGTTTCCAGTACTACTTGCCTCCAGTACCAGAGGCAGGCAAGTGGACATC
AGGGGTGTCCCCAGGGGCGTGCATCTCGGAAGTCAGGGGCTCCAGAGAGGGTTCCAGCATCCTTCAC
CACGTGGCAGGAGTCTGCCACAGAGAGGTGTTGATTGCCTTTCCTCACATTTCCAGGAACTGAGTATC
TACCAAGATCAGGAACAAAGGATCTTAAAGTTCCTGGAAGAGCTTGGGGAAGGGAAGGCCACCACAG
CACATGATCTGTCTGGGAAACTTGGGACTCCGAAGAAAGAAATCAATCGAGTTTTATACTCCCTGGCA
AAGAAGGGCAAGCTACAGAAAGAGGCAGGAACACCCCCTTTGTGGAAAATCGCGGTCTCCACTCAG
GCTTGGAACCAGCACAGCGGAGTGGTAAGACCAGACGGTCATAGCCAAGGAGCCCCAAACTCAGAC
CCGAGTTTGGAACCGGAAGACAGAAACTCCACATCTGTCTCAGAAGATCTTCTTGAGCCTTTTATTGC
AGTCTCAGCTCAGGCTTGGAACCAGCACAGCGGAGTGGTAAGACCAGACAGTCATAGCCAAGGATCC
CCAAACTCAGACCCAGGTTTGGAACCTGAAGACAGCAACTCCACATCTGCCTTGGAAGATCCTCTTGA
GTTTTTAGACATGGCCGAGATCAAGGAGAAAATCTGCGACTATCTCTTCAATGTGTCTGACTCCTCTGC
CCTGAATTTGGCTAAAAATATTGGCCTTACCAAGGCCCGAGATATAAATGCTGTGCTAATTGACATGGA
AAGGCAGGGGGATGTCTATAGACAAGGGACAACCCCTCCCATATGGCATTTGACAGACAAGAAGCGA
GAGAGGATGCAAATCAAGAGAAATACGAACAGTGTTCCTGAAACCGCTCCAGCTGCAATCCCTGAGA
CCAAAAGAAACGCAGAGTTCCTCACCTGTAATATACCCACATCAAATGCCTCAAATAACATGGTAACC
ACAGAAAAAGTGGAGAATGGGCAGGAACCTGTCATAAAGTTAGAAAACAGGCAAGAGGCCAGACCA
GAACCAGCAAGACTGAAACCACCTGTTCATTACAATGGCCCCTCAAAAGCAGGGTATGTTGACTTTGA

AAATGGCCAGTGGGCCACAGATGACATCCCAGATGACTTGAATAGTATCCGCGCAGCACCAGGTGAGT
TTCGAGCCATCATGGAGATGCCCTCCTTCTACAGTCATGGCTTGCCACGGTGTTCACCCTACAAGAAAC
TGACAGAGTGCCAGCTGAAGAACCCCATCAGCGGGCTGTTAGAATATGCCCAGTTCGCTAGTCAAACC
TGTGAGTTCAACATGATAGAGCAGAGTGGACCACCCCATGAACCTCGATTTAAATTCCAGGTTGTCATC
AATGGCCGAGAGTTTCCCCCAGCTGAAGCTGGAAGCAAGAAAGTGGCCAAGCAGGATGCAGCTATGA
AAGCCATGACAATTCTGCTAGAGGAAGCCAAAGCCAAGGACAGTGGAAAATCAGAAGAATCATCCCA
CTATTCCACAGAGAAAGAATCAGAGAAGACTGCAGAGTCCCAGACCCCCACCCCTTCAGCCACATCC
TTCTTTTCTGGGAAGAGCCCCGTCACCACACTGCTTGAGTGTATGCACAAATTGGGGAACTCCTGCGA
ATTCCGTCTCCTGTCCAAAGAAGGCCCTGCCCATGAACCCAAGTTCCAATACTGTGTTGCAGTGGGAG
CCCAAACTTTCCCCAGTGTGAGTGCTCCCAGCAAGAAAGTGGCAAAGCAGATGGCCGCAGAGGAAG
CCATGAAGGCCCTGCATGGGGAGGCGACCAACTCCATGGCTTCTGATAACCAGCCTGAAGGTATGATC
TCAGAGTCACTTGATAACTTGGAATCCATGATGCCCAACAAGGTCAGGAAGATTGGCGAGCTCGTGAG
ATACCTGAACACCAACCCTGTGGGTGGCCTTTTGGAGTACGCCCGCTCCCATGGCTTTGCTGCTGAATT
CAAGTTGGTCGACCAGTCCGGACCTCCTCACGAGCCCAAGTTCGTTTACCAAGCAAAAGTTGGGGGT
CGCTGGTTCCCAGCCGTCTGCGCACACAGCAAGAAGCAAGGCAAGCAGGAAGCAGCAGATGCGGCT
CTCCGTGTCTTGATTGGGGAGAACGAGAAGGCAGAACGCATGGGTTTCACAGAGGTAACCCCAGTGA
CAGGGGCCAGTCTCAGAAGAACTATGCTCCTCCTCTCAAGGTCCCCAGAAGCACAGCCAAAGACACT
CCCTCTCACTGGCAGCACCTTCCATGACCAGATAGCCATGCTGAGCCACCGGTGCTTCAACACTCTGA
CTAACAGCTTCCAGCCCTCCTTGCTCGGCCGCAAGATTCTGGCCGCCATCATTATGAAAAAAGACTCTG
AGGACATGGGTGTCGTCGTCAGCTTGGGAACAGGGAATCGCTGTGTAAAAGGAGATTCTCTCAGCCTA
AAAGGAGAAACTGTCAATGACTGCCATGCAGAAATAATCTCCCGGAGAGGCTTCATCAGGTTTCTCTA
CAGTGAGTTAATGAAATACAACTCCCAGACTGCGAAGGATAGTATATTTGAACCTGCTAAGGGAGGAG
AAAAGCTCCAAATAAAAAAGACTGTGTCATTCCATCTGTATATCAGCACTGCTCCGTGTGGAGATGGC
GCCCTCTTTGACAAGTCCTGCAGCGACCGTGCTATGGAAAGCACAGAATCCCGCCACTACCCTGTCTT
CGAGAATCCCAAACAAGGAAAGCTCCGCACCAAGGTGGAGAACGGAGAAGGCACAATCCCTGTGGA
ATCCAGTGACATTGTGCCTACGTGGGATGGCATTCGGCTCGGGGAGAGACTCCGTACCATGTCCTGTA
GTGACAAAATCCTACGCTGGAACGTGCTGGGCCTGCAAGGGGCACTGTTGACCCACTTCCTGCAGCC
CATTTATCTCAAATCTGTCACATTGGGTTACCTTTTCAGCCAAGGGCATCTGACCCGTGCTATTTGCTGT
CGTGTGACAAGAGATGGGAGTGCATTTGAGGATGGACTACGACATCCCTTTATTGTCAACCACCCCAA
GGTTGGCAGAGTCAGCATATATGATTCCAAAAGGCAATCCGGGAAGACTAAGGAGACAAGCGTCAAC
TGGTGTCTGGCTGATGGCTATGACCTGGAGATCCTGGACGGTACCAGAGGCACTGTGGATGGGCCACG
GAATGAATTGTCCCGGGTCTCCAAAAAGAACATTTTTCTTCTATTTAAGAAGCTCTGCTCCTTCCGTTA
CCGCAGGGATCTACTGAGACTCTCCTATGGTGAGGCCAAGAAAGCTGCCCGTGACTACGAGACGGCC
AAGAACTACTTCAAAAAAGGCCTGAAGGATATGGGCTATGGGAACTGGATTAGCAAACCCCAGGAGG
AAAAGAACTTTTATCTCTGCCCAGTA <u>GATTACAAGGATGACGACGATAAG(Flag tag)</u> TAG-3' (SEQ ID NO:333)

**ADAR2 cDNA**

5'-ATGGATATAGAAGATGAAGAAAACATGAGTTCCAGCAGCACTGATGTGAAGGAAAACCGCAATCTG
GACAACGTGTCCCCCAAGGATGGCAGCACACCTGGGCCTGGCGAGGGCTCTCAGCTCTCCAATGGGG
GTGGTGGTGGCCCCGGCAGAAAGCGGCCCCTGGAGGAGGGCAGCAATGGCCACTCCAAGTACCGCCT
GAAGAAAAGGAGGAAAACACCAGGGCCCGTCCTCCCCAAGAACGCCCTGATGCAGCTGAATGAGAT
CAAGCCTGGTTTGCAGTACACACTCCTGTCCCAGACTGGGCCCGTGCACGCGCCTTTGTTTGTCATGT
CTGTGGAGGTGAATGGCCAGGTTTTTGAGGGCTCTGGTCCCACAAAGAAAAAGGCAAAACTCCATGC
TGCTGAGAAGGCCTTGAGGTCTTTCGTTCAGTTTCCTAATGCCTCTGAGGCCCACCTGGCCATGGGGA

GGACCCTGTCTGTCAACACGGACTTCACATCTGACCAGGCCGACTTCCCTGACACGCTCTTCAATGGT
TTTGAAACTCCTGACAAGGCGGAGCCTCCCTTTTACGTGGGCTCCAATGGGGATGACTCCTTCAGTTC
CAGCGGGGACCTCAGCTTGTCTGCTTCCCCGGTGCCTGCCAGCCTAGCCCAGCCTCCTCTCCCTGCCTT
ACCACCATTCCCACCCCCGAGTGGGAAGAATCCCGTGATGATCTTGAACGAACTGCGCCCAGGACTCA
AGTATGACTTCCTCTCCGAGAGCGGGGAGAGCCATGCCAAGAGCTTCGTCATGTCTGTGGTCGTGGAT
GGTCAGTTCTTTGAAGGCTCGGGGAGAAACAAGAAGCTTGCCAAGGCCCGGGCTGCGCAGTCTGCCC
TGGCCGCCATTTTTAACTTGCACTTGGATCAGACGCCATCTCGCCAGCCTATTCCCAGTGAGGGTCTTC
AGCTGCATTTACCGCAGGTTTTAGCTGACGCTGTCTCACGCCTGGTCCTGGGTAAGTTTGGTGACCTG
ACCGACAACTTCTCCTCCCTCACGCTCGCAGAAAAGTGCTGGCTGGAGTCGTCATGACAACAGGCA
CAGATGTTAAAGATGCCAAGGTGATAAGTGTTTCTACAGGAACAAAATGTATTAATGGTGAATACATGA
GTGATCGTGGCCTTGCATTAAATGACTGCCATGCAGAAATAATATCTCGGAGATCCTTGCTCAGATTTCT
TTATACACAACTTGAGCTTTACTTAAATAACAAAGATGATCAAAAAAGATCCATCTTTCAGAAATCAGA
GCGAGGGGGGTTTAGGCTGAAGGAGAATGTCCAGTTTCATCTGTACATCAGCACCTCTCCCTGTGGAG
ATGCCAGAATCTTCTCACCACATGAGCCAATCCTGGAAGAACCAGCAGATAGACACCCAAATCGTAAA
GCAAGAGGACAGCTACGGACCAAAATAGAGTCTGGTGAGGGGACGATTCCAGTGCGCTCCAATGCGA
GCATCCAAACGTGGGACGGGGTGCTGCAAGGGGAGCGGCTGCTCACCATGTCCTGCAGTGACAAGAT
TGCACGCTGGAACGTGGTGGGCATCCAGGGATCCCTGCTCAGCATTTTCGTGGAGCCCATTTACTTCTC
GAGCATCATCCTGGGCAGCCTTTACCACGGGGACCACCTTTCCAGGGCCATGTACCAGCGGATCTCCA
ACATAGAGGACCTGCCACCTCTCTACACCCTCAACAAGCCTTTGCTCAGTGGCATCAGCAATGCAGAA
GCACGGCAGCCAGGGAAGGCCCCCAACTTCAGTGTCAACTGGACGGTAGGCGACTCCGCTATTGAGG
TCATCAACGCCACGACTGGGAAGGATGAGCTGGGCCGCGCGTCCCGCCTGTGTAAGCACGCGTTGTA
CTGTCGCTGGATGCGTGTGCACGGCAAGGTTCCCTCCCACTTACTACGCTCCAAGATTACCAAACCCA
ACGTGTACCATGAGTCCAAGCTGGCGGCAAAGGAGTACCAGGCCGCCAAGGCGCGTCTGTTCACAGC
CTTCATCAAGGCGGGGCTGGGGGCCTGGGTGGAGAAGCCCACCGAGCAGGACCAGTTCTCACTCACG
CCC <u>GATTACAAGGATGACGACGATAAG(Flag tag)</u> TAG-3' (SEQ ID NO:334)

**Coding sequence (CDS) of the disease-relevant genes**

*COL3A1*

5'-atgatgagctttgtgcaaaaggggagctggctacttctcgctctgcttcatcccactattattttggcacaacaggaagctgttgaaggaggatgttcccatcttggtcagtcctatgcggatagagatgtctggaagccagaaccatgccaaatatgtgtctgtgactcaggatccgttctctgcgatgacataatatgtgacgatcaagaattagactgccccaacccagaaattccatttggagaatgttgtgcagtttgcccacagcctccaactgctcctactcgccctcctaatggtcaaggacctcaaggcccccaagggagatccaggccctcctggtattcctgggagaaatggtgaccctggtattccaggacaaccagggtccctggttctcctggcccccctggaatctgtgaatcatgccctactggtcctcagaactattctccccagtatgattcatatgatgtcaagtctggagtagcagtaggaggactcgcaggctatcctggaccagctggccccccaggccctcccggtcccctggtacatctggtcatcctggttccctggatctccaggataccaaggacccctggtgaacctgggcaagctggtccttcaggccctccaggacctcctggtgctataggtccatctggtcctgctggaaaagatggagaatcaggtagacccggacgacctggagagcgaggattgcctggacctccaggtatcaaaggtccagctgggatacctggattccctggtatgaaaggacacagaggcttcgatggacgaaatggagaaaagggtgaaacaggtgctcctggattaaaggtgaaatggtcttccaggcgaaaatggagctcctggacccatgggtccaagaggggctcctggtgagcgaggacggccaggacttcctggggctgcaggtgctcggggtaatgacggtgctcgaggcagtgatggtcaaccaggccctcctggtcctcctggaactgccggattccctggatcccctggtgctaagggtgaagttggacctgcagggtctcctggttcaaatggtgccctggacaaagaggagaacctggacctcaggacacgctggtgctcaaggtcctcctggccctcctgggattaatggtagtcctggtggtaaaggcgaaatgggtcccgctggcattcctggagctcctggactgatgggagcccggggtcctccaggaccagccggtgctaatggtgctcctggactgcgaggtggtgcaggtgagcctggtaagaatggtgccaaaggagagcccggaccacgtggtgaacgcggtgaggctggtattccaggtgttccaggagctaaaggcgaagatggcaaggatggatcacctggagaacctggtgcaaatgggcttccaggagctgcaggagaaaaggggtgccctgggttccgaggacctgctggaccaaatggcatcccaggagaaaagggtcctgctggagagcgtggtgctccaggccctgcagggcccagaggagctgctggagaacctggcagagatggcgtccctggaggtccaggaatgaggggcatgccccggaagtccaggaggaccaggaagtgatgggaaaccagggcctcccggaagtcaaggagaaagtggtcgaccaggtcctcctgggccatctggtccccgaggtcagcctggtgtcatgggcttccccggtcctaaaggaaatgatggtgctcctggtaagaatggtgagaacgaggtggccctggaggacctggccctcagggtcctcct

ggaaagaatggtgaaactggacctcagggacccccagggcctactgggcctggtggtgacaaaggagacacaggacccccctggtccacaaggattacaaggcttgcctggtacaggtggtcctccaggagaaaatggaaaaacctggggaaccaggtccaaagggtgatgccggtgcacctggagctccaggaggcaagggtgatgctggtgcccctggtgaacgtggacctcctggattggcaggggccccaggacttagaggtggagctggtcccctggtcccgaaggaggaaaggggtgctgctggtcctcctggccacctggtgctgctggtactcctggtctgcaaggaatgcctggagaaagaggaggtcttggaagtcctggtccaaagggtgacaagggtgaaccaggcggtccaggtgctgatggtgtcccaggggaaagatggcccaagggggtcctactggtcctattggtcctcctggcccagctggccagcctggagataaaggtgaaggtggtgccccggacttccaggtatagctggacctcgtggtagccctggtgagagaggtgaaactggccctccaggacctgctggtttccctggtgctcctggacagaatggtgaacctggtggtaaaggagaaagaggggctccgggtgagaaagtgaaggaggccctcctggagttgcaggacccccggaggttctggacctgctggtcctcctggtccccaaggtgtcaaaggtgaacgtggcagtcctggtggacctggtgctgctggcttccctggtgctcgtggtcttcctggtcctcctggtagtaatggtaacccaggaccccccaggtcccagccggttctccaggcaaggatgggcccccaggtcctgcgggtaacactggtgctcctggcagccctggagtgtctggaccaaaaggtgatgctggccaaccaggagagaagggatcgcctggtgcccagggcgccaccaggagctccaggcccacttgggattgctgggatcactggagcacggggtcttgcaggaccaccaggcatgccaggtcctaggggaagccctggccctcagggtgtcaagggtgaaagtgggaaaccaggagctaacggtctcagtggagaacgtggtcccctggacccaagggtcttcctggtctggctggtacagctggtgaacctggaagagatgtgaaacctggatcagatggtcttccaggccgagatggatctcctggtggcaagggtgatcgtggtgaaaatggctctcctggtgcccctggcgctcctggtcatccaggcccacctggtcctgtcggtccagctggaaagagtggtgacagaggagaaagtggccctgctggccctgctggtgctcccggtcctgctggttcccgaggtgctcctggtcctcaaggcccacgtggtgacaaaggtgaaacaggtgaacgtggagctgctggcatcaaaggacatcgaggattccctggtaatccaggtgccccaggttctccaggccctgctggtcagcagggtgcaatcggcagtccaggacctgcaggccccagaggacctgttggacccagtggacctcctggcaaagatggaaccagtggacatccaggtcccattggaccaccagggcctcgaggtaacagaggtgaaagaggatctgaggctccccaggccacccagggcaaccaggccctcctggacctcctggtgcccctggtccttgctgtggtggtgttggagccgctgccattgctgggattggaggtgaaaagctggcggttttgccccgtattatggagatgaaccaatggatttcaaaatcaacaccgatgagattatgacttcactcaagtctgttaatggacaaatagaaagcctcat

tagtcctgatggttctcgtaaaaaccccgctagaaactgcagagacctgaaattctgccatcctgaactcaagagtggagaatact**g**ggttgaccctaaccaaggatgcaaattggatgctatcaaggtattctgtaatatggaaactggggaaacatgcataagtgccaatcctttgaatgttccacggaaacactggtggacagattctagtgctgagaagaaacacgtttggtttggagagtccatggatggtggttttcagtttagctacggcaatcctgaacttcctgaagatgtccttgatgtgcagctggcattccttcgacttctctccagccgagcttcccagaacatcacatatcactgcaaaaatagcattgcatacatggatcaggccagtggaaatgtaaagaaggccctgaagctgatggggtcaaatgaaggtgaattcaaggctgaaggaaatagcaaattcacctacacagttctggaggatggtggttgcacgaaacacactgggaatggagcaaaacagtctttgaatatcgaacacgcaaggctgtgagactacctattgtagatattgcaccctatgacattggtggtcctgatcaagaatttggtgtggacgttggccctgtttgctttttataa-3' (SEQ ID NO:335)

*BMPR2*

5'-atgacttcctcgctgcagcggccctggcggggtgccctggctaccatggaccatcctgctggtcagcgctgcggctgcttcgcagaatcaagaacggctatgtgcg
tttaaagatccgtatcagcaagaccttgggataggtgagagtagaatctctcatgaaaatgggacaatattatgctcgaaaggtagcacctgctatggcctttgggagaa
atcaaaaggggacataaatcttgtaaaacaaggatgttggtctcacattggagatccccaagagtgtcactatgaagaatgtgtagtaactaccactcctccctcaattca
gaatggaacataccgtttctgctgttgtagcacagatttatgtaatgtcaactttactgagaattttccacctcctgacacaacaccactcagtccacctcattcatttaaccga
gatgagacaataatcattgctttggcatcagtctctgtattagctgtgtttgatagttgccttatgctttggatacagaatgttgacaggagaccgtaaacaaggtcttcacagt
atgaacatgatggaggcagcagcatccgaaccctctcttgatctagataatctgaaactgttggagctgattggccgaggtcgatatggagcagtatataaaggctcctt
ggatgagcgtccagttgctgtaaaagtgtttccttttgcaaaccgtcagaattttatcaacgaaaagaacatttacagagtgcctttgatggaacatgacaacattgcccgc
tttatagttggagatgagagagtcactgcagatggacgcatggaatatttgcttgtgatggagtactatcccaatggatctttatgcaagtatttaagtctccacacaagtga
ct**g**ggtaagctcttgccgtcttgctcattctgttactagaggactggcttatcttcacacagaattaccacgaggagatcattataaacctgcaatttcccatcgagatttaaa
cagcagaaatgtcctagtgaaaaatgatggaacctgtgttattagtgactttggactgtccatgaggctgactggaaatagactggtgcgcccaggggaggaagataat
gcagccataagcgaggttggcactatcagatatatggcaccagaagtgctagaaggagctgtgaacttgagggactgtgaatcagctttgaaacaagtagacatgtat
gctcttggactaatctattgggagatatttatgagatgtacagacctcttcccaggggaatccgtaccagagtaccagatggcttttcagacagaggttggaaaccatccc
acttttgaggatatgcaggttctcgtgtctagggaaaaacagagacccaagttcccagaagcctggaaagaaaatagcctggcagtgaggtcactcaaggagacaatc
gaagactgttgggaccaggatgcagaggctcggcttactgcacagtgtgctgaggaaaaggatggctgaacttatgatgatttgggaaagaaacaaatctgtgagccca
acagtcaatccaatgtctactgctatgcagaatgaacgcaacctgtcacataataggcgtgtgccaaaaattggtccttatccagattattcttcctcctcatacattgaaga
ctctatccatcatactgacagcatcgtgaagaatatttcctctgagcattctatgtccagcacacctttgactataggggaaaaaaaccgaaattcaattaactatgaacgac
agcaagcacaagctcgaatccccagccctgaaacaagtgtcaccagcctctccaccaacacaacaaccacaaacaccacaggactcacgccaagtactggcatgac
tactatatctgagatgccatacccagatgaaacaaatctgcataccacaaatgttgcacagtcaattgggccaacccctgtctgcttacagctgacagaagaagacttgg

aaaccaacaagctagaccccaaaagaagttgataagaacctcaaggaaagctctgatgagaatctcatggagcactctctaaacagttcagtggcccagacccactga
gcagtactagttctagcttgctttacccactcataaaaacttgcagtagaagcaactggacagcaggacttcacacagactgcaaatggccaagcatgtttgattcctgatg
ttctgcctactcagatctatcctctccccaagcagcagaaaccttcccaagagacctactagtttgcctttgaacaccaaaaattcaacaaaagagccccggctaaaatttg
gcagcaagcacaaatcaaacttgaaacaagtcgaaactggagttgccaagatgaatacaatcaatgcagcagaacctcatgtggtgacagtcaccatgaatggtgtgg
caggtagaaaccacagtgttaactcccatgctgccacaacccaatatgccaatgggacagtactatctggccaaacaaccaacatagtgacacataggcccaagaa
atgttgcagaatcagtttattggtgaggacacccggctgaatattaattccagtcctgatgagcatgagcctttactgagacgagagcaacaagctggccatgatgaagg
tgttctggatcgtcttgtggacaggagggaacggccactagaaggtggccgaactaattccaataacaacaacagcaatccatgttcagaacaagatgttcttgcacag
ggtgttccaagcacagcagcagatcctgggccatcaaagcccagaagagcacagaggcctaattctctggatctttcagccacaaatgtcctggatggcagcagtata
cagataggtgagtcaacacaagatggcaaatcaggatcaggtgaaaagatcaagaaacgtgtgaaaactccctattctcttaagcggtggcgcccctccacctgggtc
atctccactgaatcgctggactgtgaagtcaacaataatggcagtaacagggcagttcattccaaatccagcactgctgtttaccttgcagaaggaggcactgctacaac
catggtgtctaaagatataggaatgaactgtctgtga-3' (SEQ ID NO:336)

*AHI1*

5'-atgcctacagctgagagtgaagcaaaagtaaaaaccaaagttcgctttgaagaattgcttaagacccacagtgatctaatgcgtgaaaagaaaaaactgaagaaaa aacttgtcaggtctgaagaaaacatctcacctgacactattagaagcaatcttcactatatgaaagaaactacaagtgatgatcccgacactattagaagcaatcttcccca tattaaagaaactacaagtgatgatgtaagtgctgctaacactaacaacctgaagaagagcacgagagtcactaaaaacaaattgaggaacacacagttagcaactga aaatcctaatggtgatgctagtgtagaggaagacaaacaaggaaagccaaataaaaaggtgataaagacggtgccccagttgactacacaagacctgaaaccggaa actcctgagaataaggttgattctacacaccagaaaacacatacaaagccacagccaggcgttgatcatcagaaaagtgagaaggcaaatgagggaagagaagaga ctgatttagaagaggatgaagaattgatgcaagcatatcagtgccatgtaactgaagaaatggcaaaggagattaagaggaaaataagaaagaaactgaaagaacag ttgacttactttccctcagatactttattccatgatgacaaactaagcagtgaaaaaaggaaaaagaaaaaggaagttccagtcttctctaaagctgaaacaagtacattga ccatctctggtgacacagttgaaggtgaacaaaagaaagaatcttcagttagatcagtttcttcagattctcatcaagatgatgaaataagctcaatggaacaaagcacag aagacagcatgcaagatgatacaaaacctaaaccaaaaaaaacaaaaaagaagactaaagcagttgcagataataatgaagatgttgatggtgatggtgttcatgaaat aacaagccgagatagcccggtttatcccaaatgtttgcttgatgatgaccttgtcttgggagtttacattcaccgaactgatagacttaagtcagattttatgatttctcaccca atggtaaaaattcatgtggttgatgagcatactggtcaatatgtcaagaaagatgatagtggacggcctgtttcatcttactatgaaaaagagaatgtggattatattcttcct attatgacccagccatatgatttaaacagttaaaatcaagacttccagagtgggaagaacaaattgtatttaatgaaaattttccctatttgcttcgaggctctgatgagagt cctaaagtcatcctgttctttgagattcttgatttcttaagcgtggatgaaattaagaataattctgaggttcaaaaccaagaatgtggctttcggaaaattgcctgggcatttc ttaagcttctgggagccaatggaaatgcaaacatcaactcaaaacttcgcttgcagctatattacccacctactaagcctcgatccccattaagtgttgttgaggcatttgaa tggtggtcaaaatgtccaagaaatcattacccatcaacactgtacgtaactgtaagaggactgaaagttccagactgtataaagccatcttaccgctctatgatggctcttc aggaggaaaaaaggtaaaccagtgcattgtgaacgtcaccatgagtcaagctcagtagacacagaacctggattagaagagtcaaaggaagtaataaagtggaaacg actccctgggcaggcttgccgtatcccaaacaaacacctcttctcactaaatgcaggagaacgaggatgttttgtcttgatttctcccacaatggaagaatattagcagca gcttgtgccagccgggatggagatatccaattattttatatgaaattccttctggacgtttcatgagagaattgtgtggccacctcaatatcatttatgatctttcctggtcaaaag atgatcactacatccttacttcatcatctgatggcactgccaggatatggaaaaatgaaataaacaatacaaatactttcagagttttacctcatccttctttgtttacacggct aaattccatccagctgtaagagagctagtagttacaggatgctatgattccatgatacggatat**g**gaaagttgagatgagagaagattctgccatattggtccgacagttt gacgttcacaaaagtttatcaactcactttgttttgatactgaaggtcatcatatgtattcaggagattgtacaggggtgattgttgtttggaatacctatgtcaagattaatga tttggaacattcagtgcaccactggactataaataaggaaattaaagaaactgagtttaagggaattccaataagttatttggagattcatcccaatggaaaacgtttgttaa tccataccaaagacagtactttgagaattatggatctccggatattagtagcaaggaagtttgtaggagcagcaaattatcgggagaagattcatagtactttgactccatg tgggactttttctgtttgctggaagtgaggatggtatagtgtatgtttggaacccagaaacaggagaacaagtagccatgtattctgacttgccattcaagtcacccattcga gacatttcttatcatccatttgaaaatatggttgcattctgtgcatttgggcaaaatgagccaattcttctgtatatttacgatttccatgttgcccagcaggaggctgaaatgtt caaacgctacaatggaacatttccattacctggaatacaccaaagtcaagatgccctatgtacctgtccaaaactaccccatcaaggctcttttcagattgatgaatttgtcc acactgaaagttcttcaacgaagatgcagctagtaaaacagaggcttgaaactgtcacagaggtgatacgttcctgtgctgcaaaagtcaacaaaaatctctcatttactt caccaccagcagtttcctcacaacagtctaagttaaaagcagtcaaacatgctgaccgctcaagagattctacatcagtttggtttcactcagaccgggattatcagcatag aaagaaagccttgtaaccatcaggtagatacagcaccaacggtagtggctctttatgactacacagcgaatcgatcagatgaactaaccatccatcgcgggagacattat ccgagtgtttttcaaagataatgaagactggtggtatggcagcataggaaagggacaggaaggttattttccagctaatcatgtggctagtgaaacactgtatcaagaact gcctcctgagataaaggagcgatcccctcctttaagccctgaggaaaaaactaaaatagaaaaatctccagctcctcaaaagcaatcaatcaataagaacaagtccca ggacttcagactaggctcagaatctatgacacattctgaaatgagaaaagaacagagccatgaggaccaaggacacataatggatacacggatgaggaagaacaag

caagcaggcagaaaagtcactctaatagagta-3' (SEQ ID NO:337)

*FANCC*

5'-atggctcaagattcagtagatctttcttgtgattatcagtttttggatgcagaagctttctgtatgggatcaggcttccactttggaaacccagcaagacacctgtcttcacgtggctcagttccaggagttcctaaggaagatgtatgaagccttgaaagagatggattctaatacagtcattgaaagattccccacaattggtcaactgttggcaaaagcttgttggaatcctttattttagcatatgatgaaagccaaaaaattctaatatggtgcttatgttgtctaattaacaaagaaccacagaattctggacaatcaaaacttaactcctggatacagggtgtattatctcatatactttcagcactcagatttgataaagaagttgctcttttcactcaaggtcttgggtatgcacctatagattactatcctggtttgcttaaaaatatggttttatcattagcgtctgaactcagagagaatcatcttaatgdatttaacactcaaaggcgaatggctcccgagcgagtggcgtccctgtcacgagtttgtgtcccacttattaccctgacagatgttgaccccctggtggaggctctcctcatctgtcatggacgtgaacctcaggaaatcctccagccagagttctttgaggctgtaaacgaggccattttgctgaagaagatttctctccccatgtcagctgtagtctgcctctggcttcggcaccttcccagccttgaaaaagcaatgctgcatctttttgaaaagctaatctccagtgagagaaattgtctgagaaggatcgaatgctttataaaagattcatcgctgcctcaagcagcctgccaccctgccatattccgggttgttgatgagatgttcaggtgtgcactcctggaaaccgatggggccctggaaatcatagccactattcaggtgtttacgcagtgctttgtagaagctctggagaaagcaagcaagcagctgcggtttgcactcaagacctactttccttacacttctccatctcttgccatggtgctgctgcaagaccctcaagatatccctcgggggcacactggctccagacactgaagcatatttctgaactgctcagagaagcagttgaagaccagactcatgggtcctgcggaggtccctttgagagctggttcctgttcattcacttcggaggatgggctgagatggtggcagagcaattactgatgtcggcagccgaaccccccacggccctgctgtggctcttggccttctactacggcccccgtgatgggaggcagcagagagcacagactatggtccaggtgaaggccgtgctgggccacctcctggcaatgtccagaagcagcagcctctcagcccaggacctgcagacggtagcaggacagggcacagacacagacctcagagctcctgcacaacagctgatcaggcaccttctcctcaacttcctgctctgggctcctggaggccacacgatcgcct**g**ggatgtcatcaccctgatggctcacactgctgagataactcacgagatcattggctttcttgaccagaccttgtacagatggaatcgtcttggcattgaaagccctagatcagaaaaactggcccgagagctccttaaagagctgcgaactcaagtctag-3' (SEQ ID NO:338)

*MYBPC3*

5'-atgcctgagccggggaagaagccagtctcagcttttagcaagaagccacggtcagtggaagtggccgcaggcagccctgccgtgttcgaggccgagacagagcgggcaggagtgaaggtgcgctggcagcgcggaggcagtgacatcagcgccagcaacaagtacggcctggccacagagggcacacggcatacgctgacagtgcgggaagtgggccctgccgaccagggatcttacgcagtcattgctggctcctccaaggtcaagttcgacctcaaggtcatagaggcagagaaggcagagcccatgctggcccctgcccctgcccctgctgaggccactggagcccctggagaagcccccggccccagccgctgagctgggagaaagtgccccaagtcccaaagggtcaagctcagcagctctcaatggtcctaccccctggagcccccgatgacccccattggcctcttcgtgatgcggccacaggatggcgaggtgaccgtgggtggcagcatcaccttctcagcccgcgtggccggcgccagcctcctgaagccgcctgtggtcaagtggttcaagggcaaatgggtggacctgagcagcaaggtgggccagcacctgcagctgcacgacagctacgaccgcgccagcaaggtctatctgttcgagctgcacatcaccgatgcccagcctgccttcactggcagctaccgctgtgaggtgtccaccaaggacaaatttgactgctccaacttcaatctcactgtccacgaggccatgggcaccggagacctggacctcctatcagccttccgccgcacgagcctggctggaggtggtcggcggatcagtgatagccatgaggacactgggattctggacttcagctcactgctgaaaaagagagacagtttccggaccccgagggactcgaagctggaggcaccagcagaggaggacgtgtgggagatcctacggcaggcacccccatctgagtacgagcgcatcgccttccagtacggcgtcactgacctgcgcggcatgctaaagaggctcaagggcatgaggcgcgatgagaagaagagcacagcctttcagaagaagctggagccggcctaccaggtgagcaaaggccacaagatccggctgaccgtggaactggctgaccatgacgctgaggtcaaatggctcaagaatggccaggagatccagatgagcggcagcaagtacatctttgagtccatcggtgccaagcgtaccctgaccatcagccagtgctcattggcggacgacgcagcctaccagtgcgtggtgggtggcgagaagtgtagcacggagctctttgtgaaagagcccctgtgctcatcacgcgccccttggaggaccagctggtgatggtggggcagcgggtggagtttgagtgtgaagtatcggaggaggggcgcaagtcaaatggctgaaggacggggtggagctgacccgggaggagaccttcaaataccggttcaagaaggacgggcagagacaccacctgatcatcaacgaggccatgctggaggacgcggggcactatgcactgtgcactagcggggccaggcgctggctgagctcattgtgcaggaaaagaagctggaggtgtaccagagcatcgcagacctgatggtgggcgcaaaggaccaggcggtgttcaaatgtgaggtctcagatgagaatgttcggggtgtgtggctgaagaatgggaaggagctggtgcccgacagccgcataaaggtgtcccacatcgggcgggtccacaaactgaccattgacgacgtcacacctgccgacgaggctgactacagctttgtgcccgagggcttcgcctgcaacctgtcagccaagctccacttcatggaggtcaagattgacttcgtacccaggcaggaacctcccaagatccacctggactgccccaggccgcataccagacaccattgtggttgtagctggaaataagctacgtctggacgtccctatctctggggaccctgctcccactgtgatctggcagaaaggctatcacgcaggggaataaggccccagccaggccagccccagatgccccagaggacacaggtgacagcgatgagtgggtgtttgacaagaagctgctgtgtgagaccgagggccgggtccgcgtggagaccaccaaggaccgcagcatcttcacggtcgagggggcagagaaggaagatgagggcgctctacacggtcacagtgaagaaccctgtgggcgaggaccaggtcaacctcacagtcaaggtcatcgacgtgccagacgcaacctgcggcccccaagatcagcaacgtgggagaggactcctgcacagtacagtgggagccgcctgcctacgatggcgggcagcccatcctgggctacatcctggacgcgcaagaagaagaagagctaccggtggatgcggctgaacttcgacctgattcaggagctgagtcatgaagcgcggcgcatgatcgagggcgtggtgtacgagatgcgcgtctacgcggtcaacgccatcggcatgtccaggcccagccctgcctcccagcccttcatgcctatcggtccccccagcgaacccacccacctggcagtagag

gacgtctctgacaccacggtctccctcaagtggcggcccccagagcgcgtgggagcaggaggcctggatggctacagcgtggagtactgcccagagggctgctca

gagtgggtggctgccctgcaggggctgacagagcacacatcgatactggtgaaggacctgcccacggggggcccggctgcttttccgagtgcgggcacacaatatg

gcaggggcctggagcccctgttaccaccacggagccggtgacagtgcaggagatcctgcaacggccacggcttcagctgcccaggcacctgcgccagaccattcag

aagaaggtcggggagcctgtgaaccttctcatccctttccagggcaagccccggcctcaggtgacctggaccaaagaggggcagcccctggcaggcgaggaggt

gagcatccgcaacagcccacagacaccatcctgttcatccgggccgctcgccgcgtgcattcaggcacttaccaggtgacggtgcgcattgagaacatggaggac

aaggccacgctggtgctgcaggttgttgacaagccaagtcctccccaggatctccgggtgactgacgcctggggtcttaatgtggctctggagtggaagccacccca

ggatgtcggcaacacggagctct**g**ggggtacacagtgcagaaagccgacaagaagaccatggagtggttcaccgtcttggagcattaccgccgcacccactgcgt

ggtgccagagctcatcattggcaatggctactacttccgcgtcttcagccagaatatggttggctttagtgacagagcggccaccaccaaggagcccgtctttatcccca

gaccaggcatcacctatgagccacccaactataaggccctggacttctccgaggccccaagcttcacccagcccctggtgaaccgctcggtcatcgcgggctacact

gctatgctctgctgtgctgtccggggtagccccaagcccaagatttcctggttcaagaatggcctggacctgggagaagacgcccgcttccgcatgttcagcaagcag

ggagtgttgactctggagattagaaagccctgcccctttgacggggggcatctatgtctgcagggccaccaacttacagggcgaggcacggtgtgagtgccgcctgga

ggtgcgagtgcctcagtga-3' (SEQ ID NO:339)

*IL2RG*

5'-atgttgaagccatcattaccattcacatccctcttattcctgcagctgcccctgctggggagtgggggctgaacacgacaattctgacgcccaatgggaatgaagacac

cacagctgatttcttcctgaccactatgcccactgactccctcagtgtttccactctgcccctcccagaggttcagtgttttgtgttcaatgtcgagtacatgaattgcacttgg

aacagcagctctgagccccagcctaccaacctcactctgcattattggtacaagaactcggataatgatAaagtccagaagtgcagccactatctattctctgaagaaat

cacttctggctgtcagttgcaaaaaaaggagatccacctctaccaaacatttgttgttcagctccaggacccacgggaacccaggagacaggccacacagatgctaaa

actgcagaatctggtgatccccctgggctccagagaacctaacacttcacaaactgagtgaatcccagctagaactgaactggaacaacagattcttgaaccactgtttgg

agcacttggtgcagtaccggactgactgggaccacagctggactgaacaatcagtggattatagacataagttctccttgcctagtgtggatgggcagaaacgctacac

gtttcgtgttcggagccgctttaacccactctgtggaagtgctcagcatt**g**gagtgaatggagccacccaatccactgggggagcaatacttcaaaagagaatcctttcc

tgtttgcattggaagccgtggttatctctgttggctccatgggattgattatcagccttctctgtgtgtatttctggctggaacggacgatgccccgaattcccaccctgaaga

acctagaggatcttgttactgaataccacgggaacttttcggcctggagtggtgtgtctaagggactggctgagagtctgcagccagactacagtgaacgactctgcctc

gtcagtgagattcccccaaaaggaggggcccttggggaggggcctggggcctccccatgcaaccagcatagcccctactgggcccccccatgttacaccctaaagc

ctgaaacctga-3' (SEQ ID NO:340)

## Discussion

Genome editing technologies are revolutionizing biomedical research. Highly active nucleases, such as zinc finger nucleases (ZFNs)[1], transcription activator-like effector nucleases (TALENs)[2-4], and Cas proteins of CRISPR (Clustered Regularly Interspaced Short Palindromic Repeats) system[5-7] have been successfully engineered to manipulate the genome in a myriad of organisms. Recently, deaminases have been harnessed to precisely change the genetic code without breaking double-stranded DNA. By coupling a cytidine or an adenosine deaminase with the CRISPR-Cas9 system, researchers created programmable base editors that enable the conversion of C•G to T•A or A•T to G•C in genomic DNA[8-10], offering novel opportunities for correcting disease-causing mutations.

Aside from DNA, RNA is an attractive target for genetic correction because RNA modification could alter the protein function without generating any permanent changes to the genome. The ADAR adenosine deaminases are currently exploited to achieve precise base editing on RNAs. Three kinds of ADAR proteins have been identified in mammals, ADAR1 (isoforms p110 and p150), ADAR2 and ADAR3 (catalytic inactive)[11,12], whose substrates are double-stranded RNAs, in which an adenosine (A) mismatched with a cytosine (C) is preferentially deaminated to inosine (I). Inosine is believed to mimic guanosine (G) during translation[13,14]. To achieve targeted RNA editing, the ADAR protein or its catalytic domain was fused with a λN peptide[15-17], a SNAP-tag[18-22] or a Cas protein (dCas13b)[23], and a guide RNA was designed to recruit the chimeric ADAR protein to the specific site. Alternatively, overexpressing ADAR1 or ADAR2 proteins together with an R/G motif-bearing guide RNA was also reported to enable targeted RNA editing[24-27].

All these reported nucleic acid editing methods in mammalian system rely on ectopic expression of two components: an enzyme and a guide RNA. Although these binary systems work efficiently in most studies, some inherent obstacles limit their broad applications, especially in therapies. Because the most effective *in vivo* delivery for gene therapy is through viral vectors[28], and the highly desirable adeno-associated virus (AAV) vectors are limited with cargo size (~4.5 kb), making it challenging for accommodating both the protein and the guide RNA[29,30]. Over-expression of ADAR1 has recently been reported to confer oncogenicity in multiple myelomas due to aberrant hyper-editing on RNAs[31], and to generate substantial global off-targeting edits[32]. In addition, ectopic expression of proteins or their domains of non-human origin has potential risk of eliciting immunogenicity[30,33]. Moreover, pre-existing adaptive immunity and p53-mediated DNA damage response may compromise the efficacy of the therapeutic protein, such as Cas9[34-38]. Although it has been attempted to utilize endogenous mechanism for RNA editing, this was tried only by injecting pre-assembled target transcript:RNA duplex into *Xenopus* embryos[39]. Alternative technologies for robust nucleic acid editing that don't rely on ectopic expression of proteins are much needed. Here, we developed a novel approach that leverages endogenous ADAR for RNA editing. We showed that expressing a deliberately designed guide RNA enables efficient and precise editing on endogenous RNAs, and corrects pathogenic mutations. This unary nucleic acid editing platform may open new avenues for therapeutics and research.

In particular, we showed that expression of a linear arRNA with adequate length is capable of guiding endogenous ADAR proteins to edit adenosine to inosine on the targeted transcripts. This system, referred to as LEAPER, utilizes endogenous ADAR proteins to achieve programmable nucleic acid editing, thus possessing advantages over existing approaches.

The rare quality of LEAPER is its simplicity because it only relies on a small size of RNA molecule to direct the endogenous proteins for RNA editing. This is reminiscent of RNAi, in which a small dsRNA could invoke native mechanism for targeted RNA degradation[51]. Because of the small size, arRNA could be readily delivered by a variety of viral and non-viral vehicles. Different from RNAi, LEAPER catalyzes the precise A to I switch without generating cutting or degradation of targeted transcripts (Fig. 18A). Although the length requirement for arRNA is longer than RNAi, it neither induces immune-stimulatory effects at the cellular level (Fig. 22E, f and Fig. 29E) nor affects the function of endogenous ADAR proteins (Fig. 22A, B), making it a safe strategy for RNA targeting. Remarkably, it has been reported that ectopic expression of ADAR proteins or their catalytic domains induces substantial global off-target edits[32] and possibly triggers cancer[31].

Recently, several groups reported that cytosine base editor could generate substantial off-target single-nucleotide variants in mouse embryos, rice or human cell lines due to the expression of an effector protein, which illustrates the advantage of LEAPER for potential therapeutic application[52-54]. Gratifyingly, LEAPER empowers efficient editing while elicits rare global off-target editing (Fig. 20 and Fig. 21). In addition, LEAPER could minimize potential immunogenicity or surmount delivery obstacles commonly shared by other methods that require the introduction of foreign proteins.

For LEAPER, we would recommend using arRNA with a minimal size above 70-nt to achieve desirable activity. In the native context, ADAR proteins non-specifically edit Alu repeats which have a duplex of more than 300-nt[55]. Of note, Alu repeats form stable intramolecular duplex, while the LEAPER results in an intermolecular duplex between arRNA and mRNA or pre-mRNA, which is supposed to be less stable and more difficult to form. Therefore, we hypothesized that an RNA duplex longer than 70-nt is stoichiometrically important for recruiting or docking ADAR proteins for effective editing. Indeed, longer arRNA resulted in higher editing yield in both ectopically expressed reporters and endogenous transcripts (Fig. 16D and Fig. 17B). However, because ADAR proteins promiscuously deaminate adenosine base in the RNA duplex, longer arRNA may incur more off-targets within the targeting window.

While LEAPER could effectively target native transcripts, their editing efficiencies and off-target rates varied. For *PPIB* transcript-targeting, we could convert 50% of targeted adenosine to inosine without evident off-targets within the covering windows (Fig. 17B, F). The off-targets became more severe for other transcripts. We have managed to reduce off-targets such as introducing A-G mismatches or consecutive mismatches to repress undesired editing. However, too many mismatches could decrease on-target efficiency. Weighing up the efficiency and potential off-targets, we would recommend arRNA with the length ranging from 100-to 150-nt for editing on endogenous transcripts. If there is a choice, it's better to select regions with less adenosine to minimize the chance of unwanted edits. Encouragingly, we have not detected any off-targets outside of the arRNA-targeted-transcript duplexes (Fig. 20).

We have optimized the design of the arRNA to achieve improved editing efficiency and demonstrated that LEAPER could be harnessed to manipulate gene function or correct pathogenic mutation. We have also shown that LEAPER is not limited to only work on UAG, instead that it works with possibly any adenosine regardless of its flanking nucleotides (Fig. 16F, G and Fig. 17C). Such flexibility is advantageous for potential therapeutic correction of genetic diseases caused by certain single point mutations. Interestingly, in editing the *IDUA* transcripts, the arRNA targeting pre-mRNA is more effective than that targeting mature RNA, indicating that nuclei are the main sites of action for ADAR proteins and LEAPER could be leveraged to manipulate splicing by modifying splice sites within pre-mRNAs. What's more, LEAPER has demonstrated high efficiency for simultaneously targeting multiple gene transcripts (Fig. 17D). This multiplexing capability of LEAPER might be developed to cure certain polygenetic diseases in the future.

It is beneficial to perform genetic correction at the RNA level. First, editing on targeted transcripts would not permanently change the genome or transcriptome repertoire, making RNA editing approaches safer for therapeutics than means of genome editing. In addition, transient editing is well suited for temporal control of treating diseases caused by occasional changes in a specific state. Second, LEAPER and other RNA editing methods would not introduce DSB on the genome, avoiding the risk of generating undesirable deletions of large DNA fragments[37]. DNA base editing methods adopting nickase Cas9 could still generate indels in the genome[8]. Furthermore, independent of native DNA repair machinery, LEAPER should also work in post-mitosis cells such as cerebellum cells with high expression of ADAR2[11].

We have demonstrated that LEAPER could apply to a broad spectrum of cell types such as human cell lines (Fig. 14C), mouse cell lines (Fig. 14D) and human primary cells including primary T cells (Fig. 27 and Fig. 28D). Efficient editing through lentiviral delivery or synthesized oligo provides increased potential for therapeutic development (Fig. 28). Moreover, LEAPER could produce phenotypic or physiological changes in varieties of applications including recovering the transcriptional regulatory activity of p53 (Fig. 7), correcting pathogenic mutations (Fig. 26), and restoring the $\alpha$-L-iduronidase activity in Hurler syndrome patient-derived primary fibroblasts (Fig. 29). It can thus be envisaged that LEAPER has enormous potential in disease treatment.

Stafforst and colleagues reported a new and seemingly similar RNA editing method, named RESTORE, which works through recruiting endogenous ADARs using synthetic antisense oligonucleotides[56]. The fundamental difference between RESTORE and LEAPER lies in the distinct nature of the guide RNA for recruiting endogenous ADAR. The guide RNA of RESTORE is limited to chemosynthetic antisense oligonucleotides (ASO) depending on complex chemical modification, while arRNA of LEAPER can be generated in a variety of ways, chemical synthesis and expression from viral or non-viral vectors (Fig. 28 and Fig. 29). Importantly, being heavily chemically modified, ASOs is restricted to act transiently in disease treatment. In contrast, arRNA could be produced through expression, a feature particularly important for the purpose of constant editing.

There are still rooms for improvements regarding LEAPER's efficiency and specificity. Because LEAPER relies on the endogenous ADAR, the expression level of ADAR proteins in target cells is one of the determinants for successful editing. According to previous report[57] and our observations (Fig. 14A, B), the ADAR1p110 is ubiquitously expressed across tissues, assuring the broad applicability of LEAPER. The ADAR1p150 is an interferon-inducible isoform[58], and has proven to be functional in LEAPER (Fig. 11E, Fig. 12B). Thus, co-transfection of interferon stimulatory RNAs with the arRNA might further improve editing efficiency under certain circumstances. Alternatively, as ADAR3 plays inhibitory roles, inhibition of ADAR3 might enhance editing efficiency in ADAR3-expressing cells. Moreover, additional modification of arRNA might increase its editing efficiency. For instance, arRNA fused with certain ADAR-recruiting scaffold may increase local ADAR protein concentration and consequently boost editing yield. So far, we could only leverage endogenous ADAR1/2 proteins for the A to I base conversion. It is exciting to explore whether more native mechanisms could be harnessed similarly for the modification of genetic elements, especially to realize potent nucleic acid editing.

Altogether, we provided a proof of principle that the endogenous machinery in cells could be co-opted to edit RNA transcripts. We demonstrated that LEAPER is a simple, efficient and safe system, shedding light on a novel path for gene editing-based therapeutics and research.

## References

1 Porteus, M. H. & Carroll, D. Gene targeting using zinc finger nucleases. Nat Biotechnol 23, 967-973 (2005).

2 Boch, J. et al. Breaking the code of DNA binding specificity of TAL-type III effectors. Science 326, 1509-1512 (2009).

3 Moscou, M. J. & Bogdanove, A. J. A simple cipher governs DNA recognition by TAL effectors. Science 326, 1501 (2009).

4 Miller, J. C. et al. A TALE nuclease architecture for efficient genome editing. Nat Biotechnol 29, 143-148 (2011).

5 Jinek, M. et al. A programmable dual-RNA-guided DNA endonuclease in adaptive bacterial immunity. Science 337, 816-821 (2012).

6 Cong, L. et al. Multiplex genome engineering using CRISPR/Cas systems. Science 339, 819-823 (2013).

7 Mali, P. et al. RNA-guided human genome engineering via Cas9. Science 339, 823-826 (2013).

8 Komor, A. C., Kim, Y. B., Packer, M. S., Zuris, J. A. & Liu, D. R. Programmable editing of a target base in genomic DNA without double-stranded DNA cleavage. Nature 533, 420-424 (2016).

9 Ma, Y. et al. Targeted AID-mediated mutagenesis (TAM) enables efficient genomic diversification in mammalian cells. Nat Methods 13, 1029-1035 (2016).

10 Gaudelli, N. M. et al. Programmable base editing of A*T to G*C in genomic DNA without DNA cleavage. Nature 551, 464-471 (2017).

11 Tan, M. H. et al. Dynamic landscape and regulation of RNA editing in mammals. Nature 550, 249-254 (2017).

12 Nishikura, K. Functions and regulation of RNA editing by ADAR deaminases. Annu Rev Biochem 79, 321-349 (2010).

13 Bass, B. L. & Weintraub, H. An unwinding activity that covalently modifies its double-stranded RNA substrate.

Cell 55, 1089-1098 (1988).

14 Wong, S. K., Sato, S. & Lazinski, D. W. Substrate recognition by ADAR1 and ADAR2. RNA 7, 846-858 (2001).

15 Montiel-Gonzalez, M. F., Vallecillo-Viejo, I., Yudowski, G. A. & Rosenthal, J. J. Correction of mutations within the cystic fibrosis transmembrane conductance regulator by site-directed RNA editing. Proc Natl Acad Sci U S A 110, 18285-18290 (2013).

16 Sinnamon, J. R. et al. Site-directed RNA repair of endogenous Mecp2 RNA in neurons. Proc Natl Acad Sci U S A 114, E9395-E9402 (2017).

17 Montiel-Gonzalez, M. F., Vallecillo-Viejo, I. C. & Rosenthal, J. J. An efficient system for selectively altering genetic information within mRNAs. Nucleic Acids Res 44, e157 (2016).

18 Hanswillemenke, A., Kuzdere, T., Vogel, P., Jekely, G. & Stafforst, T. Site-Directed RNA Editing in Vivo Can Be Triggered by the Light-Driven Assembly of an Artificial Riboprotein. JAm Chem Soc 137, 15875-15881 (2015).

19 Schneider, M. F., Wettengel, J., Hoffmann, P. C. & Stafforst, T. Optimal guideRNAs for re-directing deaminase activity of hADARI and hADAR2 in trans. Nucleic Acids Res 42, e87 (2014).

20 Vogel, P., Hanswillemenke, A. & Stafforst, T. Switching Protein Localization by Site-Directed RNA Editing under Control of Light. ACS synthetic biology 6, 1642-1649 (2017).

21 Vogel, P., Schneider, M. F., Wettengel, J. & Stafforst, T. Improving site-directed RNA editing in vitro and in cell culture by chemical modification of the guideRNA. Angewandte Chemie 53, 6267-6271 (2014).

22 Vogel, P. et al. Efficient and precise editing of endogenous transcripts with SNAP-tagged ADARs. Nat Methods 15, 535-538 (2018).

23 Cox, D. B. T. et al. RNA editing with CRISPR-Cas13. Science 358, 1019-1027 (2017).

24 Fukuda, M. et al. Construction of a guide-RNA for site-directed RNA mutagenesis utilising intracellular A-to-I RNA editing. Scientific reports 7, 41478 (2017).

25 Wettengel, J., Reautschnig, P., Geisler, S., Kahle, P. J. & Stafforst, T. Harnessing human ADAR2 for RNA repair - Recoding a PINK1 mutation rescues mitophagy. Nucleic Acids Res 45, 2797-2808 (2017).

26 Heep, M., Mach, P., Reautschnig, P., Wettengel, J. & Stafforst, T. Applying Human ADAR1p110 and ADAR1p150 for Site-Directed RNA Editing-G/C Substitution Stabilizes GuideRNAs against Editing. Genes (Basel) 8 (2017).

27 Katrekar, D. et al. In vivo RNA editing of point mutations via RNA-guided adenosine deaminases. Nat Methods 16, 239-242 (2019).

28 Yin, H., Kauffman, K. J. & Anderson, D. G. Delivery technologies for genome editing. Nat Rev Drug Discov 16, 387-399 (2017).

29 Platt, R. J. et al. CRISPR-Cas9 knockin mice for genome editing and cancer modeling. Cell 159, 440-455 (2014).

30 Chew, W. L. et al. A multifunctional AAV-CRISPR-Cas9 and its host response. Nat Methods 13, 868-874 (2016).

31 Teoh, P. J. et al. Aberrant hyperediting of the myeloma transcriptome by ADAR1 confers oncogenicity and is a marker of poor prognosis. Blood 132, 1304-1317 (2018).

32 Vallecillo-Viejo, I. C., Liscovitch-Brauer, N., Montiel-Gonzalez, M. F., Eisenberg, E. & Rosenthal, J. J. C. Abundant off-target edits from site-directed RNA editing can be reduced by nuclear localization of the editing enzyme. RNA biology 15, 104-114 (2018).

33 Mays, L. E. & Wilson, J. M. The complex and evolving story of T cell activation to AAV vector-encoded transgene products. Mol Ther 19, 16-27 (2011).

34 Wagner, D. L. et al. High prevalence of Streptococcus pyogenes Cas9-reactive T cells within the adult human population. NatMed 25, 242-248 (2019).

35 Simhadri, V. L. et al. Prevalence of Pre-existing Antibodies to CRISPR-Associated Nuclease Cas9 in the USA Population. Mol Ther Methods Clin Dev 10, 105-112 (2018).

36 Charlesworth, C. T. et al. Identification of preexisting adaptive immunity to Cas9 proteins in humans. NatMed 25, 249-254 (2019).

37 Haapaniemi, E., Botla, S., Persson, J., Schmierer, B. & Taipale, J. CR!SPR-Cas9 genome editing induces a p53-mediated DNA damage response. Nat Med 24, 927-930 (2018).

38 Ihry, R. J. et al. p53 inhibits CRISPR-Cas9 engineering in human pluripotent stem cells. Nat Med 24, 939-946 (2018).

39 Woolf, T. M., Chase, J. M. & Stinchcomb, D. T. Toward the therapeutic editing of mutated RNA sequences. Proc Natl Acad Sci U S A 92, 8298-8302 (1995).

40 Zheng, Y., Lorenzo, C. & Beal, P. A. DNA editing in DNA/RNA hybrids by adenosine deaminases that act on RNA. Nucleic Acids Res 45, 3369-3377 (2017).

41 Abudayyeh, O. O. et al. C2c2 is a single-component programmable RNA-guided RNA-targeting CRISPR effector. Science 353, aaf5573 (2016).

42 Daniel, C., Widmark, A., Rigardt, D. & Ohman, M. Editing inducer elements increases A-to-I editing efficiency in the mammalian transcriptome. Genome Biol 18, 195 (2017).

43 Chen, C. X. et al. A third member of the RNA-specific adenosine deaminase gene family, ADAR3, contains both single- and double-stranded RNA binding domains. RNA 6, 755-767 (2000).

44 Savva, Y. A., Rieder, L. E. & Reenan, R. A. The ADAR protein family. Genome Biol 13, 252 (2012).

45 Nishikura, K. A-to-I editing of coding and non-coding RNAs by ADARs. Nat Rev Mol Cell Biol 17, 83-96 (2016).

46 Floquet, C., Deforges, J., Rousset, J. P. & Bidou, L. Rescue of non-sense mutated p53 tumor suppressor gene by aminoglycosides. Nucleic Acids Res 39, 3350-3362 (2011).

47 Kern, S. E. et al. Identification of p53 as a sequence-specific DNA-binding protein. Science 252, 1708-1711 (1991).

48 Doubrovin, M. et al. Imaging transcriptional regulation of p53-dependent genes with positron emission tomography in vivo. Proc Natl Acad Sci U S A 98, 9300-9305 (2001).

49 Landrum, M. J. et al. ClinVar: public archive of interpretations of clinically relevant variants. Nucleic Acids Res 44, D862-868 (2016).

50 Ou, L. et al. ZFN-Mediated In Vivo Genome Editing Corrects Murine Hurler Syndrome. Mol Ther 27, 178-187 (2019).

51 Fire, A. et al. Potent and specific genetic interference by double-stranded RNA in Caenorhabditis elegans. Nature 391, 806-811 (1998).

52 Zuo, E. et al. Cytosine base editor generates substantial off-target single-nucleotide variants in mouse embryos. Science (2019).

53 Jin, S. et al. Cytosine, but not adenine, base editors induce genome-wide off-target mutations in rice. Science (2019).

54 Kim, D., Kim, D. E., Lee, G., Cho, S. I. & Kim, J. S. Genome-wide target specificity of CRISPR RNA-guided adenine base editors. Nat Biotechnol 37, 430-435 (2019).

55 Levanon, E. Y. et al. Systematic identification of abundant A-to-I editing sites in the human transcriptome. Nat Biotechnol 22, 1001-1005 (2004).

56 Merkle, T. et al. Precise RNA editing by recruiting endogenous ADARs with antisense oligonucleotides. Nat Biotechnol 37, 133-138 (2019).

57 Wagner, R. W. et al. Double-stranded RNA unwinding and modifying activity is detected ubiquitously in primary tissues and cell lines. Mol Cell Biol 10, 5586-5590 (1990).

58 Patterson, J. B. & Samuel, C. E. Expression and regulation by interferon of a double-stranded-RNA-specific adenosine deaminase from human cells: evidence for two forms of the deaminase. Mol Cell Biol 15, 5376-5388 (1995).

59 Gibson, D. G. et al. Enzymatic assembly of DNA molecules up to several hundred kilobases. Nat Methods 6, 343-345 (2009).

60 Zhou, Y., Zhang, H. & Wei, W. Simultaneous generation of multi-gene knockouts in human cells. FEBS Lett 590, 4343-4353 (2016).

61 Dobin, A. et al. STAR: ultrafast universal RNA-seq aligner. Bioinformatics 29, 15-21 (2013).

62 Van der Auwera, G. A. et al. From FastQ data to high confidence variant calls: the Genome Analysis Toolkit best practices pipeline. Curr Protoc Bioinformatics 43, 11 10 11-33 (2013).

63 Wang, K., Li, M. & Hakonarson, H. ANNOVAR: functional annotation of genetic variants from high-throughput sequencing data. Nucleic Acids Res 38, e164 (2010).

64 Genomes Project, C. et al. An integrated map of genetic variation from 1,092 human genomes. Nature 491, 56-65 (2012).

65 Pertea, M., Kim, D., Pertea, G. M., Leek, J. T. & Salzberg, S. L. Transcript-level expression analysis of RNA-seq experiments with HISAT, StringTie and Ballgown. Nat Protoc 11, 1650-1667 (2016).

## Claims

1. A method for editing a target RNA in a host cell, comprising introducing a deaminase-recruiting RNA (dRNA) or a construct encoding the dRNA into the host cell,

   wherein the dRNA comprises a complementary RNA sequence that hybridizes to the target RNA and a cytidine mismatch directly opposite the target adenosine in the target RNA,
   wherein the cytidine mismatch is located at least 20 nucleotides away from the 3' end of the complementary sequence, and at least 5 nucleotides away from the 5' end of the complementary sequence in the dRNA,
   wherein the dRNA is 50 to 260 nucleotides in length and does not comprise a chemically modified nucleotide, and
   wherein the dRNA is capable of recruiting an adenosine deaminase acting on RNA 1 or 2 (ADAR1 or ADAR2) to deaminate a target adenosine in the target RNA, and the dRNA does not comprise an ADAR-recruiting domain.

2. The method of claim 1, wherein the dRNA is 70 to 260 nucleotides in length.

3. The method of claim 2, wherein the dRNA is 100 to 150 nucleotides in length.

4. The method of any one of claims 1-3, wherein the ADAR1 or ADAR2 is an endogenously expressed by the host cell, or the ADAR1 or ADAR2 is an exogenous ADAR introduced to the host cell.

5. The method of claim 4, comprising introducing a construct encoding the dRNA into the host cell, wherein the construct is a viral vector or a plasmid.

6. The method of any one of claims 1-5, wherein the cytidine mismatch is located within 10 nucleotides from the center of the complementary sequence in the dRNA.

7. The method of any one of claims 1-6, wherein the complementary sequence further comprises one or more guanosines each opposite a non-target adenosine in the target RNA.

8. The method of claim 7, wherein the complementary sequence comprises two or more consecutive mismatch nucleotides opposite a non-target adenosine in the target RNA.

9. The method of any one of claims 1-8, wherein the target adenosine is in a three-base motif selected from the group consisting of UAG, UAC, UAA, UAU, CAG, CAC, CAA, CAU, AAG, AAC, AAA, AAU, GAG, GAC, GAA and GAU in the target RNA.

10. The method of claim 9, wherein the three-base motif is UAG, and wherein the dRNA comprises an A directly opposite the uridine in the three-base motif, a cytidine directly opposite the target adenosine, and a cytidine, guanosine or uridine directly opposite the guanosine in the three-base motif.

11. The method of any one of claims 1-10, wherein the target RNA is an RNA selected from the group consisting of a pre-messenger RNA, a messenger RNA, a ribosomal RNA, a transfer RNA, a long non-coding RNA and a small RNA.

12. The method of any one of claims 1-11, comprising introducing a plurality of dRNAs each targeting a different target RNA.

13. The method of any one of claims 1-12, wherein the host cell is a eukaryotic cell.

14. The method of any one of claims 1-13, wherein the host cell is a mammalian cell.

15. The method of any one of claims 1-14, wherein the host cell is a human or mouse cell.

16. A deaminase-recruiting RNA (dRNA) for deamination of a target adenosine in a target RNA by recruiting an Adenosine Deaminase Acting on RNA (ADAR), comprising a complementary RNA sequence that hybridizes to the target RNA, wherein the dRNA comprises the nucleic acid sequence of any one of SEQ ID NOs: 25-44, 142-205, 341-342.

17. A construct encoding the dRNA of claim 16.

18. A library comprising a plurality of the dRNAs of claim 16 or the construct of claim 17.

**Patentansprüche**

1. Verfahren zum Editieren einer Ziel-RNA in einer Wirtszelle, umfassend ein Einführen einer Deaminase-Rekrutierungs-RNA (dRNA) oder eines Konstrukts, das für die dRNA kodiert, in die Wirtszelle,

wobei die dRNA eine komplementäre RNA-Sequenz, die mit der Ziel-RNA hybridisiert, und eine Cytidin-Fehlpaarung direkt gegenüber dem Ziel-Adenosin in der Ziel-RNA umfasst,
wobei sich die Cytidin-Fehlpaarung mindestens 20 Nukleotide von dem 3'-Ende der komplementären Sequenz und mindestens 5 Nukleotide vom 5'-Ende der komplementären Sequenz in der dRNA befindet,
wobei die dRNA 50 bis 260 Nukleotide lang ist und kein chemisch modifiziertes Nukleotid umfasst, und
wobei die dRNA in der Lage ist, eine auf RNA 1 oder 2 wirkende Adenosin-Desaminase (ADAR1 oder ADAR2) zu rekrutieren, um ein Ziel-Adenosin in der Ziel-RNA zu desaminieren, und die dRNA keine ADAR-rekrutierende Domäne umfasst.

2. Verfahren nach Anspruch 1, wobei die dRNA 70 bis 260 Nukleotide lang ist.

3. Verfahren nach Anspruch 2, wobei die dRNA 100 bis 150 Nukleotide lang ist.

4. Verfahren nach einem der Ansprüche 1 bis 3, wobei das ADAR1 oder ADAR2 ein von der Wirtszelle endogen exprimiertes ADAR ist oder das ADAR1 oder ADAR2 ein exogenes ADAR ist, das in die Wirtszelle eingeführt wurde.

5. Verfahren nach Anspruch 4, umfassend ein Einführen eines Konstrukts, das für die dRNA kodiert, in die Wirtszelle, wobei das Konstrukt ein viraler Vektor oder ein Plasmid ist.

6. Verfahren nach einem der Ansprüche 1-5, wobei sich die Cytidin-Fehlpaarung innerhalb von 10 Nukleotiden von der Mitte der komplementären Sequenz in der dRNA befindet.

7. Verfahren nach einem der Ansprüche 1 bis 6, wobei die komplementäre Sequenz ferner ein oder mehrere Guanosine umfasst, jeweils gegenüber einem Nicht-Ziel-Adenosin in der Ziel-RNA.

8. Verfahren nach Anspruch 7, wobei die komplementäre Sequenz zwei oder mehrere aufeinanderfolgende Fehlpaarungs-Nukleotide gegenüber einem Nicht-Ziel-Adenosin in der Ziel-RNA umfasst.

9. Verfahren nach einem der Ansprüche 1 bis 8, wobei das Zieladenosin in einem Drei-Basen-Motiv ist, ausgewählt aus der Gruppe bestehend aus UAG, UAC, UAA, UAU, CAG, CAC, CAA, CAU, AAG, AAC, AAA, AAU, GAG, GAC, GAA und GAU in der Ziel-RNA.

10. Verfahren nach Anspruch 9, wobei das Drei-Basen-Motiv UAG ist und wobei die dRNA ein A direkt gegenüber dem Uridin in dem Drei-Basen-Motiv, ein Cytidin direkt gegenüber dem Zieladenosin und ein Cytidin, Guanosin oder Uridin direkt gegenüber dem Guanosin in dem Drei-Basen-Motiv umfasst.

11. Verfahren nach einem der Ansprüche 1-10, wobei die Ziel-RNA eine RNA ist, die ausgewählt ist aus der Gruppe, bestehend aus einer Prä-Messenger-RNA, einer Messenger-RNA, einer ribosomalen RNA, einer Transfer-RNA, einer langen nicht-kodierenden RNA und einer kleinen RNA.

12. Verfahren nach einem der Ansprüche 1 bis 11, umfassend ein Einführen einer Vielzahl von dRNAs, die jeweils eine andere Ziel-RNA targetieren.

13. Verfahren nach einem der Ansprüche 1-12, wobei die Wirtszelle eine eukaryotische Zelle ist.

14. Verfahren nach einem der Ansprüche 1-13, wobei die Wirtszelle eine Säugetierzelle ist.

15. Verfahren nach einem der Ansprüche 1-14, wobei die Wirtszelle eine menschliche oder Mauszelle ist.

16. Deaminase-rekrutierende RNA (dRNA) zur Desaminierung eines Zieladenosins in einer Ziel-RNA durch Rekrutieren einer auf RNA wirkenden Adenosin-Desaminase (ADAR), umfassend eine komplementäre RNA-Sequenz, die an die Ziel-RNA hybridisiert, wobei die dRNA die Nukleinsäuresequenz einer der folgenden SEQ ID NO umfasst: 25-44, 142-205, 341-342.

17. Konstrukt, das für die dRNA nach Anspruch 16 kodiert.

18. Bibliothek, umfassend eine Vielzahl der dRNAs nach Anspruch 16 oder das Konstrukt nach Anspruch 17.

**Revendications**

1. Procédé d'édition d'un ARN cible dans une cellule hôte, comprenant l'introduction d'un ARN de recrutement de désaminase (ARNd) ou d'une construction codant pour l'ARNd dans la cellule hôte,

dans lequel l'ARNd comprend une séquence d'ARN complémentaire qui s'hybride avec l'ARN cible et un mésappariement de cytidine directement en face de l'adénosine cible dans l'ARN cible,
dans lequel le mésappariement de cytidine est située à au moins 20 nucléotides de l'extrémité 3' de la séquence complémentaire et à au moins 5 nucléotides de l'extrémité 5' de la séquence complémentaire dans l'ARNd,
dans lequel l'ARNd est d'une longueur de 50 à 260 nucléotides et ne comprend pas de nucléotide chimiquement modifié, et
dans lequel l'ARNd est capable de recruter une adénosine désaminase agissant sur l'ARN 1 ou 2 (ADAR1 ou ADAR2) pour désaminer une adénosine cible dans l'ARN cible, et l'ARNd ne comprend pas de domaine de recrutement d'ADAR.

2. Procédé selon la revendication 1, dans lequel l'ARNd est d'une longueur de 70 à 260 nucléotides.

3. Procédé selon la revendication 2, dans lequel l'ARNd est d'une longueur de 100 à 150 nucléotides.

4. Procédé selon l'une quelconque des revendications 1 à 3, dans lequel l'ADAR1 ou l'ADAR2 est un ADAR exprimé de manière endogène par la cellule hôte, ou l'ADAR1 ou l'ADAR2 est un ADAR exogène introduit dans la cellule hôte.

5. Procédé selon la revendication 4 comprenant l'introduction d'une construction codant pour l'ARNd dans la cellule hôte, dans lequel la construction est un vecteur viral ou un plasmide.

6. Procédé selon l'une quelconque des revendications 1 à 5, dans lequel le mésappariement de cytidine est situé à moins de 10 nucléotides du centre de la séquence complémentaire dans l'ARNd.

7. Procédé selon l'une quelconque des revendications 1 à 6, dans lequel la séquence complémentaire comprend en outre une ou plusieurs guanosines opposées chacune à une adénosine non ciblée dans l'ARN cible.

8. Procédé selon la revendication 7, dans lequel la séquence complémentaire comprend deux nucléotides consécutifs de mésappariement ou plus en face d'une adénosine non ciblée dans l'ARN cible.

9. Procédé selon l'une quelconque des revendications 1 à 8, dans lequel l'adénosine cible se trouve dans un motif à trois bases choisi dans le groupe constitué par UAG, UAC, UAA, UAU, CAG, CAC, CAA, CAU, AAG, AAC, AAA, AAU, GAG, GAC, GAA et GAU dans l'ARN cible.

10. Procédé selon la revendication 9, dans lequel le motif à trois bases est UAG, et dans lequel l'ARNd comprend un A directement en face de l'uridine dans le motif à trois bases, une cytidine directement en face de l'adénosine cible, et une cytidine, une guanosine ou une uridine directement en face de la guanosine dans le motif à trois bases.

11. Procédé selon l'une quelconque des revendications 1 à 10, dans lequel l'ARN cible est un ARN choisi dans le groupe constitué par un ARN pré-messager, un ARN messager, un ARN ribosomal, un ARN de transfert, un long ARN non codant et un petit ARN.

12. Procédé selon l'une quelconque des revendications 1 à 11, comprenant l'introduction d'une pluralité d'ARNd ciblant chacun un ARN cible différent.

13. Procédé selon l'une quelconque des revendications 1 à 12, dans lequel la cellule hôte est une cellule eucaryote.

14. Procédé selon l'une quelconque des revendications 1 à 13, dans lequel la cellule hôte est une cellule de mammifère.

15. Procédé selon l'une quelconque des revendications 1 à 14, dans lequel la cellule hôte est une cellule humaine ou une cellule de souris.

16. ARN de recrutement de désaminase (ARNd) pour la désamination d'une adénosine cible dans un ARN cible en recrutant une adénosine désaminase agissant sur l'ARN (ADAR), comprenant une séquence d'ARN complémentaire qui s'hybride à l'ARN cible, dans lequel l'ARNd comprend la séquence d'acide nucléique de l'un quelconque des SEQ ID N° : 25-44, 142-205, 341-342.

17. Construction codant l'ARNd selon la revendication 16.

18. Bibliothèque comprenant une pluralité d'ARNd selon la revendication 16 ou la construction selon la revendication 17.

Figure.1A

Figure.1B

Figure.1C

Figure.1D

Figure.1E

Figure.1F

Figure.1G

Figure.1H

Figure.2A

Figure.2B

Figure.2C

Figure.2D

Figure.2E

Figure.2F

Figure.2G

Figure.2H

Figure.3A

Figure.3B

Figure.4A

Figure.4B

Figure.4C

Figure.4D

Figure.4E

Figure.4F

Figure.4G

Figure.4H

PPIB mRNA target

A position                    22        30  33 34    39              49                                    76   80
5'-CGGCGCCCGCCGCCCGCCCGGAGCCCGCGAGCA ACCCCAGUCCCCCCACCCGCGCGUGGCGGCGCCGGCUCCCUAGCCACCGCGGCCC

91              107                                            140
CACCCUCUUCCGGCCUCAGCUGUCCGGGCUGCUUUCGCCUCCGCCUGUGGAUGCUGCGCCTC-3'                    ↑ Targeted A

Figure.5A

Mock
EV
dRNA-151

22  30  33  34  39  49  76  80  91  107 140
A Position          ↑ Taregted A site

Editing rate(%)
0                              13

Figure.5B

KRAS mRNA target

A position  7        16 18  20          30 31 32 33  36     40 41  43 45 46 47        56           62          74   78 79  81              90
5'-GCUCCCAGGUGCGGGAGAGAGGCCUGCUGA A A AUGACUGA AUAUA A ACUUGUGGUAGUUGGAGCUGGUGGCGUAGGCA AGAGUGCCUUGAC

93 95 97    101 102  106 108 109
GAUACAGCUA AUUCAG A   AUC-3'                    ↑ Targeted A

Figure.5C

Mock
EV
dRNA-91
dRNA-111

A position:  7  16  18  20  30  31  32  33  36  40  41  43  45  46  47  56  62  74  78  79  81  90  93  95  97  101 102 106 108 109
A Position          ↑ Taregted A site

Editing rate(%)
0                              13

Figure.5D

A-G mismatch sites

dRNA-AG1: $A_{41}$, $A_{46}$, $A_{74}$

dRNA-AG2: $A_{41}$, $A_{43}$, $A_{45}$, $A_{46}$, $A_{74}$, $A_{79}$

dRNA-AG3: $A_{31}$, $A_{32}$, $A_{33}$, $A_{41}$, $A_{43}$, $A_{45}$, $A_{46}$, $A_{47}$, $A_{74}$, $A_{79}$

dRNA-AG4: $A_7$, $A_{31}$, $A_{32}$, $A_{33}$, $A_{40}$, $A_{41}$, $A_{43}$, $A_{45}$, $A_{46}$, $A_{47}$, $A_{74}$, $A_{79}$, $A_{95}$

Figure.5E

Figure.5F

Figure.5G

Figure.6A

Figure.6B

Figure.6C

Figure.6D

**Allel-1: 17 bp deletion in exon 2**

```
Mut CCACCTGTTCATTACAATGGCCCCTCAAAA------------------TGAAAATGGCCAG
    ||||||||||||||||||||||||||||||                  ||||||||||||
WT  CCACCTGTTCATTACAATGGCCCCTCAAAAGCAGGGTATGTTGACTTTGAAAATGGCCAG
```

**Allel-2: 1 bp insertion in exon 2**

```
Mut CCACCTGTTCATTACAATGGCCCCTCAAAAAGCAGGGTATGTTGACTTTGAAAATGGCCAG
    ||||||||||||||||||||||||||||||  ||||||||||||||||||||||||||||||
WT  CCACCTGTTCATTACAATGGCCCCTCAAAA  GCAGGGTATGTTGACTTTGAAAATGGCCAG
```

**Figure.6E**

**Figure.6F**

293T-WT cells

**Figure.6G**

**Figure.6H**

82

Figure.7A

Figure.7B

Figure.7C

Figure.8A

Figure.8B

Figure.9

Figure.10A

Figure.10B

Figure.10C

Figure.10D

Figure.11A

Figure.11B

Figure.11C

Figure.11D

Figure.11E

Figure.11F

Figure.11G

Figure.12A

Figure.12B

Figure.13

Figure.14A

Figure.14C

Figure.14B

Figure.14D

Figure.15A

Figure.15B

Figure.15C

Figure.16A

Figure.16B

Figure.16C

Figure.16D

Figure.16E

Figure.16F

Figure.16G

Figure.17A

Figure.17B

Figure.17C

Figure.17D

Figure.17F

**PPIB transcript targeting**

```
                        22        30 33/34   39              49                                    76   80              91              107
5'-CGGCGCCCGCCGCCCGCCCGGAGCCCGCGAGCAACCCCAGUCCCCCCCACCCGCGCGUGGCGGCGCCGGCUCCCUAGCCACCGCGGCCCCACCCUCUUCCGGCCUCAG
                    140
CUGUCCGGGCUGCUUUCGCCUCCGCCUGUGGAUGCUGCGCCUC-3'
```

Figure.17E

Figure.17G

Figure.17H

arRNA₁₁₁-AG6: A (61, 63, 65, 66, 94, 99)
arRNA₁₁₁-AG9: A (27, 61, 63, 65, 66, 94, 98, 99, 115)

Figure.17I

Figure.18A

Figure.18B

**KRAS transcript targeting**

Figure.19A

Figure.19B

**SMAD4 transcript targeting**

Figure.19C

Figure.19D

**FANCC transcript targeting**

Figure.19E

Figure.19F

Figure.20A

Figure.20B

Figure.20C

Figure.20D

Figure.21A

Figure.21B

Figure.22A

Figure.22C

Figure.22E

Figure.22B

Figure.22D

Figure.22F

*TP53^{W53X}* transcript targeting

Figure.23A

Figure.23C

Figure.23B

Figure.23D

Figure.24

Figure.25

Figure.26

Figure.27A

Figure.27B

Figure.27C

# EP 3 864 152 B1

Figure.28A

Figure.28B

Figure.28D

Figure.28C

Figure.29A

Figure.29B

Figure.29C

Figure.29D

Figure.29E

**Reporter-1:** (SEQ ID NO:1,SEQ ID NO:2, and SEQ ID NO:3)

5' mCherry | Linker | Target | eGFP | 3'

5'-
ATGGTGAGCAAGGGCGAGGAGGATAACATGGCCATCATCAAGGAGTTCATGCGCTTCAAGGTGCA
CATGGAGGGCTCCGTGAACGGCCACGAGTTCGAGATCGAGGGCGAGGGCGAGGGCCGCCCCTAC
GAGGGCACCCAGACCGCCAAGCTGAAGGTGACCAAGGGTGGCCCCCCTGCCCTTCGCCTGGGACAT
CCTGTCCCCTCAGTTCATGTACGGCTCCAAGGCCTACGTGAAGCACCCCGCCGACATCCCCGACTA
CTTGAAGCTGTCCTTCCCCGAGGGCTTCAAGTGGGAGCGCGTGATGAACTTCGAGGACGGCGGCG
TGGTGACCGTGACCCAGGACTCCTCCCTGCAGGACGGCGAGTTCATCTACAAGGTGAAGCTGCGC
GGCACCAACTTCCCCTCCGACGGCCCCGTAATGCAGAAGAAGACCATGGGCTGGGAGGCCTCCTC
CGAGCGGATGTACCCCGAGGACGGCGCCCTGAAGGGCGAGATCAAGCAGAGGCTGAAGCTGAAG
GACGGCGGCCACTACGACGCTGAGGTCAAGACCACCTACAAGGCCAAGAAGCCCGTGCAGCTGCC
CGGCGCCTACAACGTCAACATCAAGTTGGACATCACCTCCCACAACGAGGACTACACCATCGTGGA
ACAGTACGAACGCGCCGAGGGCCGCCACTCCACCGGCGGCATGGACGAGCTGTACAAG *CTGCAG*
*GGCGGAGGAGGCAGC GGCGGAGGAGGCAGC GGCGGAGGAGGCAGC*
GCCTGCTCGCGATGCTAGAGGGCTCTGCCA
GTGAGCAAGGGCGAGGAGCTGTTCACCGGGGTGGTGCCCATCCTGGTCGAGCTGGACGGCGACGTAAAC
GGCCACAAGTTCAGCGTGTCCGGCGAGGGCGAGGGCGATGCCACCTACGGCAAGCTGACCCTGAAGTTC
ATCTGCACCACCGGCAAGCTGCCCGTGCCCTGGCCCACCCTCGTGACCACCCTGACCTACGGCGTGCAGT
GCTTCAGCCGCTACCCCGACCACATGAAGCAGCACGACTTCTTCAAGTCCGCCATGCCCGAAGGCTACGT
CCAGGAGCGCACCATCTTCTTCAAGGACGACGGCAACTACAAGACCCGCGCCGAGGTGAAGTTCGAGGG
CGACACCCTGGTGAACCGCATCGAGCTGAAGGGCATCGACTTCAAGGAGGACGGCAACATCCTGGGGCA
CAAGCTGGAGTACAACTACAACAGCCACAACGTCTATATCATGGCCGACAAGCAGAAGAACGGCATCAA
GGTGAACTTCAAGATCCGCCACAACATCGAGGACGGCAGCGTGCAGCTCGCCGACCACTACCAGCAGAA
CACCCCCATCGGCGACGGCCCCGTGCTGCTGCCCGACAACCACTACCTGAGCACCCAGTCCGCCCTGAGC
AAAGACCCCAACGAGAAGCGCGATCACATGGTCCTGCTGGAGTTCGTGACCGCCGCCGGGATCACTCTC
GGCATGGACGAGCTGTACAAGTAA-3'

## Figure.30A

**Reporter-2:** (SEQ ID NO:1,SEQ ID NO:4, and SEQ ID NO:3)

5' mCherry | Linker | Target | eGFP | 3'

5'-
ATGGTGAGCAAGGGCGAGGAGGATAACATGGCCATCATCAAGGAGTTCATGCGCTTCAAGGTGCA
CATGGAGGGCTCCGTGAACGGCCACGAGTTCGAGATCGAGGGCGAGGGCGAGGGCCGCCCCTAC
GAGGGCACCCAGACCGCCAAGCTGAAGGTGACCAAGGGTGGCCCCCCTGCCCTTCGCCTGGGACAT
CCTGTCCCCTCAGTTCATGTACGGCTCCAAGGCCTACGTGAAGCACCCCGCCGACATCCCCGACTA
CTTGAAGCTGTCCTTCCCCGAGGGCTTCAAGTGGGAGCGCGTGATGAACTTCGAGGACGGCGGCG
TGGTGACCGTGACCCAGGACTCCTCCCTGCAGGACGGCGAGTTCATCTACAAGGTGAAGCTGCGC
GGCACCAACTTCCCCTCCGACGGCCCCGTAATGCAGAAGAAGACCATGGGCTGGGAGGCCTCCTC
CGAGCGGATGTACCCCGAGGACGGCGCCCTGAAGGGCGAGATCAAGCAGAGGCTGAAGCTGAAG
GACGGCGGCCACTACGACGCTGAGGTCAAGACCACCTACAAGGCCAAGAAGCCCGTGCAGCTGCC
CGGCGCCTACAACGTCAACATCAAGTTGGACATCACCTCCCACAACGAGGACTACACCATCGTGGA
ACAGTACGAACGCGCCGAGGGCCGCCACTCCACCGGCGGCATGGACGAGCTGTACAAG CTGCAG
*GGCGGAGGAGGCAGC GGCGGAGGAGGCAGC GGCGGAGGAGGCAGC*
AGAAGGTATACACGCCGGAAGAATCTGTAGAGATCCCCGGTCGCCACC
GTGAGCAAGGGCGAGGAGCTGTTCACCGGGGTGGTGCCCATCCTGGTCGAGCTGGACGGCGACGTAAAC
GGCCACAAGTTCAGCGTGTCCGGCGAGGGCGAGGGCGATGCCACCTACGGCAAGCTGACCCTGAAGTTC
ATCTGCACCACCGGCAAGCTGCCCGTGCCCTGGCCCACCCTCGTGACCACCCTGACCTACGGCGTGCAGT
GCTTCAGCCGCTACCCCGACCACATGAAGCAGCACGACTTCTTCAAGTCCGCCATGCCCGAAGGCTACGT
CCAGGAGCGCACCATCTTCTTCAAGGACGACGGCAACTACAAGACCCGCGCCGAGGTGAAGTTCGAGGG
CGACACCCTGGTGAACCGCATCGAGCTGAAGGGCATCGACTTCAAGGAGGACGGCAACATCCTGGGGCA
CAAGCTGGAGTACAACTACAACAGCCACAACGTCTATATCATGGCCGACAAGCAGAAGAACGGCATCAA
GGTGAACTTCAAGATCCGCCACAACATCGAGGACGGCAGCGTGCAGCTCGCCGACCACTACCAGCAGAA
CACCCCCATCGGCGACGGCCCCGTGCTGCTGCCCGACAACCACTACCTGAGCACCCAGTCCGCCCTGAGC
AAAGACCCCAACGAGAAGCGCGATCACATGGTCCTGCTGGAGTTCGTGACCGCCGCCGGGATCACTCTC
GGCATGGACGAGCTGTACAAGTAA-3'

## Figure.30B

**Reporter-3:** (SEQ ID NO:1,SEQ ID NO:5, and SEQ ID NO:3)

5' [mCherry] [Linker][Target] [ eGFP ] 3'

5'-
ATGGTGAGCAAGGGCGAGGAGGATAACATGGCCATCATCAAGGAGTTCATGCGCTTCAAGGTGCACATG
GAGGGCTCCGTGAACGGCCACGAGTTCGAGATCGAGGGCGAGGGCGAGGGCCGCCCCTACGAGGGCAC
CCAGACCGCCAAGCTGAAGGTGACCAAGGGTGGCCCCCTGCCCTTCGCCTGGGACATCCTGTCCCCTCAG
TTCATGTACGGCTCCAAGGCCTACGTGAAGCACCCCGCCGACATCCCCGACTACTTGAAGCTGTCCTTCC
CCGAGGGCTTCAAGTGGGAGCGCGTGATGAACTTCGAGGACGGCGGCGTGGTGACCGTGACCCAGGACT
CCTCCCTGCAGGACGGCGAGTTCATCTACAAGGTGAAGCTGCGCGGCACCAACTTCCCCTCCGACGGCCC
CGTAATGCAGAAGAAGACCATGGGCTGGGAGGCCTCCTCCGAGCGGATGTACCCCGAGGACGGCGCCCT
GAAGGGCGAGATCAAGCAGAGGCTGAAGCTGAAGGACGGCGGCCACTACGACGCTGAGGTCAAGACCA
CCTACAAGGCCAAGAAGCCCGTGCAGCTGCCCGGCGCCTACAACGTCAACATCAAGTTGGACATCACCT
CCCACAACGAGGACTACACCATCGTGGAACAGTACGAACGCGCCGAGGGCCGCCACTCCACCGGCGGCA
TGGACGAGCTGTACAAG cɪɢcᴀɢ ***GGCGGAGGAGGCAGC***
**GCCTGCTCGCGATGCTAGAGGGCTCTGCCA**
GTGAGCAAGGGCGAGGAGCTGTTCACCGGGGTGGTGCCCATCCTGGTCGAGCTGGACGGCGACGTAAAC
GGCCACAAGTTCAGCGTGTCCGGCGAGGGCGAGGGCGATGCCACCTACGGCAAGCTGACCCTGAAGTTC
ATCTGCACCACCGGCAAGCTGCCCGTGCCCTGGCCCACCCTCGTGACCACCCTGACCTACGGCGTGCAGT
GCTTCAGCCGCTACCCCGACCACATGAAGCAGCACGACTTCTTCAAGTCCGCCATGCCCGAAGGCTACGT
CCAGGAGCGCACCATCTTCTTCAAGGACGACGGCAACTACAAGACCCGCGCCGAGGTGAAGTTCGAGGG
CGACACCCTGGTGAACCGCATCGAGCTGAAGGGCATCGACTTCAAGGAGGACGGCAACATCCTGGGGCA
CAAGCTGGAGTACAACTACAACAGCCACAACGTCTATATCATGGCCGACAAGCAGAAGAACGGCATCAA
GGTGAACTTCAAGATCCGCCACAACATCGAGGACGGCAGCGTGCAGCTCGCCGACCACTACCAGCAGAA
CACCCCCATCGGCGACGGCCCCGTGCTGCTGCCCGACAACCACTACCTGAGCACCCAGTCCGCCCTGAGC
AAAGACCCCAACGAGAAGCGCGATCACATGGTCCTGCTGGAGTTCGTGACCGCCGCCGGGATCACTCTC
GGCATGGACGAGCTGTACAAGTAA-3'

## Figure.30C

**Figure.31**

Figure.31(cont)

Figure.32A

Figure.32B

Figure.33A

Figure.33B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 2018110105 W
- CN 2019082713

### Non-patent literature cited in the description

- **SAMBROOK et al.** Molecular Cloning: A Laboratory Manual. Cold Spring Harbor Press, 1989
- **AUSUBEL et al.** Current Protocols in Molecular Biology (Supplement 47. John Wiley & Sons, 1999
- Overview of principles of hybridization and the strategy of nucleic acid probe assay. **TIJSSEN.** Laboratory Techniques In Biochemistry And Molecular Biology- Hybridization With Nucleic Acid Probes Part I. Elsevier, 1993
- Site-selective versus promiscuous A-to-I editing. **WAHLSTEDT ; HELENE ; MARIE.** RNA. Wiley Interdisciplinary Reviews, 2011, vol. 2.6, 761-771
- Remington's Pharmaceutical Sciences. 1980
- **PORTEUS, M. H ; CARROLL, D.** Gene targeting using zinc finger nucleases. *Nat Biotechnol,* 2005, vol. 23, 967-973
- **BOCH, J et al.** Breaking the code of DNA binding specificity of TAL-type III effectors. *Science,* 2009, vol. 326, 1509-1512
- **MOSCOU, M. J ; BOGDANOVE, A. J.** A simple cipher governs DNA recognition by TAL effectors. *Science,* 2009, vol. 326, 1501
- **MILLER, J. C et al.** A TALE nuclease architecture for efficient genome editing. *Nat Biotechnol,* 2011, vol. 29, 143-148
- **JINEK, M et al.** A programmable dual-RNA-guided DNA endonuclease in adaptive bacterial immunity. *Science,* 2012, vol. 337, 816-821
- **CONG, L et al.** Multiplex genome engineering using CRISPR/Cas systems. *Science,* 2013, vol. 339, 819-823
- **MALI, P et al.** RNA-guided human genome engineering via Cas9. *Science,* 2013, vol. 339, 823-826
- **KOMOR, A. C ; KIM, Y. B ; PACKER, M. S. ; ZURIS, J. A ; LIU, D. R.** Programmable editing of a target base in genomic DNA without double-stranded DNA cleavage. *Nature,* 2016, vol. 533, 420-424
- **MA, Y et al.** Targeted AID-mediated mutagenesis (TAM) enables efficient genomic diversification in mammalian cells. *Nat Methods,* 2016, vol. 13, 1029-1035
- **GAUDELLI, N. M. et al.** Programmable base editing of A*T to G*C in genomic DNA without DNA cleavage. *Nature,* 2017, vol. 551, 464-471
- **TAN, M. H et al.** Dynamic landscape and regulation of RNA editing in mammals. *Nature,* 2017, vol. 550, 249-254
- **NISHIKURA, K.** Functions and regulation of RNA editing by ADAR deaminases. *Annu Rev Biochem,* 2010, vol. 79, 321-349
- **BASS, B. L ; WEINTRAUB, H.** An unwinding activity that covalently modifies its double-stranded RNA substrate. *Cell,* 1988, vol. 55, 1089-1098
- **WONG, S. K. ; SATO, S ; LAZINSKI, D. W.** Substrate recognition by ADAR1 and ADAR2. *RNA,* 2001, vol. 7, 846-858
- **MONTIEL-GONZALEZ, M. F. ; VALLECILLO-VIEJO, I. ; YUDOWSKI, G. A ; ROSENTHAL, J. J.** Correction of mutations within the cystic fibrosis transmembrane conductance regulator by site-directed RNA editing. *Proc Natl Acad Sci U S A,* 2013, vol. 110, 18285-18290
- **SINNAMON, J. R et al.** Site-directed RNA repair of endogenous Mecp2 RNA in neurons. *Proc Natl Acad Sci U S A,* 2017, vol. 114, E9395-E9402
- **MONTIEL-GONZALEZ, M. F ; VALLECILLO-VIEJO, I. C ; ROSENTHAL, J. J.** An efficient system for selectively altering genetic information within mRNAs. *Nucleic Acids Res,* 2016, vol. 44, e157
- **HANSWILLEMENKE, A. ; KUZDERE, T. ; VOGEL, P. ; JEKELY, G ; STAFFORST, T.** Site-Directed RNA Editing in Vivo Can Be Triggered by the Light-Driven Assembly of an Artificial Riboprotein. *J Am Chem Soc,* 2015, vol. 137, 15875-15881
- **SCHNEIDER, M. F. ; WETTENGEL, J. ; HOFFMANN, P. C ; STAFFORST, T.** Optimal guideRNAs for re-directing deaminase activity of hADAR1 and hADAR2 in trans. *Nucleic Acids Res,* 2014, vol. 42, e87
- **VOGEL, P. ; HANSWILLEMENKE, A ; STAFFORST, T.** Switching Protein Localization by Site-Directed RNA Editing under Control of Light. *ACS synthetic biology,* 2017, vol. 6, 1642-1649

- **VOGEL, P. ; SCHNEIDER, M. F. ; WETTENGEL, J ; STAFFORST, T.** Improving site-directed RNA editing in vitro and in cell culture by chemical modification of the guideRNA. *Angewandte Chemie,* 2014, vol. 53, 6267-6271
- **VOGEL, P et al.** Efficient and precise editing of endogenous transcripts with SNAP-tagged ADARs. *Nat Methods,* 2018, vol. 15, 535-538
- **COX, D. B. T et al.** RNA editing with CRISPR-Cas13. *Science,* 2017, vol. 358, 1019-1027
- **FUKUDA, M et al.** Construction of a guide-RNA for site-directed RNA mutagenesis utilising intracellular A-to-I RNA editing. *Scientific reports,* 2017, vol. 7, 41478
- **WETTENGEL, J. ; REAUTSCHNIG, P. ; GEISLER, S. ; KAHLE, P. J ; STAFFORST, T.** Harnessing human ADAR2 for RNA repair - Recoding a PINK1 mutation rescues mitophagy. *Nucleic Acids Res,* 2017, vol. 45, 2797-2808
- **HEEP, M. ; MACH, P. ; REAUTSCHNIG, P ; WETTENGEL, J ; STAFFORST, T.** Applying Human ADAR1p110 and ADAR1p150 for Site-Directed RNA Editing-G/C Substitution Stabilizes GuideRNAs against Editing. *Genes (Basel),* 2017, vol. 8
- **KATREKAR, D. et al.** In vivo RNA editing of point mutations via RNA-guided adenosine deaminases. *Nat Methods,* 2019, vol. 16, 239-242
- **YIN, H. ; KAUFFMAN, K. J ; ANDERSON, D. G.** Delivery technologies for genome editing. *Nat Rev Drug Discov,* 2017, vol. 16, 387-399
- **PLATT, R. J et al.** CRISPR-Cas9 knockin mice for genome editing and cancer modeling. *Cell,* 2014, vol. 159, 440-455
- **CHEW, W. L et al.** A multifunctional AAV-CRISPR-Cas9 and its host response. *Nat Methods,* 2016, vol. 13, 868-874
- **TEOH, P. J et al.** Aberrant hyperediting of the myeloma transcriptome by ADAR1 confers oncogenicity and is a marker of poor prognosis. *Blood,* 2018, vol. 132, 1304-1317
- **VALLECILLO-VIEJO, I. C. ; LISCOVITCH-BRAUER, N. ; MONTIEL-GONZALEZ, M. F ; EISENBERG, E ; ROSENTHAL, J. J. C.** Abundant off-target edits from site-directed RNA editing can be reduced by nuclear localization of the editing enzyme. *RNA biology,* 2018, vol. 15, 104-114
- **MAYS, L. E ; WILSON, J. M.** The complex and evolving story of T cell activation to AAV vector-encoded transgene products. *Mol Ther,* 2011, vol. 19, 16-27
- **WAGNER, D. L et al.** High prevalence of Streptococcus pyogenes Cas9-reactive T cells within the adult human population. *NatMed,* 2019, vol. 25, 242-248
- **SIMHADRI, V. L et al.** Prevalence of Pre-existing Antibodies to CRISPR-Associated Nuclease Cas9 in the USA Population. *Mol Ther Methods Clin Dev,* 2018, vol. 10, 105-112
- **CHARLESWORTH, C. T et al.** Identification of pre-existing adaptive immunity to Cas9 proteins in humans. *NatMed,* 2019, vol. 25, 249-254
- **HAAPANIEMI, E. ; BOTLA, S. ; PERSSON, J. ; SCHMIERER, B ; TAIPALE, J.** CRISPR-Cas9 genome editing induces a p53-mediated DNA damage response. *Nat Med,* 2018, vol. 24, 927-930
- **IHRY, R. J et al.** p53 inhibits CRISPR-Cas9 engineering in human pluripotent stem cells. *Nat Med,* 2018, vol. 24, 939-946
- **WOOLF, T. M. ; CHASE, J. M ; STINCHCOMB, D. T.** Toward the therapeutic editing of mutated RNA sequences. *Proc Natl Acad Sci U S A,* 1995, vol. 92, 8298-8302
- **ZHENG, Y. ; LORENZO, C ; BEAL, P. A.** DNA editing in DNA/RNA hybrids by adenosine deaminases that act on RNA. *Nucleic Acids Res,* 2017, vol. 45, 3369-3377
- **ABUDAYYEH, O. O et al.** C2c2 is a single-component programmable RNA-guided RNA-targeting CRISPR effector. *Science,* 2016, vol. 353, 5573
- **DANIEL, C ; WIDMARK, A. ; RIGARDT, D ; OHMAN, M.** Editing inducer elements increases A-to-I editing efficiency in the mammalian transcriptome. *Genome Biol,* 2017, vol. 18, 195
- **CHEN, C. X et al.** A third member of the RNA-specific adenosine deaminase gene family, ADAR3, contains both single- and double-stranded RNA binding domains. *RNA,* 2000, vol. 6, 755-767
- **SAVVA, Y. A. ; RIEDER, L. E ; REENAN, R. A.** The ADAR protein family. *Genome Biol,* 2012, vol. 13, 252
- **NISHIKURA, K.** A-to-I editing of coding and non-coding RNAs by ADARs. *Nat Rev Mol Cell Biol,* 2016, vol. 17, 83-96
- **FLOQUET, C. ; DEFORGES, J. ; ROUSSET, J. P ; BIDOU, L.** Rescue of non-sense mutated p53 tumor suppressor gene by aminoglycosides. *Nucleic Acids Res,* 2011, vol. 39, 3350-3362
- **KERN, S. E et al.** Identification of p53 as a sequence-specific DNA-binding protein. *Science,* 1991, vol. 252, 1708-1711
- **DOUBROVIN, M et al.** Imaging transcriptional regulation of p53-dependent genes with positron emission tomography in vivo. *Proc Natl Acad Sci U S A,* 2001, vol. 98, 9300-9305
- **LANDRUM, M. J et al.** ClinVar: public archive of interpretations of clinically relevant variants. *Nucleic Acids Res,* 2016, vol. 44, D862-868
- **OU, L et al.** ZFN-Mediated In Vivo Genome Editing Corrects Murine Hurler Syndrome. *Mol Ther,* 2019, vol. 27, 178-187
- **FIRE, A et al.** Potent and specific genetic interference by double-stranded RNA in Caenorhabditis elegans. *Nature,* 1998, vol. 391, 806-811

- **ZUO, E et al.** Cytosine base editor generates substantial off-target single-nucleotide variants in mouse embryos. *Science,* 2019
- **JIN, S et al.** Cytosine, but not adenine, base editors induce genome-wide off-target mutations in rice. *Science,* 2019
- **KIM, D. ; KIM, D. E. ; LEE, G. ; CHO, S. I ; KIM, J. S.** Genome-wide target specificity of CRISPR RNA-guided adenine base editors. *Nat Biotechnol,* 2019, vol. 37, 430-435
- **LEVANON, E. Y et al.** Systematic identification of abundant A-to-I editing sites in the human transcriptome. *Nat Biotechnol,* 2004, vol. 22, 1001-1005
- **MERKLE, T. et al.** Precise RNA editing by recruiting endogenous ADARs with antisense oligonucleotides. *Nat Biotechnol,* 2019, vol. 37, 133-138
- **WAGNER, R. W et al.** Double-stranded RNA unwinding and modifying activity is detected ubiquitously in primary tissues and cell lines. *Mol Cell Biol,* 1990, vol. 10, 5586-5590
- **PATTERSON, J. B. ; SAMUEL, C. E.** Expression and regulation by interferon of a double-stranded-RNA-specific adenosine deaminase from human cells: evidence for two forms of the deaminase. *Mol Cell Biol,* 1995, vol. 15, 5376-5388
- **GIBSON, D. G et al.** Enzymatic assembly of DNA molecules up to several hundred kilobases. *Nat Methods,* 2009, vol. 6, 343-345
- **ZHOU, Y. ; ZHANG, H ; WEI, W.** Simultaneous generation of multi-gene knockouts in human cells. *FEBS Lett,* 2016, vol. 590, 4343-4353
- **DOBIN, A et al.** STAR: ultrafast universal RNA-seq aligner. *Bioinformatics,* 2013, vol. 29, 15-21
- **VAN DER AUWERA, G. A. et al.** From FastQ data to high confidence variant calls: the Genome Analysis Toolkit best practices pipeline. *Curr Protoc Bioinformatics,* 2013, vol. 11 (10), 11-33
- **WANG, K. ; LI, M ; HAKONARSON, H.** ANNOVAR: functional annotation of genetic variants from high-throughput sequencing data. *Nucleic Acids Res,* 2010, vol. 38, e164
- **GENOMES PROJECT, C et al.** An integrated map of genetic variation from 1,092 human genomes. *Nature,* 2012, vol. 491, 56-65
- **PERTEA, M. ; KIM, D. ; PERTEA, G. M ; LEEK, J. T ; SALZBERG, S. L.** Transcript-level expression analysis of RNA-seq experiments with HISAT, StringTie and Ballgown. *Nat Protoc,* 2016, vol. 11, 1650-1667